(19) Europäisches Patentamt
European Patent Office
Office européen des brevets

(11) EP 1 683 868 A1

(12) EUROPEAN PATENT APPLICATION

(43) Date of publication:
**26.07.2006 Bulletin 2006/30**

(51) Int Cl.:
***C12N 15/62*** *(2006.01)* ***C12Q 1/66*** *(2006.01)*
***G01N 33/58*** *(2006.01)*

(21) Application number: **05450006.1**

(22) Date of filing: **21.01.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Medizinische Universität Graz**
**8036 Graz (AT)**

(72) Inventors:
- **Graier, Wolfgang, Dr.**
  **8111 Judendorf-Strassengel (AT)**
- **Osibow, Karin, Mag.**
  **8010 Graz (AT)**
- **Malli, Roland, Dr.**
  **8443 Gleinstätten (AT)**
- **Kostner, Gerhard, Dr.**
  **8044 Graz (AT)**

(74) Representative: **Sonn & Partner Patentanwälte**
**Riemergasse 14**
**1010 Wien (AT)**

# (54) Kringle domains in a calcium sensor fusion protein

(57) The present invention relates to the use of kringle domains in a sensor fusion protein, designed to measure the concentration of $Ca^{2+}$ ions in the ER or together with a proper chaperone complex in samples *ex vivo.* The sensor protein is capable to measure the $Ca^{2+}$ concentration indirectly thus avoiding the side-effect of affecting $Ca^{2+}$ related metabolisms in a cell or cell compartment. This improves applications directed at observations of $Ca^{2+}$ dependent processes *in vivo.*

Figure 1

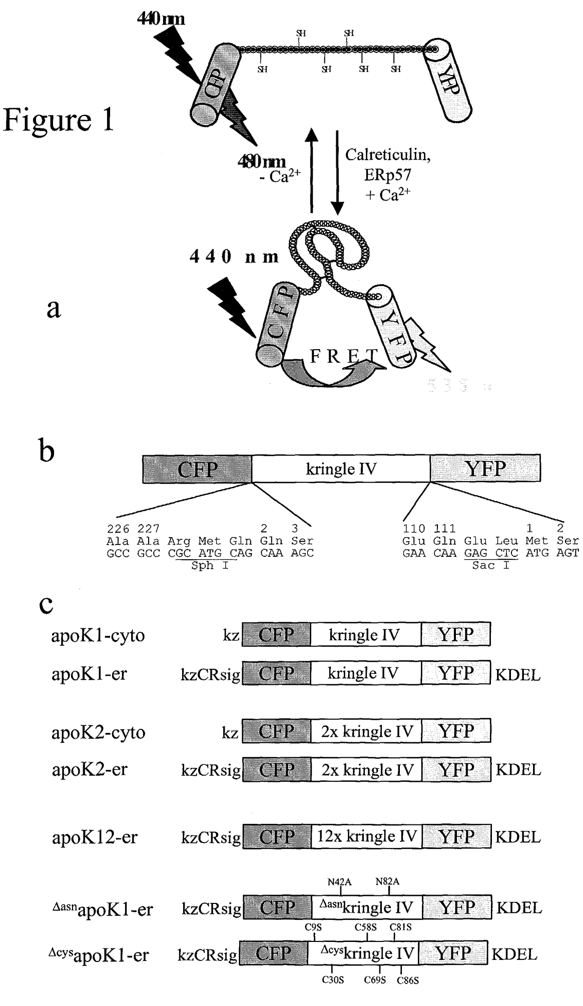


EP 1 683 868 A1

## Description

The present invention relates to sensor proteins for folding processes.

Folding processes of proteins are critical procedures in a cell. The capability to properly fold a quantity of a given protein eventually determines the amount of proteins available for the cell for regulatory and structural functions. Proteins destined for various locations along the secretory pathway are initially synthesised on membrane-bound ribosomes of the endoplasmic reticulum (ER) and are translocated into the ER lumen where they fold and undergo subunit assembly. Protein folding within the ER (as in other cellular locations) is facilitated by a class of proteins termed molecular chaperones. Molecular chaperones bind transiently to nascent polypeptide chains and function by preventing aggregation and by maintaining polypeptides in conformations competent for folding and subunit assembly. Nascent proteins that are unable to fold or assemble correctly are generally retained within the ER in association with chaperones and are degraded or might result in insoluble protein deposits, this in turn can lead to many different diseases. Therefore many methods have been developed to monitor the folding of several proteins inside a cell. The goal is to screen for misfolded target proteins but also for aberrations in a cell's folding machinery. One important chaperone complex inside the endoplasmic reticulum (ER) comprises the chaperones calnexin, calreticulin and ERp57. Both, calnexin and calreticulin bind to ERp57, a thiol oxidoreductase of the ER. This interaction enhances disulfide bond formation in nascent proteins that are bound to either calnexin or calreticulin. In addition are the activities of both, calreticulin and calnexin dependent on the presence of $Ca^{2+}$ ions in their active sites.

Although the machinery responsible for protein folding inside the endoplasmic reticulum (ER) is well established (Ellgaard and Hele-nius, Nat Rev Mol Cell Biol 4 (2003), 181-91), the understanding of the kinetics and dynamic regulation of this pivotal step in posttranslational protein maturation is incomplete. Moreover, the question how $Ca^{2+}$-dependent protein folding inside the ER (Michalak et al., Cell Calcium 32 (2002), 269-78) is controlled and ensured even under rhythmic or tonic ER $Ca^{2+}$ depletion could not be investigated in detail as the techniques currently available for monitoring protein folding are interfering with the $Ca^{2+}$ metabolism or are essentially disruptive and/or built on the use of large cell populations. Their temporal and spatial resolution is limited and does not allow continuous monitoring of folding processes within single cells or even cellular compartments. However, since malfunction in the process of protein folding has also been discussed beyond the classical scenario of inherited diseases in the context of e.g. aging, diabetes, atherosclerosis and slow (virus) diseases, non-invasive techniques with better temporal and spatial resolution are essentially required. Methods which are capable to track protein folding as described in the prior art are specifically designed to pursuit a specific goal and are not easily adapted to other problems.

One method to detect solubility and proper protein folding via fusion of marker proteins to a protein segment under investigation is described in US 6,727,070. The goal therein is to present an assay for the proper folding and solubility of recombinant proteins. This method is based on the principle of creating a fusion protein with a special marker protein which eventually delivers the information on the state of the target recombinant protein. Herein a marker is a fragment of the enzyme β-Galactosidase. Upon proper folding and solvation the marker fragment fused to the target protein and the second fragment of the marker enzyme, here available separately in solution in the cell, form an active ensemble. Such a pair of marker proteins are, in this case, the α and β fragments of β-Galactosidase. If the only source of a cell of e.g. the α fragment is attached to an insoluble protein, while the β fragment is expressed independently in solution and is therefore separated from the α fragment, no active β-Galactosidase can be formed, which can be assayed by growth on indicator agar plates using chromogenic X-gal. β-Galactosidase activity can be used to report the solubility of a target fusion protein. This concept is not capable to sustain an expression system where both parts of a marker protein or protein complex are linked to a single target protein at the same time since great mobility of the marker fragments is required to form an active complex.

Wigley et al., Nature Biotech 19 (2001), 131-136, describes an application of the US patent no. 6,727,070 to measure the solubility of the Alzheimer's amyloid β by fusion of the amyloid β to the α fragment of β-Galactosidase and a subsequent complementation assay as described in the previously mentioned US patent.

Different marker proteins are used in assays directed to the observation of protein interactions (Johnson and Varshavsky, PNAS USA 91 (1994), 10340-44 and Fields and Song, Nature 340(6230) (1989), 245-6). Johnson et al. describe the use of fusion peptides with ubiquitin fragments, attached to two different proteins. A ubiquitin-specific protease cleaves the ubiquitin fragments of both fusion proteins only if the fusion proteins are expressed in the same cell. Fields and Song provide a system to detect protein-protein interaction with the use of GAL4-fragment-fusion proteins as marker. Two compatible GAL4-fragments are attached to two different proteins under observation. Only through coexpression and complexation of the fusion proteins, the formation of an active GAL4 is facilitated. Both methods disclose systems with different marker peptides to detect protein-protein interactions, applicability to detect protein folding is not presented therein.

The publication Miyawaki et al., Nature 388 (1997), 882-887, describes a fusion protein comprising calmodulin and the calmodulin-binding domain M13, wherein two GFP (green fluorescent protein) variants are attached to the N and C termini of the calm-odulin/M13 core. Calmodulin is capable to bind four $Ca^{2+}$ ions. After this $Ca^{2+}$ binding event,

the fusion protein described by Miyawaki et al. undergoes a structural change which moves the first FP (fluorescent protein) into proximity of the second FP, which potentiates a fluorescence resonance energy transfer (FRET) between the two FPs. Through the FRET, excitation of the first GFP variant (donor) leads to the emission of photons by the second GFP variant (acceptor). A donor/acceptor pair is e.g. a blue- or cyan-emitting mutant of the GFP and a green- or yellow-emitting GFP. This construct can be used to asses the $Ca^{2+}$ concentration in a cell through structural changes imposed by the complexation of four $Ca^{2+}$ ions to the calmodulin domain.

In Philipps et al., J Mol Biol 327 (2003), 239-249, the general principle of fusing GFP variants as markers to the termini of a general protein X is disclosed. As example, folding of antibody $V_L$ intradomains is given.

In US 6,376,257 a similar fusion protein to Miyawaki et al. is described, wherein the target protein between two GFP variants is a sole calmodulin-binding protein. This fusion protein can be used to determine the calmodulin concentration in a cell or the $Ca^{2+}$ concentration if calmodulin is given in abundance in the cell.

Both methods, US 6,376,257 and Miyawaki et al., rely on a protein construct which as a prerequisite allows spacial proximity of the marker proteins (FPs). In both methods, the measurement of the $Ca^{2+}$ concentration was facilitated by direct complexation of $Ca^{2+}$ ions, whereby the concentration of free $Ca^{2+}$ in the cell is reduced which alters $Ca^{2+}$ dependent processes in a living cell. This buffering effect is insofar adversarial if processes involving a natural $Ca^{2+}$ distribution are under observation.

It should be noted that such a configuration for an arbitrary protein to be used in a construct where folding can be detected by FRET is rarely given and much research is necessary to find a useful protein or peptide configuration.

It is a general object of the present invention to find means to monitor protein folding processes especially of $Ca^{2+}$ dependent chaperone complexes formed by calreticulin and/or calnexin.

It is a further object of the present invention to provide tools to monitor the $Ca^{2+}$ concentration without interfering in cellular processes which would be favourable, since $Ca^{2+}$ plays an important regulatory role, not only in the ER but also in other cell compartments (Michalak et al. Cell Calcium 32 (2002) 269-78).

Therefore sensors that are capable to deliver a $Ca^{2+}$ dependent signal without interfering in a cell's $Ca^{2+}$ metabolism and regulation are specifically sought-after. A localisation of $Ca^{2+}$ concentrations in specific cell compartments would further enrich the information content of scientific studies.

Therefore, the present invention provides a protein or protein complex comprising a first marker moiety, at least one kringle protein domain and a second marker moiety, wherein

- said first and second marker moiety is attached to said kringle protein domain(s);
- said kringle domain(s) having a first and a second conformation state, and
- said first conformation state and said second conformation state have a different marker signal with respect to said first and second marker moiety.

The protein or protein complex according to the present invention is an efficient tool for the determination of various components and folding processes in or outside living cells, especially metal-ions, such as $Ca^{2+}$, and metal-ion dependent folding processes. The protein or protein complex according to the present invention is a primary sensor component, which is sensible to protein folding. Therefore the protein or protein complex of the present invention is also referred to as "sensor" or "kringle domain sensor".

"Kringle domains" as noted above are common domains in proteins of the fibrinolytic and coagulation cascades, such as plasminogen, thrombin and prothrombin, apo(a), urokinase and tissue-type plasminogen activators (t-PA). These kringles (kringle domains) correspond to autonomous structural and folding domains which mediate the binding of these multidomain proteins to other proteins (Patthy et al. FEBS Lett. 171 (1984), 131-6). These kringle domains share the same gross architecture, the kringle -fold, but diverge in their protein binding capacities. In principle, any kringle domain can be used according to the present invention. The kringle domain can be specifically chosen and/or adopted due to the specific need of the question to be addressed. Especially binding specificities may be selected or engineered depending on the folding process which needs to be analysed. Preferably, the kringle domain has a binding site for a ligand and the first conformation state is defined as the conformation state without the ligand and the second confirmation state is defined as the conformation state with the ligand attached to the kringle domain.

Preferred kringle domains are depicted in Fig.6 and comprise also the consensus sequence of the kringle-fold (SEQ ID NO 18). Modifications to the natural kringle domains can be performed using standard protein engineering - thereby preserving the essential kringle characteristics shown by these sequences, especially the Cys residues necessary for the disulfide bonds and the N-Glycosylation sites. Sequences comprising at least 70%, preferably at least 80%, sequence identity to one or more of the sequences depicted in Fig.6, especially to the consensus sequence, an apo(a) kringle IV domain, an urokinase, pro-thrombin or t-PA kringle, and functionally acting as a kringle domain are preferred embodiments of the present invention. As mentioned, this kringle fold is constituted by common disulfide bridges and can be folded by the chaperones calnexin and calreticulin. Therefore all these kringle domains, which show sufficient protein sequence homology to the kringle IV domain of apo(a) are suitable for use in the present concept of a kringle domain sensor.

The terms "homology" and "identity", as defined herein, are often used interchangeably. In general, sequences are aligned so that the highest order match is obtained (see, e.g.: Computational Molecular Biology, Lesk, A.M. , ed., Oxford University Press, New York,1988 ; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G. , Academic Press, 1987; and Sequence Analysis Primer, Gribskov, M. and Devereux, J., eds., M Stockton Press, New York,1991; Carillo et al. (1988) SIAM J Applied Math 48:1073).

By sequence identity, the number of conserved amino acids is determined by standard alignment algorithms programs, and is used with default gap penalties established by each supplier. Such programs are for example FAST A (Pearson et al. (1988) PNAS USA 85: 2444), the GCG program package (Devereux, J., et al., Nucleic Acids Research (1984) Nucleic Acids Res., 12, 387-395), BLASTP, BLASTN, FASTA (Atschul, S.F., et al., J Molec Biol 215: 403 (1990); Guide to Huge Computers, Martin J. Bishop, ed., Academic Press, San Diego, 1994, and Carilloet al, (1988)SIAM J Applied Math 48 : 1073). For instance, the BLAST tool of the NCBI database can be used to determine identity. Percent homology or identity of proteins and/or nucleic acid molecules can be determined, for example, by comparing sequence information using a GAP computer program (e.g. Needleman et al., (1970) J. Mol. Biol. 48:443, as revised by Smith and Waterman (1981) Adv. Appl. Math. 2:482). Briefly, the GAP program defines similarity as the number of aligned symbols (i.e., nucleotides or amino acids) which are similar, divided by the total number of symbols in the shorter of the two sequences. Default parameters for the GAP program can include : (1) a unary comparison matrix (containing a value of 1 for identities and for non-identities) and the weighted comparison matrix of Gribskov et al. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

As used herein, the term "at least 70% identical to" refers to percent identities from 70% to 100% relative to the reference polypeptide, determined by any one of the mentioned algorithms or programs.

Identity at a level of 70% or more is indicative of the fact that, assuming for exemplification purposes a test and reference polynucleotide length of 100 nucleic acids are compared, no more than 30% (i.e. 30 out of 100) of nucleic acids in the test polynucleotide differs from that of the reference polynucleotide. Similar comparisons can be made between a test and reference polypeptides. Such differences can be represented as point mutations randomly distributed over the entire length of an nucleic/amino acid sequence or they can be clustered in one or more locations of varying length up to the maximum allowable, e.g. 30/100 amino acid difference (approx. 70% identity). Differences are defined as nucleic acid or amino acid substitutions, or deletions. At the level of homologies or identities above about 85-90%, the result should be independent of the program and gap parameters set; such high levels of identity can be assessed readily, often without relying on software.

Proteins, which contain kringles with a high sequence homology to apo(a) kringle domains, are for example plasminogen, thrombin, urokinase, prothrombin and t-PAs (tissue-type plasminogen activators). These kringle domains can be used as sensor element in the sensor of the present invention, wherein the kringle domain has to contain at least all cystein residues involved in disulfide bridge binding. Apparently, all of these kringle domain containing proteins inside an eukaryotic cell are folded and processed in the ER.

Preferably, the number of kringle domains is one to five, especially one and two. The best results are usually obtained with one kringle domain. Nevertheless, the use of at least two kringle domains also results in a functional binding of the ligand and therefore a proper determination of the analyte in question, especially for obtaining a sensitive $Ca^{2+}$ dependent signal. To modify the folding kinetics this use of sensors with more kringle domains may be advantageous for certain cases.

In a preferred embodiment of the present invention the kringle domain of the sensor is a kringle IV domain (SEQ ID NO 5 and SEQ ID NO 6) of apolipoprotein(a) (apo(a); SEQ ID NO 16 and SEQ ID NO 17). The apolipoprotein (a) consists of 12 to 51 kringle domains, wherein all but one are homologous to the fourth kringle domain of plasminogen (Ikeo et al. FEBS Lett 287 (1991), 146-148). There are 10 types of kringle IV domains known in apo(a). For the sensor protein or protein complex according to the present invention all of them can be used for the kringle domain sensor, as well as homologues therefrom, which show at least 70% of protein sequence identity to any kringle IV domain of apo (a), depending on their binding specificity for chaperones which results in a folding of the sensitive core structure of the sensor. Moreover, any one or any combination therefrom can be used as the fold-sensitive kringle domain of the sensor protein of the present invention. Furthermore, in a preferred embodiment of the present invnetion, the protein or protein complex of the present invention is based on the kringle IV type 2 domain of the apolipoprotein(a).

The sensor protein or protein complex according to the present invention comprises marker moieties, as known in the state of the art, and a component comprising of at least one kringle domain, preferably from apolipoprotein(a) (apo(a)), or homologues therefrom, as noted above. Especially the kringle IV domain of apo(a) proved to be well suited to provide a sensible protein domain as a sensor for protein folding reactions (see examples).

Since the folding characteristics of kringle domains of different proteins is similar, the current invention also comprises kringle domain sensors with a kringle domain, which has a sequence having sequence identity of at least 70%, 80%, 90% or 99% to any kringle IV domain of apolipoprotein(a).

The "first and second" marker moiety may be any marker molecule which - in combination - result in a measurable difference in the overall signal ("marker signal") of these two moieties between the first and the second conformation state of the protein or protein complex according to the present invention. Preferably, the two marker moieties have a weak or no signal (below the detection limit) in one conformation state and a strong marker signal in the other conformation state. If the kringle domain in the protein complex according to the present invention binds to a ligand it is preferred that the conformation state without the ligand results in a weak or no signal and the conformation state with the ligand bound results in a strong signal. The difference in marker signal between the first and the second conformation state should be high enough to exclude interference of the marker signal with background noise, preferably at least 3 times higher than mean background noise. Usually applied systems according to the present invention use a difference of at least a factor 10, preferably at least 100, signal/noise difference.

The marker moieties are preferably moieties which have a fluorescence, e.g. small or medium sized fluorescence molecules or fluorescent proteins or peptides. Specifically, any marker protein or fragments thereof or fluorescent molecules that are sensitive to spatial nearness to the other marker are applicable. Generally, any signal or information ("marker signal") deriveable from a signal that can discern the folded and the unfolded kringle domain, depending on the set of protein marker, is possible. This spatial nearness is defined by the type of marker employed. For example FRET-markers (fluorescence resonance energy transfer) require a spatial nearness of below 10nm, preferably between 2 and 5 nm for activity. This spatial dependency of the marker also limits in this case the number of kringle domains, which can be used for a sensor of the present invention. Therefore the preferred upper limit for a FRET-marker sensor is five kringle domains. Preferred marker molecules or marker proteins, which can be used as marker moieties according to the present invention are, for example variants of the green fluorescent protein, chemical fluorophores or chromophores such as tetramethylrhodamine, fluoresceine, 5,5'-dithiobis-(2-nitro)-benzoic acid or thiol-reactive dyes or derivatives thereof like lucifer yellow. Preferred markers may be expressed *in vivo* such as the green, cyan or yellow fluorescent protein. Other chemical markers, which can be synthetically ligated to the kringle domain ex *vivo* are also employable for the sensor of the present invention. The marker moieties are attached, covalently or through tight association (i.e. affinity, ionic, van der Waals, hydrophobic interactions), to a sequence of kringle domains.

For practical reasons it is preferred to provide the marker moieties (or proteins or polypeptides) together with the kringle domain(s) as a fusion protein so that the sensor protein according to the present invention can be recombinantly produced as a whole and provided with the suitable intracellular transport systems for proper targeting in the cell. Although this is not a requisite, applications *in vivo* might require this construct in order to ensure the association of the sensor protein parts in the same cell compartment, e.g. the ER. Furthermore, this represents a more cost efficient path for the production of a sensor than producing the sensor as protein complex with separate proteins (or protein fragment entities). Therefore the present invention relates to a fusion protein comprising, a first marker protein or marker protein fragment, said kringle protein domain(s) and a second marker protein or marker protein complex are linked in this order.

In a preferred implementation of the present kringle domain sensor, the markers show a distance dependent signal based on FRET, BRET or LRET. As described above through the FRET (fluorescence resonance energy transfer), excitation of a first fluorescent molecule (donor) leads to the emission of photons by a second fluorescent molecule (acceptor). It is furthermore possible to substitute the donor by bioluminescent (BRET) or luminescent (LRET) molecules. This does not change the distance dependency of the registered signal. Such an alternative donors/acceptor pair is for example aequorin as donor and GFP as acceptor. Effective donor/acceptor pairs are characterised by suitable emission/absorbance spectra for an optimised resonance energy transfer.

In a preferred implementation of the present invention two different mutant forms of the green fluorescent protein (GFP), in particular the cyan fluorescent protein (CFP) and the yellow fluorescent protein (YFP), are used as marker proteins, wherein the detectable signal is produced by fluorescence resonance energy transfer (FRET). For example, folding of a kringle domain can be visualised by the ratio of 480 nm (donor fluorescence of CFP) and 535 nm (FRET emission of YFP) emission upon excitation of CFP at 440nm. The implementation of GFP variants as marker proteins provides a further improvement over the basic concept of the invention. It allows measurements through highly sensitive fluorescence measurement methods. FRET measurements as described in the prior art are an especially efficient method herein. A preferred embodiment of the present invention relates to a protein comprising a defined number (e.g. 1 or 2) of kringle IV sequences of apo(a) (1 kringle domain: SEQ ID NO 5 and SEQ ID NO 6; 2 kringle domains: SEQ ID NO 7 and SEQ ID NO 8), or homologues therefrom, flanked by CFP and YFP. In a cell the folding process of kringle domains is facilitated inter alia by the $Ca^{2+}$ dependent proteins calreticulin, calnexin and ERp57. This embodiment of a kringle domain sensor was - among others - specifically suitable for monitoring the constitution and activity of the calreticulin/ERp57 and/or calnexin/Erp57 complex, especially as a fluorescent indicator of $Ca^{2+}$ dependent formation of disulfide protein folding and intraluminal $Ca^{2+}$ in the endoplasmic reticulum.

In a furthermore preferred embodiment of the sensor of the present invention the enhanced cyan fluorescent protein (ECFP; SEQ ID NO 3 and SEQ ID NO 4) and the enhanced yellow fluorescent protein (EYFP; SEQ ID NO 9 and SEQ ID NO 10) are used as marker proteins.

The protein or protein complex according to the present invention has several advantages over the techniques used in the prior art, and provides further important information, e.g. localisation of the signal in the cell. Through the activity of the $Ca^{2+}$ dependent chaperones calreticulin, calnexin and ERp57 it is possible to measure the concentration of $Ca^{2+}$ with the kringle domain sensor of the present invention. It allows measurements with a time resolution of that normally achieved by conventional $Ca^{2+}$ imaging techniques. Thus, dynamic regulation of protein folding by cellular $Ca^{2+}$ signaling can be assessed even under fast fluctuations of cel-lular/ER $Ca^{2+}$ concentration (e.g. in cardiac myocytes). Second, utilising kringle domain sensors, protein folding efficiency can be repetitively monitored in one given cell under various conditions as this technique is not invasive and does not harm the cell. Such properties are of particular importance for studies focused to investigate the influence of e.g. therapeutics on protein folding. Third, and perhaps the most intriguing advantage to the present techniques is that kringle domain sensors allow the analysis of protein folding in a subset of the ER in one given cell. Considering the overwhelming evidence that point to a $Ca^{2+}$ crosstalk within the ER and the mitochondria (Malli et al., J Biol Chem 278 (2003), 44769-44779), kringle domain sensors open the possibility to design experiments to investigate the impact of intra-organelle $Ca^{2+}$ movements for protein folding.

The protein or protein complex according to the present invention allows a specific measurement of ligand binding to the kringle domain. If ligand binding is dependent on a specific conformation of the ligand, i.e. being complexed to another substance, this "other substance" can be measured as well in an indirect way by the present invention. In its specific embodiment, the present invention allows the measurement of the formation of the functional complex of calreticulin/calnexin with ERp57 in the ER and the resulting disulfide bridge formation. This is an important prerequisite for the posttranslational protein modification in the ER. This sensor also allows the determination of the $Ca^{2+}$ concentration in the ER. Due to the nature of the specific sensor according to the present invention (which does not have any $Ca^{2+}$ binding sites), the $Ca^{2+}$ sensitivity of the proteins already present *in vivo* (calreticulin and calnexin) is used for indirect determination of $Ca^{2+}$. With this process, the sensor according to the present invention does not influence the intraluminal $Ca^{2+}$ homeostasis (i.e. no buffering effect occurs).

The sensor according to the present invention can be designed in a way that it is selectively transported into the ER. This results in a lack of disturbing signals from other compartments in the time frame of the signal detection. The measurement may be performed in a non-invasive way, i.e. a destruction of the cell is not necessary which allows physiological measurements over a very long time period and is dependent on the durability of the marker molecules, which are for example affected by fluorescence bleaching in the case of fluorescence markers. Suitable measurements can be finished within very short time ranges. It was also possible to maintain continuous measurements in living single cells for 90 minutes or even longer.

The present invention provides a novel tool to monitor intracellular $Ca^{2+}$ concentration by kringle domain folding via chaperone complex activity. The most preferred uses of the sensor lie in the tracking of the folding processes *in vivo,* e.g. to monitor the effects of a medication or drug on $Ca^{2+}$ regulation. In this case, the sensor of the present invention is expressed preferably directly in the cell under observation. The present invention relates to a protein or protein complex as described above, characterised in that it is expressed in eukaryotic cells, in particular endothelial cells, by transformation or transfection of said cell.

Since the chaperones calnexin or calreticulin are naturally present in the ER of an eukaryotic cell it is preferred to include signaling sequences in the sensor molecule according to the present invention as known in the state of the art that direct the kringle domain sensor of the current invention into the lumen of the ER. Inside the ER the kringle domains of the sensor can be folded under natural conditions. Such ER signalling sequences, which can be added to the sensor protein of the current invention are described e.g. in Walter and Johnson, Annu Rev Cell Biol 10 (1994), 87-119.

The invention also provides a protein construct that includes a set of sequences that target the protein to the endoplasmic reticulum *in vivo.* These comprise a calreticulin signal sequence, MLLSVPLLLGLLGLAAAD (SEQ ID NO 1) and the sequence of the amino acids KDEL (SEQ ID NO 11). As shown in the examples this construct proved to enable the sensor to be localised in the ER and to associate with calreticulin. Therefore another aspect of the current invention relates to a protein or protein complex that contains a calreticulin signal sequence (SEQ ID NO 1) and/or the sequence KDEL (SEQ ID NO 11; ER retention signal) and/or is folded by calreticulin and/or calnexin and/or ERp57.

The invention further relates to gene sequences coding for fusion proteins according to the present invention, i.e. a kringle domain sensor construct.

In addition, gene cassettes containing these sequences form a part of the present invention. Gene cassettes comprise in addition to the sequences coding for a kringle domain sensor, the adjacent marker protein coding sequences and auxiliary sequences to regulate the expression of the main genes. Gene cassettes may include in-trons partitioning the coding sequences, which are spliced during gene expression or cDNA (Sambrook, J., Fritsch, E.F., and Maniatis, T., in Molecular Cloning: A Laboratory Manual. Cold Spring Harbor Laboratory Press, NY, Vol. 1, 2, 3 (1989); D. Glover, DNA Cloning: A Practical Approach, Vol. 1-3, IRL Press; S. Colowick, Methods In Enzymology, Academic Press, Inc.).

Furthermore, vectors, in particular expression vectors, containing DNA or nucleotide sequences for the kringle domain sensor or gene cassettes as noted above are part of the current invention.

In order to express the kringle domain sensor in host cells, these cells are transformed or transfected with these vectors by standard means known in the state of the art. The present invention relates to a method to transfect a cell using such a vector.

Another aspect of the present invention are transfected cells, especially eukaryotic cells (e.g. endothelial cells), transfected by a vector as described above by a method as known in the state of the art.

Generally, the present invention relates to cells expressing a protein or protein complex according to the present invention.

Cells, expressing the kringle domain sensor of the present invention, can be made permeable for $Ca^{2+}$ ions whereby these cells were made usable for the detection of extracellular $Ca^{2+}$. The preferred range of the concentration of free $Ca^{2+}$ which can be detected by a cell expressing the preferred form of the sensor, the apoK sensor, is between $10^{-6}$ and $10^{-2}$ M with exceptional sensitivity (see examples). Although the permeabilising agents ionomycin and digitonin can preferably be used to allow free movement of $Ca^{2+}$ ions into and out of the cell, all methods known in the state of the art can be used to make the cell permeable for $Ca^{2+}$ ions. Cells have special voltage gated $Ca^{2+}$ channels and several methods known in the state of the art are applicable to enable $Ca^{2+}$ to pass freely through these or other types of channels, e.g. applying small voltage, weak electroporation. The present invention extends to methods employing these cells for the determination of the $Ca^{2+}$ concentration in samples.

The proteins calreticulin, calnexin and ERp57, which are active *in vivo* in the endoplasmic reticulum (ER) are natural chaperones for the folding process of kringle domain containing proteins such as apo(a), in dependence on the concentration of available $Ca^{2+}$ ions. Generally, only one chaperone (calnexin or calreticulin) together with ERp57 is necessary for the folding process. Therefore it is possible to provide a composition, comprising the kringle domain sensor according to the present invention together with at least one of these chaperones which can be used to track the folding process in any medium. Although the monitoring of the folding process *in vivo* might be of most interest it is also possible to follow the folding reaction in other media *ex vivo.* Preferably the reducing conditions (GSH/GSSG ratio) of the ER are provided additionally to the sample medium.

Another aspect of the current invention relates to a method for producing the protein or protein complex according to the present invention, characterised by the steps of

- cloning of DNA expression vectors, preferably plasmids containing the nucleotide sequence for a protein or protein complex according to the present invention;
- transfecting of cells with these expression vectors;
- expressing of the protein or protein complex and
- harvesting of the protein or protein complex.

The harvesting step can of course be performed by standard methods known in the state of the art. For uses ex vivo the kringle domain sensor of the present invention can be isolated from cell lysates using traditional techniques. Apart from these methods it is possible to design a kringle domain sensor with specific ER/export signalling sequences, which lead to the secretion of said kringle domain sensor. Normally, wild type kringle domain containing proteins are secreted by the producing cells (i.e. they do not contain the signalling sequence KDEL (SEQ ID NO 11), which traps the proteins in the ER). Using such natural signal sequences allows an easy production and isolation of the sensor protein of the current invention, which can be easily harvested from the supernatant of a cell culture. If necessary, the kringle domain sensor product is purified by standard techniques known in the state of the art.

Through the $Ca^{2+}$ dependency of calnexin and calreticulin the present sensor is capable to deliver a $Ca^{2+}$ dependent signal without actually binding $Ca^{2+}$ or interfering with the $Ca^{2+}$ distribution, especially *in vivo,* through the folding processes of apoK or homologous kringle domains. Alternatively, any metal-ion bound by calreticulin or calnexin whereby these chaperones change their activities can be detected. Furthermore, since the chaperones calreticulin or calnexin together with ERp57 form stable complexes with the kringle domain sensor it is possible to deliver a sensor protein complex which can be used to measure the $Ca^{2+}$ concentration or metal-ion concentration in a sample *ex vivo.*

Therefore the present invention relates to a method to determine the concentration of a metal ion, in particular $Ca^{2+}$, *in vivo* or in samples ex *vivo* with the help of a $Ca^{2+}$ dependent chaperone, preferably ERp57, calreticulin and/or calnexin, wherein the obtained signal of the moieties of the protein or protein complex according to the present invention provides the means to measure the concentration of the metal ion.

For example, a metal binding ligand which binds to the kringle domain e.g. if the metal ion is bound to the ligand, may be employed in this invention.

Through the detection of the folding process by adequate marker proteins as described above, the activity of calreticulin, calnexin, calreticulin complexes or calnexin complexes can be measured. Therefore the present invention also relates to a method for determining these activities.

Especially, the ER provides the natural environment for these folding processes. These vital reactions can be quantitatively measured using the kringle domain sensor of the present invention. Therefore the current invention provides a method wherein the protein or protein complex according to the present invention is folded or partially folded in the ER.

Furthermore, the current invention relates to any method which makes use of the present sensor to measure the concentration of $Ca^{2+}$ *in vivo* or in samples *ex vivo.* The association of the present sensor with these chaperone complexes (immunoprecipitation, see examples) allows the use of these complexes as a tool to assay $Ca^{2+}$ concentrations in samples ex *vivo* e.g. by FRET assay. The primary analyte for the kringle domain sensor in association with chaperones is $Ca^{2+}$. Apart from the advantage of the present sensor due to the $Ca^{2+}$ buffering capabilities of prior art sensor proteins *in vivo* also the enhanced sensitivity of the present sensor protein *(in vivo* as well *ex vivo)* in addition to the fast response is an essential improvement of the present invention over the prior art.

According to another aspect, the present invention relates to a method for determining the concentration of $Ca^{2+}$ in samples with the produced kringle domain sensor, which comprises the steps of

- contacting the sample with the protein or protein complex according to the present invention together with the chaperones calreticulin and/or calnexin and ERp57;
- exciting the first variant of GFP at a first wavelenght;
- measuring the emission of the first variant of GFP at a second wavelength (fluorescence);
- measuring the emission of the second variant of GFP at a third wavelength (FRET) and
- comparison or regression analysis of the FRET efficiency, determined by the ratio of FRET of the second GFP variant and the fluorescence of the first variant of GFP, of the sample with the FRET efficiency of standards.

Furthermore the current invention relates to a method to determine the concentration of intracellular $Ca^{2+}$, which comprises the steps of

- providing a cell wherein the present kringle domain sensor is expressed;
- detecting a different marker signal with respect to the first and second marker moieties and
- determining the $Ca^{2+}$ concentration in a cell based on said different marker signal.

By permeabilising the cells by any means described above it is further possible to use these "sensor" cells to measure the concentration of $Ca^{2+}$ in a sample. This can be facilitated with a single cell whereby the fluorescence and FRET efficiency are measured for example by laser scanning microscopy or in batch with a quantity of cells which is necessary to detect the signal.

As mentioned previously, one of the major improvements of the present invention is the circumvention of the $Ca^{2+}$ buffering effect known from sensor proteins of the prior art. Therefore the current invention relates furthermore to methods using kringle domain sensors for such an indirect determination of the concentration of $Ca^{2+}$ whereby this side-effect does not occur. The concentration of $Ca^{2+}$ is measured indirectly wherein the kringle domain sensor does not directly bind the $Ca^{2+}$ analyte. The $Ca^{2+}$ concentration is measured indirectly through the activity of the $Ca^{2+}$ dependent chaperones calnexin and/or calreticulin. Only for the short time of the folding process is a contact with the active site of the chaperone and therefore the $Ca^{2+}$ ion established. With the present invention it could be shown that the present kringle domain sensor does not react directly to the concentration of $Ca^{2+}$.

Another aspect of the present invention relates to a test kit for determining the concentration of $Ca^{2+}$ in a sample comprising a protein or protein complex according to the present invention and means to detect the marker signal, depending on the employed marker moieties. Further possible components of the test kit include a complex of calreticulin or calnexin with ERp57, standards for metal ions, preferably $Ca^{2+}$, $Mn^{2+}$, $Ni^{2+}$, $Cd^{2+}$, for calibration of the concentration of the metal-analyte and to determine the cross-reactivity of other metal ions, any one of the following reagents: reducing and/or oxidising agents, preferably Glutathion (reduced (GSH) and/or oxidised (GSSG)), buffer and any stabilising salt or agent, preferably Tris, HCl, NaCl, Triton X-100, KCl, $MgCl_2$, Hepes acid and/or EGTA. The nature of the suitable stabilising salt depends on the marker proteins or marker protein fragments employed.

Suitable means for detecting the marker signal, especially if it is a fluorescent signal, are selected from fluorescence spectrometers, fluorescence microscopes, fluorescence imaging systems, fluorescence plate readers, laser scanning microscopes, etc. For *in vivo* measurements, preferably transfected cell populations or single cells are used. The detection is thereby only limited by the sensitivity, accuracy and resolution of the measurement system in time and space and not by the sensor according to the present invention.

A special implementation of a test kit comprises stabilised cells capable of expressing the present kringle domain sensor protein, which can be used to measure the $Ca^{2+}$ concentration as described above.

In a further implementation the test kit comprises stabilised cells capable of expressing the present kringle domain sensor protein and cell permeabilizing means, preferably the agents ionomycin and/or digitonin.

In another further implementation the test kit comprises stabilised cells capable of expressing the present kringle domain sensor protein and satbilising salts or agents, preferably Tris, Hcl, NaCl, Triton X-100, KCl, $MgCl_2$, Hepes

and/or EGTA.

The present invention is further illustrated by the following examples and drawing figures, yet without being restricted thereto.

Figure 1: Schematic structure of apoK sensors. *Panel a:* Proposed model of the $Ca^{2+}$-dependent conformational shift from the linear extended to the compact structure of the type IV kringle of apo (a) within apoK1 thereby facilitating FRET to occur by reducing the distance between the donor (CFP) and acceptor (YFP) fluorophores. *Panel b:* Domain structure of apoK1 indicating the boundries and utilised restriction sites between CFP, kringle IV of apo(a) and YFP. *Panel c*: Consitition of apoK sensors and control constructs for expression and imaging in mammalian cells. Positions of the mutated amino acids are numbered according to the mature apo(a) without signal sequence. CRsig, ER signal sequence of calreticulin, MLLSVPLLLGLLGLAAAD (SEQ ID NO 1), KDEL (SEQ ID NO 11), ER retention signal; kz, Kozak sequence (SEQ ID NO 2) for optimal translation in mammalian cells. The significant mutations in the fluorescent proteins are: CFP, F46L /S65T /Y66W / N146I / M153T / V163A / N212K; and YFP, S65G /S72A /T203Y.

Figure 2: Intracellular distribution of the apoK1 constructs. Endothelial cells were transiently transfected with either apoK1-cyto *(panel a)* or apoK1-er (left *panel b)* and mt-DsRed (right *panel b).* An overlay of the apoK1-er and mt-DsRed is shown in *panel b* lower image. Distribution of the proteins was monitored at 488 nm excitation and 535 nm emission for apoK constructs and at 575 nm excitation and 630 nm emission for mt-DsRed, respectively, on an array laser scanning microscope. The bars indicate 5 $\mu$m.

Figure 3: Characterisation of apoK1-er. *Panel a:* Spectral scan of the apoK1-er fluorescence at 430 nm excitation within 420 to 550 nm emission (continuous line). Dotted and discontinuous lines indicate the fluorescence spectra of CFP and YFP, respectively. *Panel b:* Co-immunoprecipitation of apoKler with calreticulin. HEK293 cells stably expressing apoKler were lysed in the presence or absence of $Ca^{2+}$ and the sensor co-precipitated with polyclonal anti-calreticulin antibody immobilised to Prote-inG Sepharose beads. The arrow indicated 60 kDa band. The lanes show the crude lysate (CL, left), the supernatant (S, middle) and the precipitate (P, right) in the presence of 1 mM $Ca^{2+}$. *Panel* c: *In situ* concentration response curves to $Ca^{2+}$ (in the range of $10^{-6}$ to $10^{-1}$ M free $Ca^{2+}$) of apoK1-er (left; apoK1-er, continuous lines, n=14; apoK1-cyto, dotted line; n=14) and YC4-er (right; n=6) in permeabilised endothelial cells cells (1.5 $\mu$M inomycin and 10 $\mu$M digitonin).

Figure 4: ApoK1-er but not apoK1-cyto shown $Ca^{2+}$ dependent changes in its FRET efficiency as an indicator of $Ca^{2+}$-modulated formation of protein dislufide-bridges on the calreticulin/Erp57 complex in the endoplasmic reticulum. *Panel a:* Cells expressing either apoK1-er (continuous lines; n=9) or apoK1-cyto (dotted line; n=8) were permeabilised with 1.5 $\mu$M ionomycin and 10 $\mu$M digitonin and free $Ca^{2+}$ concentration was set to $10^{-3}$ or $10^{-6}$ M $Ca^{2+}$ as indicated by the bars. *Panel b:* As indicated 15 $\mu$M BHQ were added to cells expressing apoK1-er. (n=10). X-axes indicate the time in 1 min sections. *Panel c:* Effect of exhaustive $Ca^{2+}$ depletion of the endoplasmic reticulum by 15 $\mu$M BHQ in cells expressing (from left) apoK1-er (n=10) or the control constructs apoK1-cyto (n=14), apoK12-er (n=12), $^{\Delta asn}$apoK1-er (n=9) or $^{\Delta cys}$apoK1-er (n=18). The Y-axis indicates the intraluminal $Ca^{2+}$ concentration were expressed as the normalised FRET signal $\frac{Ratio(\frac{F535}{F480})}{Ratio_{min}}$.

Figure 5: ApoK1-er can be utilised as an intraluminal $Ca^{2+}$ sensor in the endoplasmic reticulum. Endothelial cells were transiently transfected with apoK1-er. After 48h, cells were mounted onto a deconvolution Imaging microscope and perfused with physiological saline. As indicated cells were stimulated with 100 $\mu$M histamine followed by a period in which extracellular $Ca^{2+}$ was removed. X-axes indicate the time in 1 min sections. Intraluminal $Ca^{2+}$ concentration were expressed as the normalised FRET signal $\frac{Ratio(\frac{F535}{F480})}{Ratio_{min}}$ (left legend) and $\mu$M free $Ca^{2+}$ (right le-gend) calculated by using the equation curve provided in figure 3c, left panel (range: 100-350 $\mu$M free $Ca^{2+}$). Data present the mean $\pm$SEM (n=9). *P<0.05 vs. baseline. #P<0.05 vs. in the presence of histamine in $Ca^{2+}$ containing solution.

Figure 6: Kringle domain alignment of different kringle domain containing proteins showing the general consensus sequence of kringle domains (SEQ ID NO 18). Sequences were obtained from the NCBI database (http://www.ncbi.nlm.nih.gov). The consensus sequence is recorded there in the entrez-CDD (conserved protein domain database) under entry No cd00108. Amino acids in capital letters depict the aligned residues, amino acids in lower case are unaligned. Of particular importance are the 6 cystein residues, found in all sequences. (Feature 1 is the binding site of epsilon-aminohexanoic acid as shown in a structure bound to human plasminogen kringle 1 (Rejante MR, Eur J Biochem. 221(3) (1994), 939-49) .)

Figure 7: Sequence of ApoK1er (SEQ ID NO 15) with references to protease digestion sites, signal sequences and protein fragment origin.

Figure 8: Sequence of ApoK2er (SEQ ID NO 13) with references to protease digestion sites, signal sequences and protein fragment origin.

EXAMPLES:

Materials and methods

DNA cloning

One (SEQ ID NOs 5 and 6) and two (SEQ ID NOs 7 and 8) kringles of apo(a) (SEQ ID NOs 16 and 17) were amplified by PCR, thereby introducing Sph I and Sac I restriction sites at their 5' and 3' ends, respectively. YC4-er and YC2.1 (Miyawaki et al., Nature 388 (1997), 882-7) were transferred into pUC19 (New England Biolabs, Frankfurt, Germany) as full length Hind III/EcoR I fragments and the calmodulin-M13 regions were exchanged for the restricted PCR products. The resultant constructs, termed apoK1/2-cyto, apoK1-er (SEQ ID NOs 14 and 15) and apoK2-er (SEQ ID NOs 12 and 13), were confirmed by automated sequencing. The same PCR-based strategy was used to generate apoK12-er, containing 12 kringles of apo(a). In order to create ER-targeted sensors devoid of all 6 cysteines or both putative N-glycosylation sites, respectively, site directed mutagenesis with primers bearing the intended mutations was performed. By use of internal restriction sites as indicated in figure 1c, apoK1-er was substituted sequentially for the respective mutated fragments. To allow introduction of the constructs via Not I/EcoR I into the mammalian expression vector pcDNA3 (Clontech, BD Biosciences, Vienna, Austria), an additional Not I site was introduced between Hind III and EcoR I and all following restriction sites were removed by blunt end ligation.

Transfection

EA.hy 926 cells at passage ≧ 40 were grown on glass cover slips to app. 80 % confluency and transiently transfected with 2 μg of plasmid two days prior to experiments (Transfast™, Promega, Vienna, Austria). To yield stable expression, HEK 293 cells transfected with 5 μg DNA per 6 cm dish were propagated in medium containing 100 μg/ml G418 and fluorescent colonies were consequently subcloned.

Immunohistochemistry

Following fixation by 4.5 % paraformaldehyde, EA.hy 926 cells grown on glass cover slips were permeabilised by 0.6 % Triton X-100 and incubated with a specific polyclonal antibody against calreticulin (Santa Cruz Biotechology Inc., Szabo Scandic, Vienna, Austria). A Cy3-labeled secondary antibody (Clontech, Vienna, Austria) was used for visualisation.

Confocal microscopy

Image acquisition was performed on an array confocal laser scanning microscope (VoxCell Scan, Visitech) consisting of a Zeiss Axiovert 200M (Vienna, Austria) and a QLC laser confocal scanning module (VisiTech, Visitron Systems, Puchheim, Germany) controlled by Metamorph 5.0 (Universal Imaging, Visitron Systems) as described in the prior art (Malli et al., J Biol Chem 278 (2003), 44769-44779) .

Spectrofluorometry

For analysis in a spectrofluorometer (Hitachi F4500; Inula, Vienna, Austria), HEK 293 cells stably expressing apoK1-er and apoK1-cyto were lysed by sonification in a buffer containing 20 mM TrisHCl, pH adjusted to 7.5, 100 mM NaCl and 0.5 % Triton X-100.

FRET measurement

Experiments were performed on a Nikon inverted microscope (Eclipse 300TE; Nikon, Optoteam, Vienna Austria) equipped with a CFI Plan Fluor 40x oil immersion objective, an epifluorescence system (Opti Quip, Highland Mills, NY, USA), computer controlled z-stage and filter wheel (Ludl Electronic Products, Hawthorne, NY, USA), and a liquid-cooled CCD-camera (Quantix KAF 1400G2, Roper Scientific, Acton, MA, USA) and controlled by Metafluor 4.0 (Visitron Systems). FRET-based sensors were exitated at 440 nm (440AF21; Omega Optical, Brattleboro, VT, USA) and emission was monitored at 480 and 535 nm using an optical beam splitter (Dual View Micro-Im-ager™; Optical Insights, Visitron Systems). Hepes-buffered solutions containing (in mM) 145 NaCl, 5 KC1, 1 $MgCl_2$, and 10 Hepes acid, pH adjusted to 7.4 with 2 $CaCl_2$ or 1 EGTA were used.

Co-immunoprecipitation

Lysates of HEK 293 cells stably expressing apoK1-er were incubated with specific antibodies against calreticulin or calnexin (Santa Cruz Biotechnology Inc.), respectively, and the immune complexes precipitated with Protein-G Sepharose 4 Fast Flow (Immunoprecipitation Starter Pack; Amersham Pharmacia Biotech) according to the manufacturer's protocol. Proteins were separated on 12.5 % SDS-polyacrylamide gels and detected by western blot analysis using chemiluminescence (Luminol, Santa Cruz Biotechnology Inc.) according to standard protocols.

Results

To achieve a high resolution and dynamic readout of protein folding, the advantage of the structural shift from a linear extended to a compact kringle conformation during the maturation of apo(a) (White et al., J Biol Chem 272 (1997) 5048-55) was taken as basis for the design of a fluorescent protein sensor for protein folding. In order to create a fusion protein, a defined number of kringle IV sequences of apo(a) were flanked by the cDNA coding for cyan mutant green fluorescent protein (CFP) and yellow mutant green fluorescent protein (YFP) at the 5'- and 3'-ends, respectively (Fig. 1a,b). We expected that during folding of the protein by the binding to the chaperone/protein disulfide isomerase complex (i.e. calreticulin/ERp57, calnexin/ERp57) a structural shortening occurs due to the formation of the kringle(s) that facilitates fluorescence resonance energy transfer (FRET) between the 5'-flanking donor (CFP) and the 3'-located acceptor (YFP) (Fig. 1a). FRET efficiency decreases by the $6^{th}$ potency of the distance between the donor and the acceptor and depends on the relative orientation of the fluorophores (Lippincott-Schwartz et al., Nat Rev Mol Cell Biol 2 (2001), 444-56), wherein the later is unpredictable and assumed to be constant. First, two constructs with either one (apoK1) or two (apoK2) kringle IV domain(s) between the two fluorophores were designed and tested to achieve good FRET efficiency upon protein folding while minor FRET should occur in the unfolded situation (Fig. 1b). The respective proteins were expressed in endothelial cells and localised in the cytosol (Fig. 2a). Because $Ca^{2+}$-dependent maturation of kringle IV at the calreticulin/ERp57 complex occurs in the ER *in vivo,* the calreticulin signal sequence, MLLSVPLLLGLLGLAAAD, and KDEL, which were successfully used for ER-targeted $Ca^{2+}$ probes (Miyawaki et al., Nature 388 (1997), 882-7; Zoratti et al., Br J Pharmacol 140 (2003), 1351-1362), were introduced at the 5'- and 3'-ends of the constructs, respectively (apoK1-er; Fig. 1b and c). As a consequence the introduction of the calreticulin signal sequence successfully achieved targeting of the kringle IV containing sensors to the ER (Fig. 2b). The structural integrity of the ER (Fig. 2b), the mitochondrial organisation and the well established network of ER and mitochondria (Fig. 2b) were not affected by overexpression of apoK1-er. Immunohistochemistry revealed co-localisation of apoK1-er with calreticulin (Fig. 3b).

In order to investigate whether the appearance of FRET from the apoK sensors of the present invention is based on the successful protein processing and folding that achieves a structural protein shortening and, thus, facilitates energy transfer between the two narrow fluorophores, several apoK variants were designed and targeted into the ER lumen. First, the two putative existing glycosylation sites of apoK1-er at positions 42 and 82 of kringle IV were mutated by an exchange of the constitutive L-asparagine to L-alanine ($^{\Delta asn}$apoK1-er; Fig. 1c). Such approach has been used frequently to prevent the initial protein glycosylation inside the ER, which is prerequisite for further processing, and, thus, to preclude that the respective protein is not subject of the protein folding machinery. Second, the existing cysteines at position 9, 30, 58, 69, 81 and 86 of kringle IV were exchanged by site directed mutagenesis to L-serine ($^{\Delta cys}$apoK1-er; Fig. 1c). Since binding of ERp57 to its substrate as well as the folding/shortening of the apo(a)/apoK sensors is thought to depend on the formation of di-sulfid bridges that built up the known kringle structure (Fig. 1a), such construct may not be suitable for $Ca^{2+}$-dependent folding at the calreticulin/ERp57 complex. Third, 12 copies of the kringle IV domain were cloned between CFP and YFP (apoK12-er; Fig. 1c) to enlarge the distance between the fluorescence donor and the putative energy acceptor and, thus, to prevent FRET to occur even after the protein was folded properly. Notably, all construct nicely targeted into the ER and did not exhibit any differences in intracellular localisation compared with the apoK1-er construct.

In HEK293 cells that stably expressed apoK1, this sensor exhibits the expected spectrum representing the overlay of the two fluorescent proteins CFP and YFP, though the spectra of apoK1-cyto and apoK1-er performed in intact cells differed from each other (Fig. 3a). In contrast to that of apoK1-cyto, the fluorescence profile of apoK1-er changed upon chelating intraluminal $Ca^{2+}$ indicating the loss of FRET by protein unfolding. ApoK1-er was isolated from stably expressing HEK293 using an affinity column carrying an antibody against apo(a). The isolated protein did not reveal any sensitivity to $Ca^{2+}$ ions up to 2 mM free $Ca^{2+}$, thus indicating that the designed protein per se does not sense intraluminal $Ca^{2+}$ of the ER. In addition, no changes in the fluorescence properties of the isolated and reduced apoK1-er protein were found if the redox potential was alternated stepwise from 0.1 mM GSSG/0 mM GSH to 0.1 mM GSSG/ 10 mM GSH. Thus, at least in the physiological redox range of the ER (Frickel et al., J Biol Chem 279 (2004), 18277-18287), the apoK1-er protein was not sensitive to changes in redox state of its environment. In line with the immunohistochemical data described above, apoK1-er protein was co-immunoprecipitated with calreticulin and ERp57, suggesting that inside the ER lumen this putative folding sensor is recognised by the prominent $Ca^{2+}$-dependent chaperone and forms a complex with the respective disulfide isomerase.

In order to verify whether the apoK1-er protein undergoes protein folding in the ER lumen of a living cell, appearance of FRET due to the shortened distance between the donor and acceptor proteins was monitored initially *in situ* by a conventional $Ca^{2+}$calibration assay. Endothelial cells were transiently transfected with either apoK1-er or apoK1-cyto. After 48 h, the cells were permeabilised by 10 $\mu$M ionomycin in the absence of extracellular $Ca^{2+}$ and intraluminal $Ca^{2+}$ concentration was stepweise elevated by addition of 1 $\mu$M to 10 mM free $Ca^{2+}$. In single ionomycin-permeabilised cells folding of apoK1-er was visualised at 480 nm excitation and 480 nm (donor fluorescence) and 535 nm (FRET) emission (Fig. 1a). Inside the ER lumen, apoK1-er folded in a $Ca^{2+}$-dependent manner where the $K_D$ was at 124.2 (120.7 -127.7) $\mu$M with a Hill coefficient of 1.148 to 1.245 (Fig. 3c, left panel). The kinetics of $Ca^{2+}$-dependent FRET appearance and disappearance was evaluated in ionomycin-permeabilised cells while intraluminal $Ca^{2+}$ was changed rapidly (within 0.2 min from 1 $\mu$M to 1 mM free $Ca^{2+}$ (Fig. 4a). Herein, the on- and offset kinetics of this sensor were calculated and revealed similar association and dissociation kinetics of apoK1-er $\tau_{on}$ = 1.563 and $\tau_{off}$ = 1.774 min). Considering such on/off kinetics, is is assumed that FRET occurs upon the $Ca^{2+}$-depending binding of apoK1-er to the calreticulin/ERp57 complex that results in protein folding, while no FRET can be further measured once the sensor is dissociated from the chaperone complex. In cells transiently expressing untargeted apoK1 in the cytosol (i.e. apoK1-cyto Fig.2a), no change in the fluorescence of the protein was found by switching from high to low $Ca^{2+}$ conditions (Fig.4a). These data indicate that the predicted $Ca^{2+}$-dependent protein shortening that facilitated the appearance of FRET between the flanking fluorophores needs the environment of the ER, presumably calreticulin and ERp57. In line with these data, an exhaustive ER depletion by 15 $\mu$M 2,5-di-tert-butylhydroquinone (BHQ), an inhibitor of sarco/endoplasmic reticulum $Ca^{2+}$ ATPases (SERCAs), reduced the FRET signal of apoK1-er and, to a lesser extent, that of apoK2-er but not that of apoK1-cyto (Fig. 4b). Moreover, in cells expressing the ER-targeted apoK12-er and the mutants $^{\Delta asn}$apoK1-er and $^{\Delta cys}$apoK1-er, which were designed to avoid either protein folding ($^{\Delta asn}$apoK1-er and $^{\Delta cys}$apoK1-er) or FRET (apoK12-er), no effect of BHQ was observed (Fig. 4c). The comparison of the measurable dynamic range of the respective shift in the wavelengths properties that point to an appearance of FRET (i.e. changes in Ratio ($F_{535}/F_{480}$)) of the apoK variants used indicate that FRET was highest in the apoK1-er construct and only there it could be modulated by chelating $Ca^{2+}$ with EGTA or $Ca^{2+}$ depletion of the ER by BHQ. Thus, the ability of apoK1-er to undergo folding, the decrease in the distance between the two fluorophores by protein shortening upon folding and its localisation in the ER are pivotal for FRET in this sensor protein.

Notably, the observed reduction of apoK1-er FRET by BHQ reached app. 70% FRET reduction compared with that found at the removal of extracellular $Ca^{2+}$ in ionomycin-permeabilised cells. These data are in line with the $Ca^{2+}$-sensitivity curve obtained for apoK1-er (Fig. 3c, left panel) that indicate that the concentration for $Ca^{2+}$ to regulate apoK1-er folding occurs ranges between $10^{-6}$ to $10^{-2}$ M free $Ca^{2+}$, which is in the physiological range of the intraluminal ER $Ca^{2+}$ concentration (Corbett et al., Trends Biochem Sci 25 (2000), 307-11). Considering the continuous ER depletion and refilling phenomena in cells that are due to chemical, physical or cell cycle-associated stimuli, it is unclear whether or not and, if so, to what extend the efficiency of protein folding in the ER underlies strictly such dynamic regulation of cellular $Ca^{2+}$ homeostasis. Utilising apoK1-er, experiments were designed to elucidate the consequences of ER $Ca^{2+}$ depletion by a physiological stimulus (i.e. histamine) on protein folding in endothelial cells. In the presence of extracellular $Ca^{2+}$, endothelial cells are capable to retain most of the ER's folding capacity even if the cells were stimulated with 100 $\mu$M histamine (Fig. 5). However, protein folding was abolished upon stimulation with histamine if extracellular $Ca^{2+}$ was removed (Fig. 5) due to the fact that under such conditions, ER $Ca^{2+}$ content cannot be maintained anymore. These findings suggest that under physiological conditions, $Ca^{2+}$-dependent protein folding in the ER is ensured even under agonist stimulation. Nevertheless, the degree of $Ca^{2+}$ depletion of the ER by physiological paracrines, such like inositol 1,4,5-trisphosphate-generating substances (e.g. acetylcholine, ATP, ADP, bradykinin or histamine) may differ between various cell types and thus the statement needs further approval in other cells. Another important aspect that is brought to attention is that obviously ER protein folding, at least that by calreticulin/ERp57, not only needs extracellular $Ca^{2+}$ as a source for ER refilling during agonist stimulation (Malli et al., J Biol Chem 278 (2003), 44769-44779) but also highly depends on an intact $Ca^{2+}$ entry machinery of the cells. In view of the numerous reports that described the pathological attenuation of cellular $Ca^{2+}$ entry pathways in e.g. endothelial cells in many human diseases such like atherosclerosis, diabetes mellitus or hypertension, one might expect alterations in ER protein folding in such diseases. In line with this suggestion, delayed/disturbed protein folding has been reported at least in atherosclerosis and diabetes mellitus (Csermely et al., Trends Genet 17 (2001), 701-4).

In comparison to the measurements of intraluminal $Ca^{2+}$ by FRET using the consensus binding site of myosine light chain kinase, M13, as folding protein, as used in YC4-er/vYC4-er (Zoratti et al., Br J Pharmacol 140 (2003), 1351-1362; Malli et al., J Biol Chem 278 (2003), 44769-44779), with that of ER protein folding with apoK1-er, several improvements of the present invention are made obvious. Notably, both the vYC4-er and the original YC4-er utilise FRET that appears upon binding of $Ca^{2+}$/E31Q-calmodulin (a mutant with lower $Ca^{2+}$sensitivity) to the consensus binding site of myosine light chain kinase, M13, similar to US patent no. 6,376,257. The $Ca^{2+}$calibration curve for YC4-er in endothelial cells (Fig. 3c, right panel) indicates a $K_D$ of 344.6 (325.4-365.0) μM, which is similar to that published in HeLa cells (Arnaudeau et al., J Biol Chem 276 (2001), 29430-9). While this $Ca^{2+}$sensor is an useful tool for biological research, it introduces a $Ca^{2+}$ binding protein into the lumen of the ER and, thus, elevates ER $Ca^{2+}$ buffering capacity inside this organelle. Since such property enhances the overall $Ca^{2+}$ storage capacity of the ER, it affects secondary $Ca^{2+}$-mediated function inside the ER (Miyakawa et al., Embo J 18(1999), 1303-8). Moreover, elevating the ER's $Ca^{2+}$ buffering capacity influences the intraluminal $Ca^{2+}$homeostasis and the inter-organelle $Ca^{2+}$ signaling with e.g. the mitochondria. Particularly in view of the proposed regulatory function of intraluminal $Ca^{2+}$ for activa-tion/termination of the so called capacitative $Ca^{2+}$ entry (Nilius et al., Endothelium 10 (2003), 5-15) and the importance of the inter-organelle $Ca^{2+}$ crosstalk, $Ca^{2+}$ sensors that affect intraluminal $Ca^{2+}$ buffer capacity might be critical. On the other side, apoK1-er does not contain any $Ca^{2+}$ binding domain but binds to constitutive calreticulin/ERp57 in a $Ca^{2+}$-dependent manner and, thus, also measures ER $Ca^{2+}$ concentration. Therefore, this property can be further utilised to monitor the free $Ca^{2+}$ concentration inside the ER without manipulating the $Ca^{2+}$ buffering/storage capacity of this organelle. Importantly, in the physiological range of the ER $Ca^{2+}$ concentration (i.e. 10-700 μM) apoK1-er exhibits a more steep $Ca^{2+}$ dependency compared with YC4er and thus, is even more sensitive to small $Ca^{2+}$ changes/fluctuations inside the ER.

Discussion

In the prior art, the question how the formation of disulfide bridges during $Ca^{2+}$-dependent protein folding by calreticulin and ERp57 inside the ER is controlled and ensured even under rhythmic or tonic ER $Ca^{2+}$ depletion could not be investigated in detail so far as the techniques available for monitoring protein folding were essentially disruptive and/or built on the use of large cell populations. Therefore their temporal and spatial resolution is limited and does not allow continuous monitoring of folding processes within single cells or even cellular compartments. With the structural shift from a linear extended to a compact kringle conformation during the maturation of apo(a) a high resolution and dynamic readout of disulfide bridge formation at a chaperone complex was achieved with the presented design of a fluorescent protein sensor for the activity of the calreticulin/ERp57 complex in the ER. This sensor principle is superior over the prior art in regard of the $Ca^{2+}$ buffering effect observed with prior methods. The formation of stable complexes of apoK sensors with the chaperone complex further allows the possibility for a method to measure the $Ca^{2+}$ concentration in samples *ex vivo*. At last the possibility to use a prepared cells which are permeable to $Ca^{2+}$ ions provide the means to create highly sensible fluorescent sensors based on the presented principle.

**Claims**

1. Protein or protein complex comprising a first marker moiety, at least one kringle protein domain and a second marker moiety, wherein

- said first and second marker moiety is attached to said kringle protein domain(s);
- said kringle domain(s) having a first and a second conformation state, and
- said first conformation state and said second conformation state have a different marker signal with respect to said first and second marker moiety.

2. Protein or protein complex according to claim 1, wherein the kringle domain(s) is(are) any or any combination of kringle domain(s) of the apolipoprotein(a).

3. Protein or protein complex according to claims 1 or 2, wherein the kringle domain(s) are kringle IV domain(s) of any subtype of the apolipoprotein(a).

4. Protein or protein complex according to any one of claims 1 to 3, wherein the kringle domain(s) is(are) kringle IV type 2 domain(s) of the apolipoprotein(a).

5. Protein or protein complex according to any one of claims 1 to 4, wherein the kringle domain(s) comprises a sequence having sequence identity of at least 70%, 80%, 90% or 99% to any kringle IV domain of apolipoprotein(a).

6. Protein according to any one of claims 1 to 5, wherein the protein is a fusion protein comprising a first and a second marker protein or marker protein fragment as first and second marker moiety, said first marker protein or marker protein fragment, said kringle protein domain(s) and said second marker protein or marker protein complex are linked in this order.

7. Protein or protein complex according to any one of claims 1 to 6, wherein the markers signal is a distance dependant signal based on FRET (fluorescence resonance emission transfer), BRET (bioluminescence resonance emission transfer) or LRET (luminescence resonance emission transfer).

8. Protein or protein complex according to any one of claims 1 or 7, wherein the marker moieties are mutant forms or wild types of the green fluorescent protein (GFP).

9. Protein or protein complex according to claim 8, wherein the first marker protein is the cyan fluorescent protein (CFP) mutant of GFP and/or the second marker protein is the yellow fluorescent protein (YFP) mutant of the GFP.

10. Protein or protein complex according to claim 8, wherein the first marker protein is the enhanced cyan fluorescent protein (ECFP) and/or the second marker protein is the enhanced yellow fluorescent protein (EYFP).

11. Protein or protein complex according to any one of claims 1 to 10, **characterised in that** it is expressed in eukaryotic cells, in particular endothelial cells, by transformation or transfection of said cell.

12. Protein or protein complex according to claims 11, **characterised in that** it contains a signalling sequence, which directs the protein or protein complex into the lumen of the endoplasmic reticulum ER.

13. Protein or protein complex according to any one of claims 1 to 12, **characterised in that** it contains a calreticulin signal sequence and/or the sequence KDEL and/or is folded by calreticulin and/or calnexin and/or ERp57.

14. DNA or nucleic acid sequence of proteins or protein fragments according to any of claims 6 to 13.

15. Gene cassette comprising at least one of the DNA or nucleic acid sequences according to claim 14.

16. Vector, preferably an expression vector including a DNA or nucleotide sequence or gene cassette according to claim 14 or 15.

17. Method to transform a cell using a vector according to claim 16.

18. Transformed cell, especially eukaryotic cell or endothelial cell, transformed by the method according to claim 17.

19. Eukaryotic cell, especially endothelial cell, wherein the protein or protein complex according to any one of claims 6 to 13 is expressed.

20. Method for producing the protein or protein complex according to any one of claims 6 to 13 **characterised by** the following steps

- cloning of DNA expression vectors, preferably plasmids containing a nucleotide sequence according to claims 14 or 15
- transfecting of cells with these expression vectors
- expressing of the protein or protein complex
- harvesting of the protein or protein complex.

21. Method to deterime the concentration of a metal ion, in particular $Ca^{2+}$, *in vivo* or in samples *ex vivo* with the help of a $Ca^{2+}$ dependent chaperone, preferably calreticulin, ERp57 and/or calnexin, wherein the obtained signal of the marker protein(s) of the protein or protein complex according to any one of claims 1 to 13 provides the means to measure the concentration of the metal ion.

22. Method for determining the activity of calreticulin, calnexin, calreticulin complexes or calnexin complexes, wherein the protein or protein complex according to any one of claims 1 to 13 is used to determine the activity of calreticulin, calnexin, calreticulin complexes or calnexin complexes.

23. Method wherein the protein or protein complex according to any one of claims 11 to 13 is folded or partially folded in the ER.

24. Method for determining the concentration of $Ca^{2+}$ in a sample ex *vivo* by fluorescence resonance energy transfer (FRET), comprising

- contacting the sample with a protein or protein complex according to any one of claims 1 to 13 together with the chaperones calreticulin and/or calnexin and ERp57;
- exciting the first variant of GFP at a first wavelenght;
- measuring the emission of the first variant of GFP at a second wavelength (fluorescence);
- measuring the emission of the second variant of GFP at a third wavelength (FRET) and
- comparison or regression analysis of the FRET efficiency, determined by the ratio of FRET of the second GFP variant and the fluorescence of the first variant of GFP, of the sample with the FRET efficiency of standards.

25. Method for monitoring intracellular $Ca^{2+}$ concentration in a cell, **characterised by** the following steps

- providing a cell according to claim 19 or introducing the protein or protein complex according to any one of claims 1 to 13 into a cell;
- detecting a different marker signal with respect to the first and second marker protein or marker protein fragment and
- determining the $Ca^{2+}$ concentration in a cell based on said different marker signal.

26. Method for determining the concentration of $Ca^{2+}$ in samples ex *vivo* employing the method according to claim 25 wherein the cell or cells are made permeable to $Ca^{2+}$ ions in the sample.

27. Method according to any one of claim 24 to 26, wherein the $Ca^{2+}$ concentration is determined indirectly, through the activity of the $Ca^{2+}$ dependent chaperones calnexin and/or calreticulin.

28. Test kit for determining the concentration of $Ca^{2+}$ in a sample comprising a protein or protein complex according to any one of claims 1 to 13 and means to detect the marker signal.

29. Test kit according to claim 28 further comprising a complex of calreticulin or calnexin with ERp57.

30. Test kit according to claim 28 further comprising standards for metal ions, preferably $Ca^{2+}$, $Mn^{2+}$, $Ni^{2+}$, $Cd^{2+}$, for calibration of the concentration of the metal-analyte and to determine the cross-reactivity of other metal ions.

31. Test kit according to claim 28 further comprising any one of the following reagents: reducing and/or oxidising agents, preferably Glutathion (reduced (GSH) and/or oxidised (GSSG)), buffer and any stabilising salt or agent, preferably Tris, HCl, NaCl, Triton X-100, KCl, $MgCl_2$, Hepes acid and/or EGTA.

**32.** Test kit for determining the concentration of $Ca^{2+}$ in a sample comprising cell(s) according to claim 19 and means to detect the marker signal.

**33.** Test kit according to claim 32 further comprising cell permeabilising agents, preferably ionomycin and/or digitonin.

**34.** Test kit according to claim 32 further comprising any stabilising salt or agent, preferably Tris, HCl, NaCl, Triton X-100, KCl, $MgCl_2$, Hepes acid and/or EGTA.

Figure 1

a

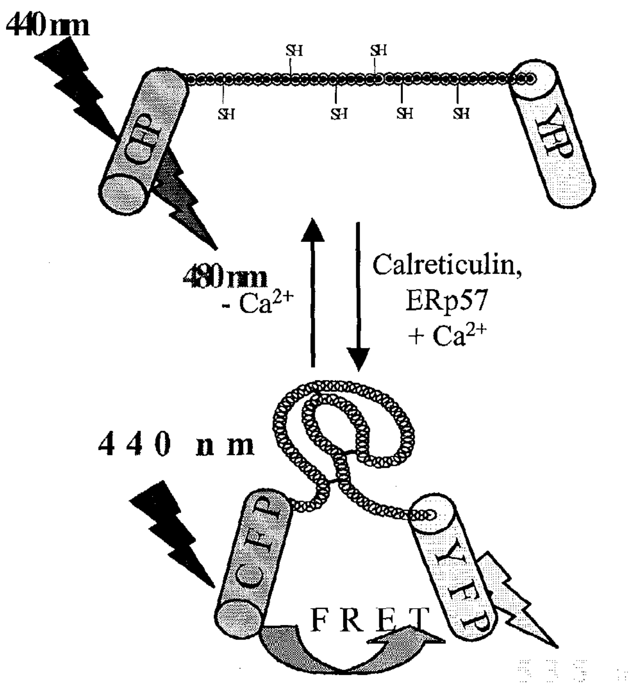
440 nm
SH
SH
SH
SH
SH
SH
CFP
YFP
480 nm
- Ca2+
Calreticulin,
ERp57
+ Ca2+
4 4 0 n m
CFP
YFP
FRET

b

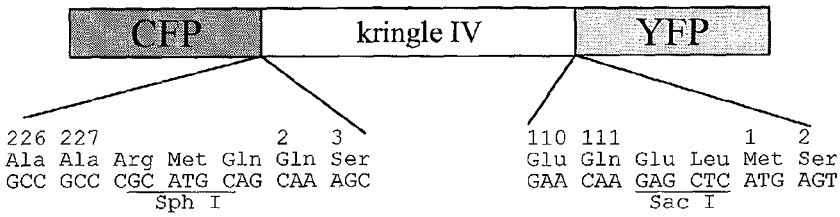
CFP
kringle IV
YFP
226 227 2 3
Ala Ala Arg Met Gln Gln Ser
GCC GCC CGC ATG CAG CAA AGC
Sph I
110 111 1 2
Glu Gln Glu Leu Met Ser
GAA CAA GAG CTC ATG AGT
Sac I

c

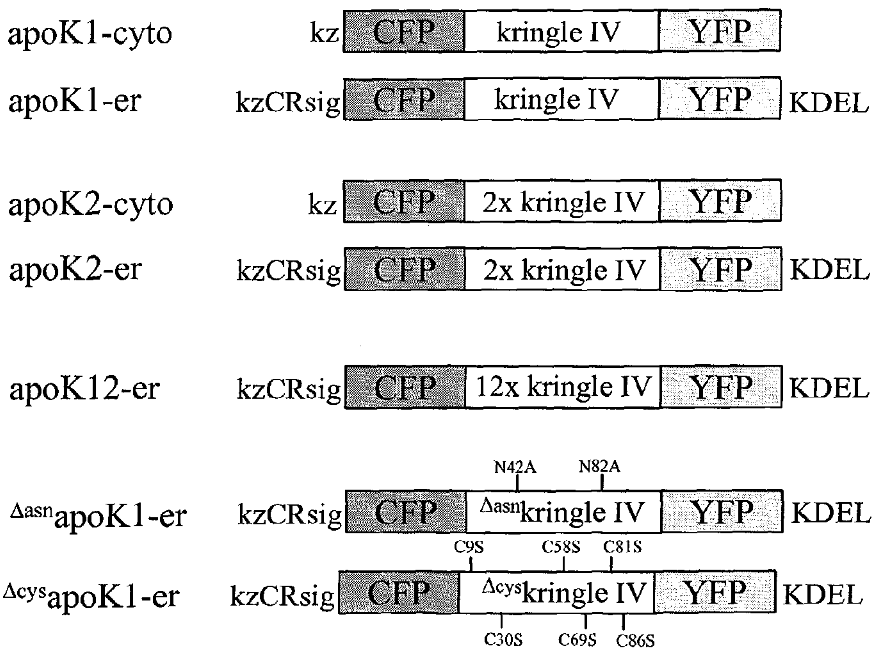
apoK1-cyto
kz CFP kringle IV YFP
apoK1-er
kzCRsig CFP kringle IV YFP KDEL
apoK2-cyto
kz CFP 2x kringle IV YFP
apoK2-er
kzCRsig CFP 2x kringle IV YFP KDEL
apoK12-er
kzCRsig CFP 12x kringle IV YFP KDEL
N42A N82A
ΔasnapoK1-er
kzCRsig CFP Δasnkringle IV YFP KDEL
C9S C58S C81S
ΔcysapoK1-er
kzCRsig CFP Δcyskringle IV YFP KDEL
C30S C69S C86S

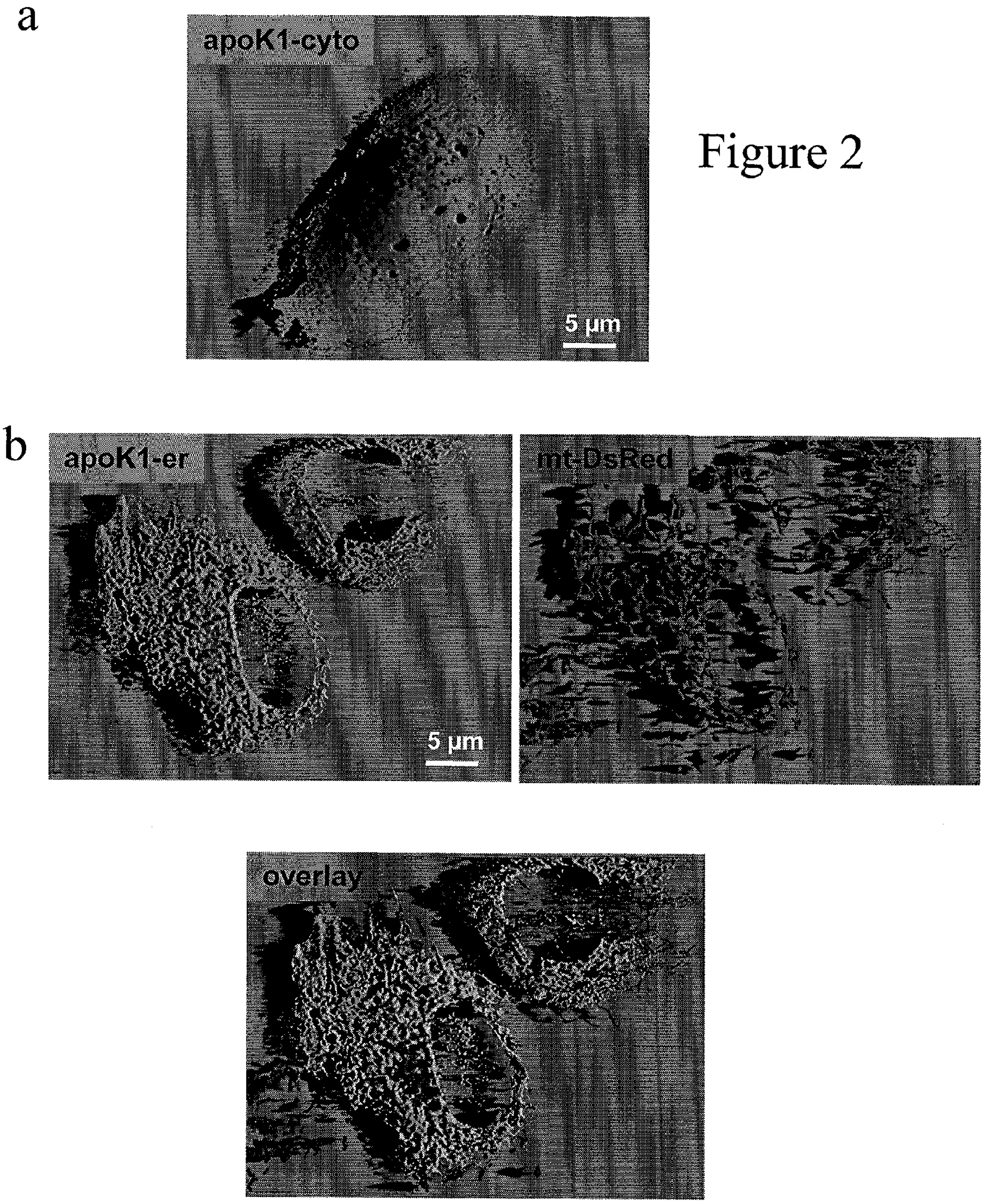


Figure 2

a

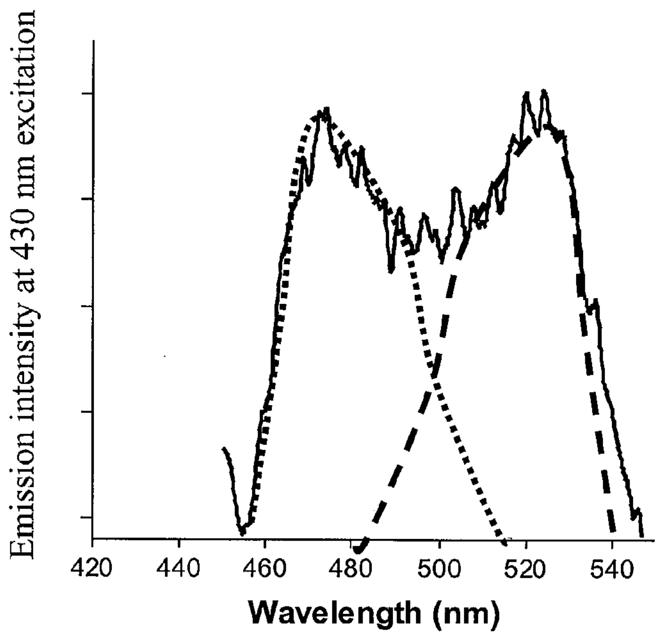
Emission intensity at 430 nm excitation
420
440
460
480
500
520
540
Wavelength (nm)

b

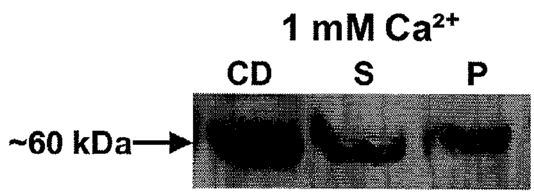
1 mM Ca2+
CD
S
P
~60 kDa

Figure 3

c

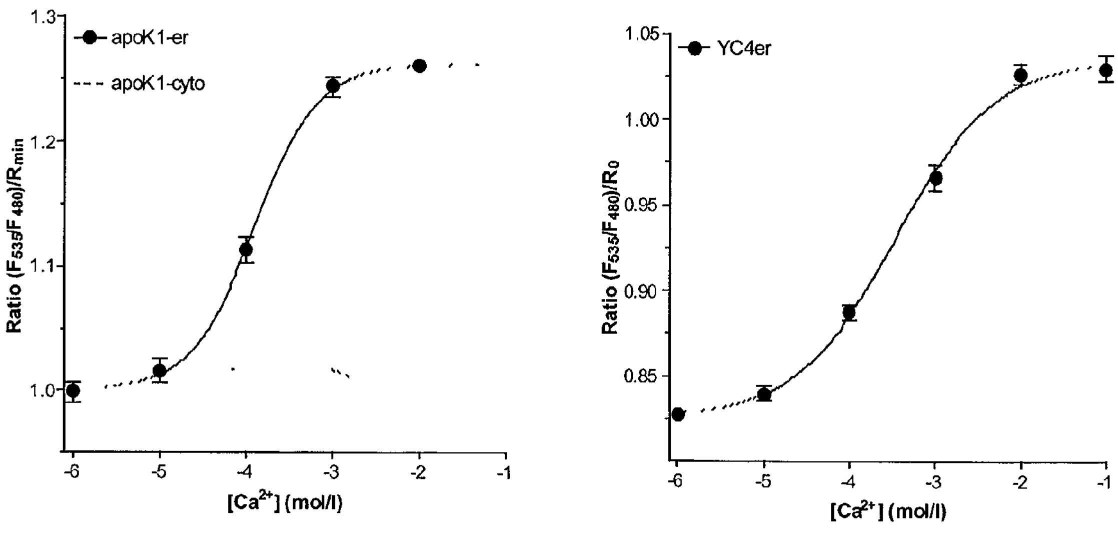
apoK1-er
apoK1-cyto
Ratio (F535/F480)/Rmin
[Ca2+] (mol/l)
YC4er
Ratio (F535/F480)/R0
[Ca2+] (mol/l)

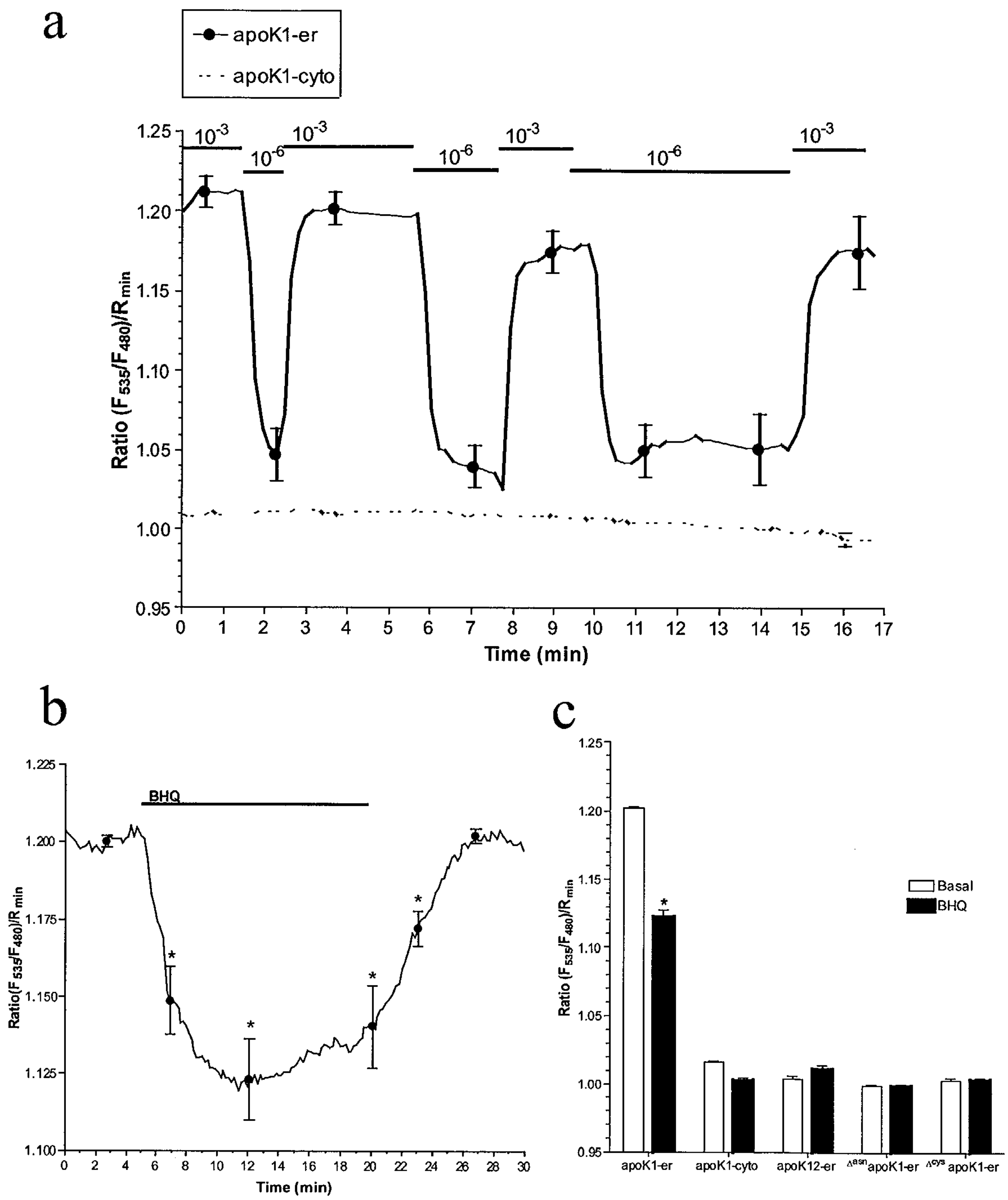
a
apoK1-er
apoK1-cyto
10-3
10-6
Ratio (F535/F480)/Rmin
Time (min)
b
BHQ
Ratio(F535/F480)/Rmin
Time (min)
c
Basal
BHQ
Ratio (F535/F480)/Rmin
apoK1-er
apoK1-cyto
apoK12-er
Δasn apoK1-er
Δcys apoK1-er

Figure 4

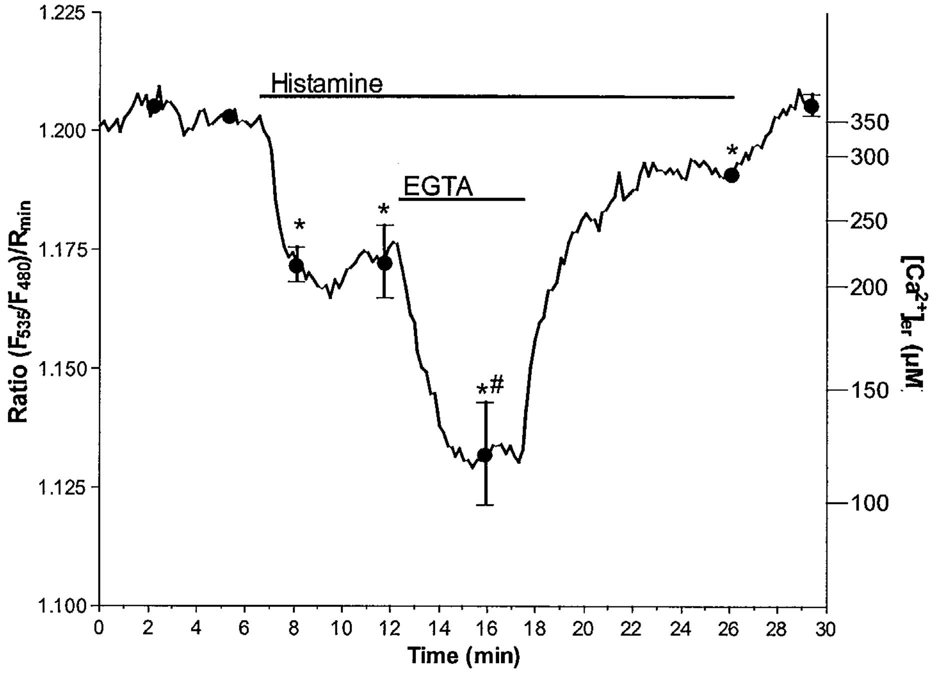

Histamine
EGTA
*
*
*#
*
1.225
1.200
1.175
1.150
1.125
1.100
Ratio (F535/F480)/Rmin
0
2
4
6
8
10
12
14
16
18
20
22
24
26
28
30
Time (min)
350
300
250
200
150
100
[Ca2+]er (µM)


Figure 5

# Figure 6

kringle domain consensus sequence and 38 aligned sequences

```
                       10        20        30        40        50        60        70        80
                   ....*....|....*....|....*....|....*....|....*....|....*....|....*....|....*....|
Feature 1                                      #             #                                  #
consensus       1 TRDCYWGn--GESYRGTVSTTKSGKPCQRWns-----qlPHQHKFNPerfp----eglLEENYCRNPDGDp----EGPWC 65
1NK1_A         98 IRNCIIGk--GRSYKGTVSITKSGIKCQPWss-----miPHEHSFLPssyr----gkdLQENYCRNPRGEe----GGPWC 162
1B2I_A          2 SEECMHGs--GENYDGKISKTMSGLECQAWds-----qsPHAHGYIPskfp----nknLKKNYCRNPDRE-----LRPWC 65
1A0H_A          4 LETCVPDr--GREYRGRLAVTTHGSRCLAWsseq-akalSKDQDFNPav--------pLAENFCRNPDGDe----EGAWC 68
1KRN            1 VQDCYHGd--GQSYRGTSSTTTTGKKCQSWss-----mtPHRHQKTPenyp----nagLTMNYCRNPDAD-----KGPWC 64
1CEA_B          1 LSECKTGn--GKNYRGTMSKTKNGITCQKWss-----tsPHRPRFSPathp----segLEENYCRNPDNDp----QGPWC 65
2PF2           63 EGNCAEGv--GMNYRGNVSVTRSGIECQLWrs-----ryPHKPEINSt thp---gadLRENFCRNPDGSi----TGPWC 127
1URK           42 SKTCYEGn--GHFYRGKASTDTMGRPCLPWns-----atVLQQTYHAhrsda-lqlglGKHNYCRNPDNR-----RRPWC 108
gi 123114     367 PQDCYHGa--GEQYRGTVSKTRKGVQCQRWsa-----etPHKPQFTFtsep----haqLEENFCRNPDGDs----HGPWC 431
gi 123114     107 VRTCIMNn--GVGYRGTMATTVGGLPCQAWsh-----kfPNDHKYTPtlr------ngLEENFCRNPDGDp----GGPWC 169
gi 1399751    115 IRDCVAGn--GNTYRGTVSKTKSGRTCQRWrl-----kfPHDHKFSPihw------peLEENYCRNPDSDp----EGPWC 177
gi 9989696    280 YHQCYNGs--GMDYRGTASTTKSGHQCQPWal-----qhPHSHHLSStdfp----elgGGHAYCRNPGGQm----EGPWC 344
gi 2507247    279 KYQCLKGt--GKNYGGTVAVTESGHTCQRWse-----qtPHKHNRTPenfp----cknLEENYCRNPNGE-----KAPWC 342
gi 4826868    310 NHKCYNSt--GVDYRGTVSVTKSGRQCQPWns-----qyPHTHTFTAlrfp----elnGGHSYCRNPGNQk----EAPWC 374
gi 2507247    482 EADCMIGt--GKSYRGKKATTVAGVPCQEWaa-----qePHQHSIFTpetn---pqsgLERNYCRNPDGDv----NGPWC 547
gi 123116     208 EVECMTCn--GESYRGLMDHTESGKICQRWdh-----qtPHRHKFLPeryp----dkgFDDNYCRNPDGQ-----PRPWC 271
gi 422541     461 TRMCYNNn--GRFYQGSVNVTASGISCQRWse-----qaPHFHRRLPeifp----elaNSDNFCRNPGGEs----ERPWC 525
gi 4758502    191 SDDCYVGd--GYSYRGKMNRTVNQHACLYWns-----hlLLQENYNMfmeda-ethgiGEHNFCRNPDAD-----EKPWC 257
gi 290665      14 DVPCVRRe--GRDYRGDLNITWTGKPCLPWrgsy-snflPSQFTTAG-----------LTSNYCRNPDGDs----EGVWC 75
gi 547643     283 DERCFLGn--GTGYRGVASTSASGLSCLAWns------dLLYQELHVdsvgaaallglGPHAYCRNPDND-----ERPWC 349
gi 289825     204 GQPCESEk--GMLYTGTLSVTVSGARCLPWasek-akalLQDKTINPev--------kLLENYCRNPDADd----EGVWC 268
gi 123116     302 TTECIQGq--GEGYRGTVNTIWNGIPCQRWds-----qyPHEHDMTPenfk----ckdLRENYCRNPDGS-----ESPWC 365
gi 123114     280 TVSCFRGk--GEGYRGTANTTTAGVPCQRWda-----qiPHQHRFTPekya----ckdLRENFCRNPDGS-----EAPWC 343
gi 2499860    100 LSECKVGn--GKYYRGTVSKTKTGLTCQKWsa-----etPHKPRFSPdenp----segLDQNYCRNPDNDp----KGPWC 164
gi 123114     188 EAACVWCn--GEEYRGAVDRTESGRECQRWdl-----qhPHQHPFEPgkfl----dqgLDDNYCRNPDGS-----ERPWC 251
gi 1399751    374 EPDCYHGn--GELYSGRVSKTRKGIKCRRWeekrndlelSLDQPYLVp----------LEENYCRNPDRDs----HGPWC 437
gi 1480911     20 NSECFDIe-nPESYQGAISRTLGGETCQSWdl-----qtPHKHKYTSsnyp---nsglAGNNYCRNPDQDw----RGPWC 86
gi 1130676    105 LRECIVAn--GTSYRGTRDTTERGLRCQHWqa-----ttPHDHRFLPslr------ngLEENYCRNPDRDk----RGPWC 167
gi 123116     388 GQDCYRGn--GKNYMGNLSQTRSGLTCSMWdk-----nmEDLHRHIFwepd----askLNENYCRNPDDDa----HGPWC 452
gi 2116554    200 SYDCRNGn--GRFYMGTMNVSKSGIPCQRWdt-----qyPHKHFQPPlvfh----qllEGENYCRNAGGEe----PHPWC 264
gi 1352406    204 KASCYDDrdrGLSYRGMAGTTLSGAPCQSWaseatywnvTAEQVLNWg---------lGDHAFCRNPDND-----TRPWC 269
gi 2499868    216 DGDCYTGn--GLAYRGTRSHTKSGASCLPWnsvfltskiYTAWKSNApal------glGKHNHCRNPDGD-----AQPWC 282
gi 289825     106 EGNCAANl--GQNYRGTINYTKSGIECQVWts-----kyPHIPKFNAsiy------pdLTENYCRNPDNNs----EGPWC 168
gi 1079221     78 TTACVKGt--GEGYRGTAALTVSGKACQAWas------qTPGDVYSCq---------gLVSNYCRNPDGE-----KLPWC 135
gi 542586     234 TENCYWEd--GSTYRGVANVSASGKPCLRWsw------lMKEISDFPe---------lIGQNYCRNPGSVe----NSPWC 292
gi 1351379    125 RATCYEGq--GVTYRGTWSTAESRVECINWns-----slLTRRTYNGrmpda-fnlglGNHNYCRNPNGA-----PKPWC 191
gi 6434248    325 THWCYVNs--GTQYEGTVAQTSSGKQCAPWi-------dSTSRDFNVhrfp----elmNSKNYCRNPGGKk----SRPWC 387
gi 7495617    219 GRECIAR---DKEYSGTANRTSDGKMCLMWndp----tvLFRRERDLe---------iLNHNFCRFVNDAdgkksATPVC 282
gi 2078470     22 SGGCFWDn--GHLYREDQTSPAPGLRCLNWlda---qsgLASAPVSGa----------GNHSYCRNPDEDp----RGPWC 82
```

# Figure 6 cont.

```
                         90        100
                   ....*....|....*....|
Feature 1          #         #
consensus      66 YTtdp--nvrWEYCDIPRCE 83
1NK1_A        163 FTsnp--evrYEVCDIPQCS 180
1B2I_A         66 FTtdp--nkrWELCDIPRCT 83
1A0H_A         69 YVadq--pgdFEYCDLNYCE 86
1KRN           65 FTtdp--svrWEYCNLKKCS 82
1CEA_B         66 YTtdp--ekrYDYCDILECE 83
2PF2          128 YTtsp--tlrREECSVPVCG 145
1URK          109 YVqvgl-kplVQECMVHDCA 127
gi 123114     432 YTmdp--rtpFDYCALRRCA 449
gi 123114     170 YTtdp--avrFQSCGIKSCR 187
gi 1399751    178 YTtdk--nirHQYCGIKKCE 195
gi 9989696    345 FTqnk--nvrMELCDVPSCS 362
gi 2507247    343 YTtns--evrWEYCTIPSCE 360
gi 4826868    375 FTlde--nfkSDLCDIPACD 392
gi 2507247    548 YTmnp--rkpFDYCDVPQCE 565
gi 123116     272 YTldp--htrWEYCAIKTCA 289
gi 422541     526 YTmdr--dirWEFCNVPQCI 543
gi 4758502    258 FIkvtndkvkWEYCDVSACS 277
gi 290665      76 YTkgve-gtdVDYCQLNYCE 94
gi 547643     350 YVvkds-alsWEYCRLEACE 368
gi 289825     269 VIdep---pyFEYCDLHYCD 285
gi 123116     366 FTtdp--nirVGYCSQIPNC 383
gi 123114     344 FTlrp--gmrAAFCYQIRRC 361
gi 2499860    165 YTmdp--evrYEYCEIIQCE 182
gi 123114     252 YTtdp--qieREFCDLPRCG 269
gi 1399751    438 YTmdp--ntpFDYCAIKPCE 455
gi 1480911     87 YTtne--fmrWDYCDIPICS 104
gi 1130676    168 YTvdp--nvrHQSCGIKKCE 185
gi 123116     453 YTgnp--lipWDYCPISRCE 470
gi 2116554    265 YTvde--svrWQHCDIPMCP 282
gi 1352406    270 FIwkgd-rlsWNYCRLAPCQ 288
gi 2499868    283 HVwkdr-qltWEYCDVPQCV 301
gi 289825     169 YTrdp--tveREECPIPVCG 186
gi 1079221    136 YT--------TEYCNVPSCT 147
gi 542586     293 FVdssr-eriIELCDIPKCA 311
gi 1351379    192 YVikag-kftSESCSVPVCS 210
gi 6434248    388 YSkpm---gqEEYCDVPQCP 404
gi 7495617    283 YTkp----neLSQCYIPPCP 298
gi 2078470     83 YVsgeagvpeKRPCEDLRCP 102
```

# Figure 7

## ApoK1er

```
     Hind III    Not I                       ER signal sequence of calreticulin
   1 AAG CTT GCG GCC GCC ACC ATG CTG CTG CCC GTC CCC CTG CTG CTG GGC CTG CTG   54
   1                 Kozak    M   L   L   P   V   P   L   L   L   G   L   L   13

                                             Enhanced cyan fluorescent protein (ECFP)
  55 GCC GCC GCC GAC GTG GGC AGC AAG GGC GAG GAG CTG TTG ACC GGG GTG GTG CCC  108
  14  A   A   A   D   V   G   S   K   G   E   E   L   L   T   G   V   V   P   31

 109 ATC CTG GTC GAG CTG GAC GGC GAC GTA AAC GGC CAC AGG TTC AGC GTG TCC GGC  162
  32  I   L   V   E   L   D   G   D   V   N   G   H   R   F   S   V   S   G   49

 163 GAG GGC GAG GGC GAT GCC ACC TAC GGC AAG CTG ACC CTG AAG TTC ATC TGC ACC  216
  50  E   G   E   G   D   A   T   Y   G   K   L   T   L   K   F   I   C   T   67

 217 ACC GGC AAG CTG CCC GTG CCC TGG CCC ACC CTC GTG ACC ACC CTG ACC TGG GGC  270
  68  T   G   K   L   P   V   P   W   P   T   L   V   T   T   L   T   W   G   85

 271 GTG CAG TGC TTC AGC CGC TAC CCC GAC CAC ATG AAG CAG CAC GAC TTC TTC AAG  324
  86  V   Q   C   F   S   R   Y   P   D   H   M   K   Q   H   D   F   F   K  103

 325 TCC GCC ATG CCC GAA GGC TAC GTC CAG GAG CGC ACC ATC TTC TTC AAG GAC GAC  378
 104  S   A   M   P   E   G   Y   V   Q   E   R   T   I   F   F   K   D   D  121

 379 GGC AAC TAC AAG ACC CGC GCC GAG GTG AAG TTC GAG GGC GAC ACC CTG GTG AAC  432
 122  G   N   Y   K   T   R   A   E   V   K   F   E   G   D   T   L   V   N  139

 433 CGC ATC GAG CTG AAG GGC ATC GAC TTG AAG GAG GAC GGC AAC ATC CTG GGG CAC  486
 140  R   I   E   L   K   G   I   D   L   K   E   D   G   N   I   L   G   H  157

 487 AAG CTG GAG TAC AAC TAT ATC AGC CAC AAC GTC TAT ATC ACC GCC GAC AAG CAG  540
 158  K   L   E   Y   N   Y   I   S   H   N   V   Y   I   T   A   D   K   Q  175

 541 AAG AAC GGC ATC AAG GCC CAC TTC AAG ATG CGC CAC AAC ATC GAG GAC GGC AGC  594
 176  K   N   G   I   K   A   H   F   K   M   R   H   N   I   E   D   G   S  193

 595 GTG CAG GTG GCC GAC CAC TAC CAG CAG AAC ACC CCC ATC GGC GAC GGC CCC GTG  648
 194  V   Q   V   A   D   H   Y   Q   Q   N   T   P   I   G   D   G   P   V  211

 649 CTG CTG CCC GAC AAC CAC TAC CTG AGC ACC CAG TCC GCC CTG AGC AAA GAC CCC  702
 212  L   L   P   D   N   H   Y   L   S   T   Q   S   A   L   S   K   D   P  229

                                                                      Sph I
 703 AAC GAG AAG CGC GAT CAC ATG GTC CTG CTG GAG TTC GTG ACC GCC GCC CGC ATG  756
 230  N   E   K   R   D   H   M   V   L   L   E   F   V   T   A   A   R   M  247

                         kringle of Apo(a)
 757 CAG CAA AGC CAT GTG GTC CAG GAT TGC TAC CAT GGT GAT GGA CAG AGT TAT CGA  810
 248 E→Q  Q   S   H   V   V   Q   D   C   Y   H   G   D   G   Q   S   Y   R  265

 811 GGC ACG TAC TCC ACC ACT GTC ACA GGA AGG ACC TGC CAA GCT TGG TCA TCT ATG  864
 266  G   T   Y   S   T   T   V   T   G   R   T   C   Q   A   W   S   S   M  283

 865 ACA CCA CAT CAA CAT AAT AGG ACC ACA GAA AAC TAC CCA AAT GCT GGC TTG ATC  918
 284  T   P   H   Q   H   N   R   T   T   E   N   Y   P   N   A   G   L   I  301

 919 ATG AAC TAC TGC AGG AAT CCA GAT GCT GTG GCA GCT CCT TAT TGT TAT ACG AGG  972
 302  M   N   Y   C   R   N   P   D   A   V   A   A   P   Y   C   Y   T   R  319

 973 GAT CCC GGT GTC AGG TGG GAG TAC TGC AAC CTG ACG CAA TGC TCA GAC GCA GAA 1026
 320  D   P   G   V   R   W   E   Y   C   N   L   T   Q   C   S   D   A   E  337

1027 GGG ACT GCC GTC GCG CCT CCG ACT GTT ACC CCG GTT CCA AGC CTA GAG GCT CCT 1080
 338  G   T   A   V   A   P   P   T   V   T   P   V   P   S   L   E   A   P  355
```

# Figure 7 cont.

```
                     Sac I                        Enhanced yellow fluorescent protein (EYFP)
1081  TCC GAA CAA GAG CTC ATG GTG AGC AAG GGC GAG GAG CTG TTC ACC GGG GTG GTG 1134
 356   S   E   Q   E   L   M   V   S   K   G   E   E   L   F   T   G   V   V   373

1135  CCC ATC CTG GTC GAG CTG GAC GGC GAC CTA AAC GGC CAC AAG TTC AGC GTG TCC 1188
 374   P   I   L   V   E   L   D   G   D   L   N   G   H   K   F   S   V   S   391

1189  GGC AGA GGC GAG GGC GAT GCC ACC TAC GGG AAG CTG ACC CTG AAG TTC ATC TGC 1242
 392   G   R   G   E   G   D   A   T   Y   G   K   L   T   L   K   F   I   C   409

1243  ACC ACC GGC AAG CTG CCC GTG CCC TGG CCC ACC CTC GTG ACC ACC TTC GGC TAC 1296
 410   T   T   G   K   L   P   V   P   W   P   T   L   V   T   T   F   G   Y   427

1297  GGC GTG CAG TGC TTC GCC CGC TAC CCC GAC CAC ATG AAG CAG CAC GAC TTC TTC 1350
 428   G   V   Q   C   F   A   R   Y   P   D   H   M   K   Q   H   D   F   F   445

1351  AAG TCC GCC ATG CCC GAA GGC TAC GTC CAG GAG CGC ACC ATC TTC TTC AAG GAC 1404
 446   K   S   A   M   P   E   G   Q   E   R   T   I   F   F   K   D   D   G   463

1405  GAC GGC AAC TAC AAG ACC CGC GCC GAG GTG AAG TTC GAG GGC GAC ACC CTG GTG 1458
 464   N   Y   K   T   R   Y   V   A   E   V   K   F   E   G   D   T   L   V   481

1459  AAC CGC ATC GAG CTG AAG GGC ATC GAC TTC AAG GAG GAC GGC AAC ATC CTG GGG 1512
 482   N   R   I   E   L   K   G   I   D   F   K   E   D   G   N   I   L   G   499

1513  CAC AAG CTG GAG TAC AAC TAC AAC AGC CAC AAC GTC TAT ATC ATG GCC GAC AAG 1566
 500   H   K   L   E   Y   N   Y   N   S   H   N   V   Y   I   M   A   D   K   517

1567  CAG AAG AAC GGC ATG AAG GTG AAC TTC AAG ATC CGC CAC AAC ATC GAG GAC GGC 1620
 518   Q   K   N   G   M   K   V   N   F   K   I   R   H   N   I   E   D   G   535

1621  AGC GTG CAG CTC GCC GAC CAC TAC CAG CAG AAC ACC CCC ATC GGC GAC GGC CCC 1674
 536   S   V   Q   L   A   D   H   Y   Q   Q   N   T   P   I   G   D   G   P   553

1675  GTG CTG CTG CCC GAC AAC CAC TAC CTG AGC TAC CAG TCC GCC CTG AGC AAA GAC 1728
 554   V   L   L   P   D   N   H   Y   L   S   Y   Q   S   A   L   S   K   D   571

                                       ER remaining sequence     EcoR I
1729  CCC AAC GAG AAG CGC GAT CAC AAG GAC GAG CTG TAA GAA TTC                 1770
 572   P   N   E   K   R   D   H   K   D   E   L   *                           582
```

# Figure 8

## ApoK2er

```
      Hind III    Not I                  ER signal sequence of calreticulin
   1  AAG CTT GCG GCC GCC ACC ATG CTG CTG CCC GTC CCC CTG CTG CTG GGC CTG CTG    54
   1                  Kozak    M   L   L   P   V   P   L   L   L   G   L   L    13

                                   Enhanced cyan fluorescent protein (ECFP)
  55  GCC GCC GCC GAC GTG GGC AGC AAG GGC GAG GAG CTG TTG ACC GGG GTG GTG CCC   108
  14   A   A   A   D   V   G   S   K   G   E   E   L   L   T   G   V   V   P    31

 109  ATC CTG GTC GAG CTG GAC GGC GAC GTA AAC GGC CAC AGG TTC AGC GTG TCC GGC   162
  32   I   L   V   E   L   D   G   D   V   N   G   H   R   F   S   V   S   G    49

 163  GAG GGC GAG GGC GAT GCC ACC TAC GGC AAG CTG ACC CTG AAG TTC ATC TGC ACC   216
  50   E   G   E   G   D   A   T   Y   G   K   L   T   L   K   F   I   C   T    67

 217  ACC GGC AAG CTG CCC GTG CCC TGG CCC ACC CTC GTG ACC ACC CTG ACC TGG GGC   270
  68   T   G   K   L   P   V   P   W   P   T   L   V   T   T   L   T   W   G    85

 271  GTG CAG TGC TTC AGC CGC TAC CCC GAC CAC ATG AAG CAG CAC GAC TTC TTC AAG   324
  86   V   Q   C   F   S   R   Y   P   D   H   M   K   Q   H   D   F   F   K   103

 325  TCC GCC ATG CCC GAA GGC TAC GTC CAG GAG CGC ACC ATC TTC TTC AAG GAC GAC   378
 104   S   A   M   P   E   G   Y   V   Q   E   R   T   I   F   F   K   D   D   121

 379  GGC AAC TAC AAG ACC CGC GCC GAG GTG AAG TTC GAG GGC GAC ACC CTG GTG AAC   432
 122   G   N   Y   K   T   R   A   E   V   K   F   E   G   D   T   L   V   N   139

 433  CGC ATC GAG CTG AAG GGC ATC GAC TTG AAG GAG GAC GGC AAC ATC CTG GGG CAC   486
 140   R   I   E   L   K   G   I   D   L   K   E   D   G   N   I   L   G   H   157

 487  AAG CTG GAG TAC AAC TAT ATC AGC CAC AAC GTC TAT ATC ACC GCC GAC AAG CAG   540
 158   K   L   E   Y   N   Y   I   S   H   N   V   Y   I   T   A   D   K   Q   175

 541  AAG AAC GGC ATC AAG GCC CAC TTC AAG ATG CGC CAC AAC ATC GAG GAC GGC AGC   594
 176   K   N   G   I   K   A   H   F   K   M   R   H   N   I   E   D   G   S   193

 595  GTG CAG GTG GCC GAC CAC TAC CAG CAG AAC ACC CCC ATC GGC GAC GGC CCC GTG   648
 194   V   Q   V   A   D   H   Y   Q   Q   N   T   P   I   G   D   G   P   V   211

 649  CTG CTG CCC GAC AAC CAC TAC CTG AGC ACC CAG TCC GCC CTG AGC AAA GAC CCC   702
 212   L   L   P   D   N   H   Y   L   S   T   Q   S   A   L   S   K   D   P   229

                                                                   Sph I
 703  AAC GAG AAG CGC GAT CAC ATG GTC CTG CTG GAG TTC GTG ACC GCC GCC CGC ATG   756
 230   N   E   K   R   D   H   M   V   L   L   E   F   V   T   A   A   R   M   247

                      kringle of Apo(a)
 757  CAG CAA AGC CAT GTG GTC CAG GAT TGC TAC CAT GGT GAT GGA CAG AGT TAT CGA   810
 248  E→Q  Q   S   H   V   V   Q   D   C   Y   H   G   D   G   Q   S   Y   R   265

 811  GGC ACG TAC TCC ACC ACT GTC ACA GGA AGG ACC TGC CAA GCT TGG TCA TCT ATG   864
 266   G   T   Y   S   T   T   V   T   G   R   T   C   Q   A   W   S   S   M   283

 865  ACA CCA CAT CAA CAT AAT AGG ACC ACA GAA AAC TAC CCA AAT GCT GGC TTG ATC   918
 284   T   P   H   Q   H   N   R   T   T   E   N   Y   P   N   A   G   L   I   301

 919  ATG AAC TAC TGC AGG AAT CCA GAT GCT GTG GCA GCT CCT TAT TGT TAT ACG AGG   972
 302   M   N   Y   C   R   N   P   D   A   V   A   A   P   Y   C   Y   T   R   319

 973  GAT CCC GGT GTC AGG TGG GAG TAC TGC AAC CTG ACG CAA TGC TCA GAC GCA GAA  1026
 320   D   P   G   V   R   W   E   Y   C   N   L   T   Q   C   S   D   A   E   337

1027  GGG ACT GCC GTC GCG CCT CCG ACT GTT ACC CCG GTT CCA AGC CTA GAG GCT CCT  1080
 338   G   T   A   V   A   P   P   T   V   T   P   V   P   S   L   E   A   P   355
```

# Figure 8 cont.

```
1081  TCC GAA CAA GCA CCG ACT GAG CAA AGG CCT GGG GTG CAG GAG TGC TAC CAT GGT 1134
 356   S   E   Q   A   P   T   E   Q   R   P   G   V   Q   E   C   Y   H   G   373

1135  AAT GGA CAG AGT TAT CGA GGC ACA TAC TCC ACC ACT GTC ACA GGA AGA ACC TGC 1188
 374   N   G   Q   S   Y   R   G   T   Y   S   T   T   V   T   G   R   T   C   391

1189  CAA GCT TGG TCA TCT ATG ACA CCA CAC TCG CAT AGT CGG ACC CCA GAA TAC TAC 1242
 392   A   W   S   S   M   T   P   H   S   H   S   R   T   P   E   Y   Y   P   409

1243  CCA AAT GCT GGC TTG ATC ATG AAC TAC TGC AGG AAT CCA GAT GCT GTG GCA GCT 1296
 410   N   A   G   L   I   M   N   Y   Q   C   R   N   P   D   A   V   A   A   427

1297  CCT TAT TGT TAT ACG AGG GAT CCC GGT GTC AGG TGG GAG TAC TGC AAC CTG ACG 1350
 428   P   Y   C   Y   T   R   D   P   G   V   R   W   E   Y   C   N   L   T   445

1351  CAA TGC TCA GAC GCA GAA GGG ACT GCC GTC GCG CCT CCG ACT GTT ACC CCG GTT 1404
 446   Q   C   S   D   A   E   G   T   A   V   A   P   P   T   V   T   P   V   463

                                                Sac I    Enhanced yellow fluorescent protein (EYFP)
1459  CCA AGC CTA GAG GCT CCT TCC GAA CAA GAG CTC ATG GTG AGC AAG GGC GAG GAG 1458
 464   P   S   L   E   A   P   S   E   Q   E   L   M   V   S   K   G   E   E   481

1459  CTG TTC ACC GGG GTG GTG CCC ATC CTG GTC GAG CTG GAC GGC GAC CTA AAC GGC 1512
 482   L   F   T   G   V   V   P   I   L   V   E   L   D   G   D   L   N   G   499

1135  CAC AAG TTC AGC GTG TCC GGC AGA GGC GAG GGC GAT GCC ACC TAC GGG AAG CTG 1566
 500   H   K   F   S   V   S   G   R   G   E   G   D   A   T   Y   G   K   L   517

1189  ACC CTG AAG TTC ATC TGC ACC ACC GGC AAG CTG CCC GTG CCC TGG CCC ACC CTC 1620
 518   T   L   K   F   I   C   T   T   G   K   L   P   V   P   W   P   T   L   535

1243  GTG ACC ACC TTC GGC TAC GGC GTG CAG TGC TTC GCC CGC TAC CCC GAC CAC ATG 1674
 536   V   T   T   F   G   Y   G   V   Q   C   F   A   R   Y   P   D   H   M   553

1297  AAG CAG CAC GAC TTC TTC AAG TCC GCC ATG CCC GAA GGC TAC GTC CAG GAG CGC 1728
 554   K   Q   H   D   F   F   K   S   A   M   P   E   G   Q   E   R   T   I   571

1351  ACC ATC TTC TTC AAG GAC GAC GGC AAC TAC AAG ACC CGC GCC GAG GTG AAG TTC 1782
 572   F   F   K   D   D   G   N   Y   K   T   R   Y   V   A   E   V   K   F   589

1405  GAG GGC GAC ACC CTG GTG AAC CGC ATC GAG CTG AAG GGC ATC GAC TTC AAG GAG 1836
 590   E   G   D   T   L   V   N   R   I   E   L   K   G   I   D   F   K   E   607

1837  GAC GGC AAC ATC CTG GGG CAC AAG CTG GAG TAC AAC TAC AAC AGC CAC AAC GTC 1890
 608   D   G   N   I   L   G   H   K   L   E   Y   N   Y   N   S   H   N   V   625

1891  TAT ATC ATG GCC GAC AAG CAG AAG AAC GGC ATG AAG GTG AAC TTC AAG ATC CGC 1944
 626   Y   I   M   A   D   K   Q   K   N   G   M   K   V   N   F   K   I   R   643

1945  CAC AAC ATC GAG GAC GGC AGC GTG CAG CTC GCC GAC CAC TAC CAG CAG AAC ACC 1998
 644   H   N   I   E   D   G   S   V   Q   L   A   D   H   Y   Q   Q   N   T   661

1999  CCC ATC GGC GAC GGC CCC GTG CTG CTG CCC GAC AAC CAC TAC CTG AGC TAC CAG 2052
 662   P   I   G   D   G   P   V   L   L   P   D   N   H   Y   L   S   Y   Q   679

                                                            ER remaining sequence
2053  TCC GCC CTG AGC AAA GAC CCC AAC GAG AAG CGC GAT CAC AAG GAC GAG CTG TAA 2106
 680   S   A   L   S   K   D   P   N   E   K   R   D   H   K   D   E   L   *   697

      EcoR I
2107  GAA TTC                                                                 2112
```

European Patent Office

# EUROPEAN SEARCH REPORT

Application Number
EP 05 45 0006

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | TRUONG KEVIN ET AL: "FRET-based in vivo Ca2+ imaging by a new calmodulin-GFP fusion molecule." NATURE STRUCTURAL BIOLOGY, vol. 8, no. 12, December 2001 (2001-12), pages 1069-1073, XP002280931 ISSN: 1072-8368 in particular see abstract and the tandem fusion of Fig. 1 and discussion * the whole document * ----- | 1-3 | C12N15/62<br>C12Q1/66<br>G01N33/58 |
| Y | XU QIN ET AL: "Apolipoprotein E4 domain interaction occurs in living neuronal cells as determined by fluorescence resonance energy transfer." THE JOURNAL OF BIOLOGICAL CHEMISTRY. 11 JUN 2004, vol. 279, no. 24, 11 June 2004 (2004-06-11), pages 25511-25516, XP002336163 ISSN: 0021-9258 See Fig. 1 constructs and Material and Methods page 25512 left column ----- | 1-3 | TECHNICAL FIELDS SEARCHED (Int.Cl.7)<br>C12N |
| Y,D | MIYAWAKI A ET AL: "FLUORESCENT INDICATORS FOR CA2+ BASED ON GREEN FLUORESCENT PROTEINSAND CALMODULIN" NATURE, MACMILLAN JOURNALS LTD. LONDON, GB, vol. 388, no. 6645, 28 August 1997 (1997-08-28), pages 882-887, XP002058386 ISSN: 0028-0836 in particular see Fig. 1 and "methods" page 887 ----- -/-- | 1-3 | |

The present search report has been drawn up for all claims

| Place of search | Date of completion of the search | Examiner |
|---|---|---|
| Munich | 18 July 2005 | Vix, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

4

EPO FORM 1503 03.82 (P04C01)

European Patent Office

# EUROPEAN SEARCH REPORT

Application Number
EP 05 45 0006

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | MICHALAK M ET AL: "Ca2+ signaling and calcium binding chaperones of the endoplasmic reticulum." CELL CALCIUM. 2002 NOV-DEC, vol. 32, no. 5-6, November 2002 (2002-11), pages 269-278, XP002336138 ISSN: 0143-4160 * the whole document * ----- | 20-34 | |
| A,D | US 2004/002065 A1 (THOMAS PHILIP JORDAN ET AL) 1 January 2004 (2004-01-01) * the whole document * ----- | 21-34 | |
| P,X | YANG X ET AL: "A new pair for inter- and intra-molecular FRET measurement" BIOCHEMICAL AND BIOPHYSICAL RESEARCH COMMUNICATIONS, ACADEMIC PRESS INC. ORLANDO, FL, US, vol. 330, no. 3, 13 May 2005 (2005-05-13), pages 914-920, XP004833725 ISSN: 0006-291X * the whole document * ----- | 1-12 | TECHNICAL FIELDS SEARCHED (Int.Cl.7) |

The present search report has been drawn up for all claims

| Place of search | Date of completion of the search | Examiner |
|---|---|---|
| Munich | 18 July 2005 | Vix, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4
EPO FORM 1503 03.82 (P04C01)

## ANNEX TO THE EUROPEAN SEARCH REPORT ON EUROPEAN PATENT APPLICATION NO. EP 05 45 0006

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2005

| Patent document cited in search report | Publication date | Patent family member(s) | Publication date |
|---|---|---|---|
| US 2004002065 A1 | 01-01-2004 | AU 3705501 A | 27-08-2001 |
| | | CA 2398834 A1 | 23-08-2001 |
| | | EP 1257829 A2 | 20-11-2002 |
| | | JP 2004500092 T | 08-01-2004 |
| | | US 2005019829 A1 | 27-01-2005 |
| | | WO 0160840 A2 | 23-08-2001 |

EPO FORM P0459

For more details about this annex : see Official Journal of the European Patent Office, No. 12/82

SEQUENCE LISTING

<110> Medizinische Universität Graz
<120> kringle domain sensor
<130> r44674
<160> 18
<170> PatentIn version 3.1

<210> 1
<211> 18
<212> PRT
<213> Homo sapiens

<400> 1

Met Leu Leu Ser Val Pro Leu Leu Leu Gly Leu Leu Gly Leu Ala Ala
1 5 10 15

Ala Asp

<210> 2
<211> 18
<212> DNA
<213> Homo sapiens

<400> 2
aagcttgcgg ccgccacc 18

<210> 3
<211> 678
<212> DNA
<213> Synthetic Construct

<400> 3
agcaagggcg aggagctgtt gaccggggtg gtgcccatcc tggtcgagct ggacggcgac 60

gtaaacggcc acaggttcag cgtgtccggc gagggcgagg gcgatgccac ctacggcaag 120

ctgaccctga agttcatctg caccaccggc aagctgcccg tgccctggcc caccctcgtg 180

accaccctga cctggggcgt gcagtgcttc agccgctacc ccgaccacat gaagcagcac 240

gacttcttca agtccgccat gcccgaaggc tacgtccagg agcgcaccat cttcttcaag 300

gacgacggca actacaagac ccgcgccgag gtgaagttcg agggcgacac cctggtgaac 360

cgcatcgagc tgaagggcat cgacttgaag gaggacggca acatcctggg gcacaagctg 420

gagtacaact atatcagcca caacgtctat atcaccgccg acaagcagaa gaacggcatc 480

aaggcccact tcaagatgcg ccacaacatc gaggacggca gcgtgcaggt ggccgaccac 540

taccagcaga acacccccat cggcgacggc cccgtgctgc tgcccgacaa ccactacctg 600

agcacccagt ccgccctgag caaagacccc aacgagaagc gcgatcacat ggtcctgctg 660

```
gagttcgtga ccgccgcc                                                    678

<210>  4
<211>  226
<212>  PRT
<213>  Synthetic Construct

<400>  4

Ser Lys Gly Glu Glu Leu Leu Thr Gly Val Val Pro Ile Leu Val Glu
1               5                   10                  15

Leu Asp Gly Asp Val Asn Gly His Arg Phe Ser Val Ser Gly Glu Gly
            20                  25                  30

Glu Gly Asp Ala Thr Tyr Gly Lys Leu Thr Leu Lys Phe Ile Cys Thr
        35                  40                  45

Thr Gly Lys Leu Pro Val Pro Trp Pro Thr Leu Val Thr Thr Leu Thr
    50                  55                  60

Trp Gly Val Gln Cys Phe Ser Arg Tyr Pro Asp His Met Lys Gln His
65                  70                  75                  80

Asp Phe Phe Lys Ser Ala Met Pro Glu Gly Tyr Val Gln Glu Arg Thr
                85                  90                  95

Ile Phe Phe Lys Asp Asp Gly Asn Tyr Lys Thr Arg Ala Glu Val Lys
            100                 105                 110

Phe Glu Gly Asp Thr Leu Val Asn Arg Ile Glu Leu Lys Gly Ile Asp
        115                 120                 125

Leu Lys Glu Asp Gly Asn Ile Leu Gly His Lys Leu Glu Tyr Asn Tyr
    130                 135                 140

Ile Ser His Asn Val Tyr Ile Thr Ala Asp Lys Gln Lys Asn Gly Ile
145                 150                 155                 160

Lys Ala His Phe Lys Met Arg His Asn Ile Glu Asp Gly Ser Val Gln
                165                 170                 175

Val Ala Asp His Tyr Gln Gln Asn Thr Pro Ile Gly Asp Gly Pro Val
            180                 185                 190
```

Leu Leu Pro Asp Asn His Tyr Leu Ser Thr Gln Ser Ala Leu Ser Lys
195 200 205

Asp Pro Asn Glu Lys Arg Asp His Met Val Leu Leu Glu Phe Val Thr
210 215 220

Ala Ala
225

<210> 5
<211> 333
<212> DNA
<213> Homo sapiens

<400> 5
cagcaaagcc atgtggtcca ggattgctac catggtgatg gacagagtta tcgaggcacg 60

tactccacca ctgtcacagg aaggacctgc caagcttggt catctatgac accacatcaa 120

cataatagga ccacagaaaa ctacccaaat gctggcttga tcatgaacta ctgcaggaat 180

ccagatgctg tggcagctcc ttattgttat acgagggatc ccggtgtcag gtgggagtac 240

tgcaacctga cgcaatgctc agacgcagaa gggactgccg tcgcgcctcc gactgttacc 300

ccggttccaa gcctagaggc tccttccgaa caa 333

<210> 6
<211> 111
<212> PRT
<213> Homo sapiens

<400> 6

Gln Gln Ser His Val Val Gln Asp Cys Tyr His Gly Asp Gly Gln Ser
1 5 10 15

Tyr Arg Gly Thr Tyr Ser Thr Thr Val Thr Gly Arg Thr Cys Gln Ala
20 25 30

Trp Ser Ser Met Thr Pro His Gln His Asn Arg Thr Thr Glu Asn Tyr
35 40 45

Pro Asn Ala Gly Leu Ile Met Asn Tyr Cys Arg Asn Pro Asp Ala Val
50 55 60

Ala Ala Pro Tyr Cys Tyr Thr Arg Asp Pro Gly Val Arg Trp Glu Tyr
65 70 75 80

Cys Asn Leu Thr Gln Cys Ser Asp Ala Glu Gly Thr Ala Val Ala Pro

85 90 95

Pro Thr Val Thr Pro Val Pro Ser Leu Glu Ala Pro Ser Glu Gln
100 105 110

<210> 7
<211> 675
<212> DNA
<213> Homo sapiens

<400> 7
cagcaaagcc atgtggtcca ggattgctac catggtgatg gacagagtta tcgaggcacg 60

tactccacca ctgtcacagg aaggacctgc caagcttggt catctatgac accacatcaa 120

cataatagga ccacagaaaa ctacccaaat gctggcttga tcatgaacta ctgcaggaat 180

ccagatgctg tggcagctcc ttattgttat acgagggatc ccggtgtcag gtgggagtac 240

tgcaacctga cgcaatgctc agacgcagaa gggactgccg tcgcgcctcc gactgttacc 300

ccggttccaa gcctagaggc tccttccgaa caagcaccga ctgagcaaag gcctggggtg 360

caggagtgct accatggtaa tggacagagt tatcgaggca catactccac cactgtcaca 420

ggaagaacct gccaagcttg gtcatctatg acaccacact cgcatagtcg gaccccagaa 480

tactacccaa atgctggctt gatcatgaac tactgcagga atccagatgc tgtggcagct 540

ccttattgtt atacgaggga tcccggtgtc aggtgggagt actgcaacct gacgcaatgc 600

tcagacgcag aagggactgc cgtcgcgcct ccgactgtta ccccggttcc aagcctagag 660

gctccttccg aacaa 675

<210> 8
<211> 225
<212> PRT
<213> Homo sapiens

<400> 8

Gln Gln Ser His Val Val Gln Asp Cys Tyr His Gly Asp Gly Gln Ser
1 5 10 15

Tyr Arg Gly Thr Tyr Ser Thr Thr Val Thr Gly Arg Thr Cys Gln Ala
20 25 30

Trp Ser Ser Met Thr Pro His Gln His Asn Arg Thr Thr Glu Asn Tyr
35 40 45

Pro Asn Ala Gly Leu Ile Met Asn Tyr Cys Arg Asn Pro Asp Ala Val
50 55 60

```
Ala Ala Pro Tyr Cys Tyr Thr Arg Asp Pro Gly Val Arg Trp Glu Tyr
65                  70                  75                  80

Cys Asn Leu Thr Gln Cys Ser Asp Ala Glu Gly Thr Ala Val Ala Pro
                85                  90                  95

Pro Thr Val Thr Pro Val Pro Ser Leu Glu Ala Pro Ser Glu Gln Ala
            100                 105                 110

Pro Thr Glu Gln Arg Pro Gly Val Gln Glu Cys Tyr His Gly Asn Gly
        115                 120                 125

Gln Ser Tyr Arg Gly Thr Tyr Ser Thr Thr Val Thr Gly Arg Thr Cys
    130                 135                 140

Ala Trp Ser Ser Met Thr Pro His Ser His Ser Arg Thr Pro Glu Tyr
145                 150                 155                 160

Tyr Pro Asn Ala Gly Leu Ile Met Asn Tyr Gln Cys Arg Asn Pro Asp
                165                 170                 175

Ala Val Ala Ala Pro Tyr Cys Tyr Thr Arg Asp Pro Gly Val Arg Trp
            180                 185                 190

Glu Tyr Cys Asn Leu Thr Gln Cys Ser Asp Ala Glu Gly Thr Ala Val
        195                 200                 205

Ala Pro Pro Thr Val Thr Pro Val Pro Ser Leu Glu Ala Pro Ser Glu
    210                 215                 220

Gln
225
```

<210> 9
<211> 654
<212> DNA
<213> Synthetic Construct

<400> 9

```
atggtgagca agggcgagga gctgttcacc ggggtggtgc ccatcctggt cgagctggac      60

ggcgacctaa acggccacaa gttcagcgtg tccggcagag gcgagggcga tgccacctac     120

gggaagctga ccctgaagtt catctgcacc accggcaagc tgcccgtgcc ctggcccacc     180

ctcgtgacca ccttcggcta cggcgtgcag tgcttcgccc gctaccccga ccacatgaag     240
```

```
cagcacgact tcttcaagtc cgccatgccc gaaggctacg tccaggagcg caccatcttc    300
ttcaaggacg acggcaacta caagacccgc gccgaggtga agttcgaggg cgacaccctg    360
gtgaaccgca tcgagctgaa gggcatcgac ttcaaggagg acggcaacat cctggggcac    420
aagctggagt acaactacaa cagccacaac gtctatatca tggccgacaa gcagaagaac    480
ggcatgaagg tgaacttcaa gatccgccac aacatcgagg acggcagcgt gcagctcgcc    540
gaccactacc agcagaacac ccccatcggc gacggccccg tgctgctgcc cgacaaccac    600
tacctgagct accagtccgc cctgagcaaa gaccccaacg agaagcgcga tcac          654
```

```
<210>  10
<211>  218
<212>  PRT
<213>  Synthetic Construct

<400>  10

Met Val Ser Lys Gly Glu Glu Leu Phe Thr Gly Val Val Pro Ile Leu
1               5                   10                  15

Val Glu Leu Asp Gly Asp Leu Asn Gly His Lys Phe Ser Val Ser Gly
            20                  25                  30

Arg Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Phe Gly Tyr Gly Val Gln Cys Phe Ala Arg Tyr Pro Asp His Met Lys
65                  70                  75                  80

Gln His Asp Phe Phe Lys Ser Ala Met Pro Glu Gly Gln Glu Arg Thr
                85                  90                  95

Ile Phe Phe Lys Asp Asp Gly Asn Tyr Lys Thr Arg Tyr Val Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140
```

Asn Tyr Asn Ser His Asn Val Tyr Ile Met Ala Asp Lys Gln Lys Asn
145 150 155 160

Gly Met Lys Val Asn Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Tyr Gln Ser Ala Leu
195 200 205

Ser Lys Asp Pro Asn Glu Lys Arg Asp His
210 215

<210> 11
<211> 15
<212> DNA
<213> Homo sapiens

<400> 11
aaggacgagc tgtaa 15

<210> 12
<211> 2112
<212> DNA
<213> Homo sapiens

<400> 12
aagcttgcgg ccgccaccat gctgctgccc gtcccccctgc tgctgggcct gctggccgcc 60

gccgacgtgg gcagcaaggg cgaggagctg ttgaccgggg tggtgcccat cctggtcgag 120

ctggacggcg acgtaaacgg ccacaggttc agcgtgtccg gcgagggcga gggcgatgcc 180

acctacggca agctgaccct gaagttcatc tgcaccaccg gcaagctgcc cgtgccctgg 240

cccaccctcg tgaccaccct gacctggggc gtgcagtgct tcagccgcta ccccgaccac 300

atgaagcagc acgacttctt caagtccgcc atgcccgaag gctacgtcca ggagcgcacc 360

atcttcttca aggacgacgg caactacaag acccgcgccg aggtgaagtt cgagggcgac 420

accctggtga accgcatcga gctgaagggc atcgacttga aggaggacgg caacatcctg 480

gggcacaagc tggagtacaa ctatatcagc cacaacgtct atatcaccgc cgacaagcag 540

aagaacggca tcaaggccca cttcaagatg cgccacaaca tcgaggacgg cagcgtgcag 600

gtggccgacc actaccagca gaacaccccc atcggcgacg gccccgtgct gctgcccgac 660

aaccactacc tgagcaccca gtccgccctg agcaaagacc ccaacgagaa gcgcgatcac 720

atggtcctgc tggagttcgt gaccgccgcc cgcatgcagc aaagccatgt ggtccaggat 780

tgctaccatg gtgatggaca gagttatcga ggcacgtact ccaccactgt cacaggaagg 840

acctgccaag cttggtcatc tatgacacca catcaacata ataggaccac agaaaactac 900

ccaaatgctg gcttgatcat gaactactgc aggaatccag atgctgtggc agctccttat 960

tgttatacga gggatcccgg tgtcaggtgg gagtactgca acctgacgca atgctcagac 1020

gcagaaggga ctgccgtcgc gcctccgact gttaccccgg ttccaagcct agaggctcct 1080

tccgaacaag caccgactga gcaaaggcct ggggtgcagg agtgctacca tggtaatgga 1140

cagagttatc gaggcacata ctccaccact gtcacaggaa gaacctgcca agcttggtca 1200

tctatgacac cacactcgca tagtcggacc ccagaatact acccaaatgc tggcttgatc 1260

atgaactact gcaggaatcc agatgctgtg gcagctcctt attgttatac gagggatccc 1320

ggtgtcaggt gggagtactg caacctgacg caatgctcag acgcagaagg gactgccgtc 1380

gcgcctccga ctgttacccc ggttccaagc ctagaggctc cttccgaaca agagctcatg 1440

gtgagcaagg gcgaggagct gttcaccggg gtggtgccca tcctggtcga gctggacggc 1500

gacctaaacg gccacaagtt cagcgtgtcc ggcagaggcg agggcgatgc cacctacggg 1560

aagctgaccc tgaagttcat ctgcaccacc ggcaagctgc ccgtgccctg gcccaccctc 1620

gtgaccacct tcggctacgg cgtgcagtgc ttcgcccgct accccgacca catgaagcag 1680

cacgacttct tcaagtccgc catgcccgaa ggctacgtcc aggagcgcac catcttcttc 1740

aaggacgacg gcaactacaa gacccgcgcc gaggtgaagt tcgagggcga caccctggtg 1800

aaccgcatcg agctgaaggg catcgacttc aaggaggacg gcaacatcct ggggcacaag 1860

ctggagtaca actacaacag ccacaacgtc tatatcatgg ccgacaagca gaagaacggc 1920

atgaaggtga acttcaagat ccgccacaac atcgaggacg gcagcgtgca gctcgccgac 1980

cactaccagc agaacacccc catcggcgac ggccccgtgc tgctgcccga caaccactac 2040

ctgagctacc agtccgccct gagcaaagac cccaacgaga agcgcgatca caaggacgag 2100

ctgtaagaat tc 2112

<210> 13
<211> 695
<212> PRT
<213> Homo sapiens

<400> 13

Met Leu Leu Pro Val Pro Leu Leu Leu Gly Leu Leu Ala Ala Ala Asp
1 5 10 15

```
Val Gly Ser Lys Gly Glu Glu Leu Leu Thr Gly Val Val Pro Ile Leu
            20                  25                  30

Val Glu Leu Asp Gly Asp Val Asn Gly His Arg Phe Ser Val Ser Gly
        35                  40                  45

Glu Gly Glu Gly Asp Ala Thr Tyr Gly Lys Leu Thr Leu Lys Phe Ile
    50                  55                  60

Cys Thr Thr Gly Lys Leu Pro Val Pro Trp Pro Thr Leu Val Thr Thr
65                  70                  75                  80

Leu Thr Trp Gly Val Gln Cys Phe Ser Arg Tyr Pro Asp His Met Lys
                85                  90                  95

Gln His Asp Phe Phe Lys Ser Ala Met Pro Glu Gly Tyr Val Gln Glu
            100                 105                 110

Arg Thr Ile Phe Phe Lys Asp Asp Gly Asn Tyr Lys Thr Arg Ala Glu
        115                 120                 125

Val Lys Phe Glu Gly Asp Thr Leu Val Asn Arg Ile Glu Leu Lys Gly
    130                 135                 140

Ile Asp Leu Lys Glu Asp Gly Asn Ile Leu Gly His Lys Leu Glu Tyr
145                 150                 155                 160

Asn Tyr Ile Ser His Asn Val Tyr Ile Thr Ala Asp Lys Gln Lys Asn
                165                 170                 175

Gly Ile Lys Ala His Phe Lys Met Arg His Asn Ile Glu Asp Gly Ser
            180                 185                 190

Val Gln Val Ala Asp His Tyr Gln Gln Asn Thr Pro Ile Gly Asp Gly
        195                 200                 205

Pro Val Leu Leu Pro Asp Asn His Tyr Leu Ser Thr Gln Ser Ala Leu
    210                 215                 220

Ser Lys Asp Pro Asn Glu Lys Arg Asp His Met Val Leu Leu Glu Phe
225                 230                 235                 240

Val Thr Ala Ala Arg Met Gln Gln Ser His Val Val Gln Asp Cys Tyr
                245                 250                 255
```

His Gly Asp Gly Gln Ser Tyr Arg Gly Thr Tyr Ser Thr Thr Val Thr
260 265 270

Gly Arg Thr Cys Gln Ala Trp Ser Ser Met Thr Pro His Gln His Asn
275 280 285

Arg Thr Thr Glu Asn Tyr Pro Asn Ala Gly Leu Ile Met Asn Tyr Cys
290 295 300

Arg Asn Pro Asp Ala Val Ala Ala Pro Tyr Cys Tyr Thr Arg Asp Pro
305 310 315 320

Gly Val Arg Trp Glu Tyr Cys Asn Leu Thr Gln Cys Ser Asp Ala Glu
325 330 335

Gly Thr Ala Val Ala Pro Pro Thr Val Thr Pro Val Pro Ser Leu Glu
340 345 350

Ala Pro Ser Glu Gln Ala Pro Thr Glu Gln Arg Pro Gly Val Gln Glu
355 360 365

Cys Tyr His Gly Asn Gly Gln Ser Tyr Arg Gly Thr Tyr Ser Thr Thr
370 375 380

Val Thr Gly Arg Thr Cys Ala Trp Ser Ser Met Thr Pro His Ser His
385 390 395 400

Ser Arg Thr Pro Glu Tyr Tyr Pro Asn Ala Gly Leu Ile Met Asn Tyr
405 410 415

Gln Cys Arg Asn Pro Asp Ala Val Ala Ala Pro Tyr Cys Tyr Thr Arg
420 425 430

Asp Pro Gly Val Arg Trp Glu Tyr Cys Asn Leu Thr Gln Cys Ser Asp
435 440 445

Ala Glu Gly Thr Ala Val Ala Pro Pro Thr Val Thr Pro Val Pro Ser
450 455 460

Leu Glu Ala Pro Ser Glu Gln Glu Leu Met Val Ser Lys Gly Glu Glu
465 470 475 480

Leu Phe Thr Gly Val Val Pro Ile Leu Val Glu Leu Asp Gly Asp Leu

485 490 495

Asn Gly His Lys Phe Ser Val Ser Gly Arg Gly Glu Gly Asp Ala Thr
500 505 510

Tyr Gly Lys Leu Thr Leu Lys Phe Ile Cys Thr Thr Gly Lys Leu Pro
515 520 525

Val Pro Trp Pro Thr Leu Val Thr Thr Phe Gly Tyr Gly Val Gln Cys
530 535 540

Phe Ala Arg Tyr Pro Asp His Met Lys Gln His Asp Phe Phe Lys Ser
545 550 555 560

Ala Met Pro Glu Gly Gln Glu Arg Thr Ile Phe Phe Lys Asp Asp Gly
565 570 575

Asn Tyr Lys Thr Arg Tyr Val Ala Glu Val Lys Phe Glu Gly Asp Thr
580 585 590

Leu Val Asn Arg Ile Glu Leu Lys Gly Ile Asp Phe Lys Glu Asp Gly
595 600 605

Asn Ile Leu Gly His Lys Leu Glu Tyr Asn Tyr Asn Ser His Asn Val
610 615 620

Tyr Ile Met Ala Asp Lys Gln Lys Asn Gly Met Lys Val Asn Phe Lys
625 630 635 640

Ile Arg His Asn Ile Glu Asp Gly Ser Val Gln Leu Ala Asp His Tyr
645 650 655

Gln Gln Asn Thr Pro Ile Gly Asp Gly Pro Val Leu Leu Pro Asp Asn
660 665 670

His Tyr Leu Ser Tyr Gln Ser Ala Leu Ser Lys Asp Pro Asn Glu Lys
675 680 685

Arg Asp His Lys Asp Glu Leu
690 695

<210> 14
<211> 1770
<212> DNA
<213> Homo sapiens

<400> 14

aagcttgcgg ccgccaccat gctgctgccc gtccccctgc tgctgggcct gctggccgcc 60
gccgacgtgg gcagcaaggg cgaggagctg ttgaccgggg tggtgcccat cctggtcgag 120
ctggacggcg acgtaaacgg ccacaggttc agcgtgtccg gcgagggcga gggcgatgcc 180
acctacggca agctgaccct gaagttcatc tgcaccaccg gcaagctgcc cgtgccctgg 240
cccaccctcg tgaccaccct gacctggggc gtgcagtgct tcagccgcta ccccgaccac 300
atgaagcagc acgacttctt caagtccgcc atgcccgaag gctacgtcca ggagcgcacc 360
atcttcttca aggacgacgg caactacaag acccgcgccg aggtgaagtt cgagggcgac 420
accctggtga accgcatcga gctgaagggc atcgacttga aggaggacgg caacatcctg 480
gggcacaagc tggagtacaa ctatatcagc cacaacgtct atatcaccgc cgacaagcag 540
aagaacggca tcaaggccca cttcaagatg cgccacaaca tcgaggacgg cagcgtgcag 600
gtggccgacc actaccagca gaacaccccc atcggcgacg gccccgtgct gctgcccgac 660
aaccactacc tgagcaccca gtccgccctg agcaaagacc ccaacgagaa gcgcgatcac 720
atggtcctgc tggagttcgt gaccgccgcc cgcatgcagc aaagccatgt ggtccaggat 780
tgctaccatg gtgatggaca gagttatcga ggcacgtact ccaccactgt cacaggaagg 840
acctgccaag cttggtcatc tatgacacca catcaacata ataggaccac agaaaactac 900
ccaaatgctg gcttgatcat gaactactgc aggaatccag atgctgtggc agctccttat 960
tgttatacga gggatcccgg tgtcaggtgg gagtactgca acctgacgca atgctcagac 1020
gcagaaggga ctgccgtcgc gcctccgact gttaccccgg ttccaagcct agaggctcct 1080
tccgaacaag agctcatggt gagcaagggc gaggagctgt tcaccggggt ggtgcccatc 1140
ctggtcgagc tggacggcga cctaaacggc cacaagttca gcgtgtccgg cgagggcgag 1200
ggcgatgcca cctacgggaa gctgaccctg aagttcatct gcaccaccgg caagctgccc 1260
gtgccctggc ccaccctcgt gaccaccttc ggctacggcg tgcagtgctt cgcccgctac 1320
cccgaccaca tgaagcagca cgacttcttc aagtccgcca tgcccgaagg ctacgtccag 1380
gagcgcacca tcttcttcaa ggacgacggc aactacaaga cccgcgccga ggtgaagttc 1440
gagggcgaca ccctggtgaa ccgcatcgag ctgaagggca tcgacttcaa ggaggacggc 1500
aacatcctgg ggcacaagct ggagtacaac tacaacagcc acaacgtcta tatcatggcc 1560
gacaagcaga agaacggcat gaaggtgaac ttcaagatcc gccacaacat cgaggacggc 1620
agcgtgcagc tcgccgacca ctaccagcag aacaccccca tcggcgacgg ccccgtgctg 1680
ctgcccgaca accactacct gagctaccag tccgccctga gcaaagaccc caacgagaag 1740

cgcgatcaca aggacgagct gtaagaattc 1770

<210> 15
<211> 581
<212> PRT
<213> Homo sapiens

<400> 15

```
Met Leu Leu Pro Val Pro Leu Leu Leu Gly Leu Leu Ala Ala Ala Asp
1               5                   10                  15

Val Gly Ser Lys Gly Glu Glu Leu Leu Thr Gly Val Val Pro Ile Leu
            20                  25                  30

Val Glu Leu Asp Gly Asp Val Asn Gly His Arg Phe Ser Val Ser Gly
        35                  40                  45

Glu Gly Glu Gly Asp Ala Thr Tyr Gly Lys Leu Thr Leu Lys Phe Ile
    50                  55                  60

Cys Thr Thr Gly Lys Leu Pro Val Pro Trp Pro Thr Leu Val Thr Thr
65                  70                  75                  80

Leu Thr Trp Gly Val Gln Cys Phe Ser Arg Tyr Pro Asp His Met Lys
                85                  90                  95

Gln His Asp Phe Phe Lys Ser Ala Met Pro Glu Gly Tyr Val Gln Glu
            100                 105                 110

Arg Thr Ile Phe Phe Lys Asp Asp Gly Asn Tyr Lys Thr Arg Ala Glu
        115                 120                 125

Val Lys Phe Glu Gly Asp Thr Leu Val Asn Arg Ile Glu Leu Lys Gly
    130                 135                 140

Ile Asp Leu Lys Glu Asp Gly Asn Ile Leu Gly His Lys Leu Glu Tyr
145                 150                 155                 160

Asn Tyr Ile Ser His Asn Val Tyr Ile Thr Ala Asp Lys Gln Lys Asn
                165                 170                 175

Gly Ile Lys Ala His Phe Lys Met Arg His Asn Ile Glu Asp Gly Ser
            180                 185                 190
```

```
Val Gln Val Ala Asp His Tyr Gln Gln Asn Thr Pro Ile Gly Asp Gly
        195                 200                 205

Pro Val Leu Leu Pro Asp Asn His Tyr Leu Ser Thr Gln Ser Ala Leu
    210                 215                 220

Ser Lys Asp Pro Asn Glu Lys Arg Asp His Met Val Leu Leu Glu Phe
225                 230                 235                 240

Val Thr Ala Ala Arg Met Gln Gln Ser His Val Val Gln Asp Cys Tyr
                245                 250                 255

His Gly Asp Gly Gln Ser Tyr Arg Gly Thr Tyr Ser Thr Thr Val Thr
            260                 265                 270

Gly Arg Thr Cys Gln Ala Trp Ser Ser Met Thr Pro His Gln His Asn
        275                 280                 285

Arg Thr Thr Glu Asn Tyr Pro Asn Ala Gly Leu Ile Met Asn Tyr Cys
    290                 295                 300

Arg Asn Pro Asp Ala Val Ala Ala Pro Tyr Cys Tyr Thr Arg Asp Pro
305                 310                 315                 320

Gly Val Arg Trp Glu Tyr Cys Asn Leu Thr Gln Cys Ser Asp Ala Glu
                325                 330                 335

Gly Thr Ala Val Ala Pro Pro Thr Val Thr Pro Val Pro Ser Leu Glu
            340                 345                 350

Ala Pro Ser Glu Gln Glu Leu Met Val Ser Lys Gly Glu Glu Leu Phe
        355                 360                 365

Thr Gly Val Val Pro Ile Leu Val Glu Leu Asp Gly Asp Leu Asn Gly
    370                 375                 380

His Lys Phe Ser Val Ser Gly Arg Gly Glu Gly Asp Ala Thr Tyr Gly
385                 390                 395                 400

Lys Leu Thr Leu Lys Phe Ile Cys Thr Thr Gly Lys Leu Pro Val Pro
                405                 410                 415

Trp Pro Thr Leu Val Thr Thr Phe Gly Tyr Gly Val Gln Cys Phe Ala
            420                 425                 430
```

```
Arg Tyr Pro Asp His Met Lys Gln His Asp Phe Phe Lys Ser Ala Met
        435                 440                 445

Pro Glu Gly Gln Glu Arg Thr Ile Phe Phe Lys Asp Asp Gly Asn Tyr
    450                 455                 460

Lys Thr Arg Tyr Val Ala Glu Val Lys Phe Glu Gly Asp Thr Leu Val
465                 470                 475                 480

Asn Arg Ile Glu Leu Lys Gly Ile Asp Phe Lys Glu Asp Gly Asn Ile
                485                 490                 495

Leu Gly His Lys Leu Glu Tyr Asn Tyr Asn Ser His Asn Val Tyr Ile
            500                 505                 510

Met Ala Asp Lys Gln Lys Asn Gly Met Lys Val Asn Phe Lys Ile Arg
        515                 520                 525

His Asn Ile Glu Asp Gly Ser Val Gln Leu Ala Asp His Tyr Gln Gln
    530                 535                 540

Asn Thr Pro Ile Gly Asp Gly Pro Val Leu Leu Pro Asp Asn His Tyr
545                 550                 555                 560

Leu Ser Tyr Gln Ser Ala Leu Ser Lys Asp Pro Asn Glu Lys Arg Asp
                565                 570                 575

His Lys Asp Glu Leu
            580

<210>  16
<211>  13938
<212>  DNA
<213>  Homo sapiens

<400>  16
ctgggattgg gacacacttt ctggacactg ctggccagtc ccaaaatgga acataaggaa      60

gtggttcttc tacttctttt atttctgaaa tcagcagcac ctgagcaaag ccatgtggtc     120

caggattgct accatggtga tggacagagt tatcgaggca cgtactccac cactgtcaca     180

ggaaggacct gccaagcttg gtcatctatg acaccacatc aacataatag gaccacagaa     240

aactacccaa atgctggctt gatcatgaac tactgcagga atccagatgc tgtggcagct     300

ccttattgtt atacgaggga tcccggtgtc aggtgggagt actgcaacct gacgcaatgc     360
```

```
tcagacgcag aagggactgc cgtcgcgcct ccgactgtta ccccggttcc aagcctagag     420
gctccttccg aacaagcacc gactgagcaa aggcctgggg tgcaggagtg ctaccatggt     480
aatggacaga gttatcgagg cacatactcc accactgtca caggaagaac ctgccaagct     540
tggtcatcta tgacaccaca ctcgcatagt cggaccccag aatactaccc aaatgctggc     600
ttgatcatga actactgcag gaatccagat gctgtggcag ctccttattg ttatacgagg     660
gatcccggtg tcaggtggga gtactgcaac ctgacgcaat gctcagacgc agaagggact     720
gccgtcgcgc ctccgactgt taccccggtt ccaagcctag aggctccttc cgaacaagca     780
ccgactgagc aaaggcctgg ggtgcaggag tgctaccatg gtaatggaca gagttatcga     840
ggcacatact ccaccactgt cacaggaaga acctgccaag cttggtcatc tatgacacca     900
cactcgcata gtcggacccc agaatactac ccaaatgctg gcttgatcat gaactactgc     960
aggaatccag atgctgtggc agctccttat tgttatacga gggatcccgg tgtcaggtgg    1020
gagtactgca acctgacgca atgctcagac gcagaaggga ctgccgtcgc gcctccgact    1080
gttaccccgg ttccaagcct agaggctcct tccgaacaag caccgactga gcaaaggcct    1140
ggggtgcagg agtgctacca tggtaatgga cagagttatc gaggcacata ctccaccact    1200
gtcacaggaa gaacctgcca agcttggtca tctatgacac cacactcgca tagtcggacc    1260
ccagaatact acccaaatgc tggcttgatc atgaactact gcaggaatcc agatgctgtg    1320
gcagctcctt attgttatac gagggatccc ggtgtcaggt gggagtactg caacctgacg    1380
caatgctcag acgcagaagg gactgccgtc gcgcctccga ctgttacccc ggttccaagc    1440
ctagaggctc cttccgaaca agcaccgact gagcaaaggc ctggggtgca ggagtgctac    1500
catggtaatg gacagagtta tcgaggcaca tactccacca ctgtcacagg aagaacctgc    1560
caagcttggt catctatgac accacactcg catagtcgga ccccagaata ctacccaaat    1620
gctggcttga tcatgaacta ctgcaggaat ccagatgctg tggcagctcc ttattgttat    1680
acgagggatc ccggtgtcag gtgggagtac tgcaacctga cgcaatgctc agacgcagaa    1740
gggactgccg tcgcgcctcc gactgttacc ccggttccaa gcctagaggc tccttccgaa    1800
caagcaccga ctgagcaaag gcctggggtg caggagtgct accatggtaa tggacagagt    1860
tatcgaggca catactccac cactgtcaca ggaagaacct gccaagcttg gtcatctatg    1920
acaccacact cgcatagtcg gaccccagaa tactacccaa atgctggctt gatcatgaac    1980
tactgcagga atccagatgc tgtggcagct ccttattgtt atacgaggga tcccggtgtc    2040
aggtgggagt actgcaacct gacgcaatgc tcagacgcag aagggactgc cgtcgcgcct    2100
ccgactgtta ccccggttcc aagcctagag gctccttccg aacaagcacc gactgagcaa    2160
```

aggcctgggg tgcaggagtg ctaccatggt aatggacaga gttatcgagg cacatactcc 2220
accactgtca caggaagaac ctgccaagct tggtcatcta tgacaccaca ctcgcatagt 2280
cggaccccag aatactaccc aaatgctggc ttgatcatga actactgcag gaatccagat 2340
gctgtggcag ctccttattg ttatacgagg gatcccggtg tcaggtggga gtactgcaac 2400
ctgacgcaat gctcagacgc agaagggact gccgtcgcgc ctccgactgt taccccggtt 2460
ccaagcctag aggctccttc cgaacaagca ccgactgagc aaaggcctgg ggtgcaggag 2520
tgctaccatg gtaatggaca gagttatcga ggcacatact ccaccactgt cacaggaaga 2580
acctgccaag cttggtcatc tatgacacca cactcgcata gtcggacccc agaatactac 2640
ccaaatgctg gcttgatcat gaactactgc aggaatccag atgctgtggc agctccttat 2700
tgttatacga gggatcccgg tgtcaggtgg gagtactgca acctgacgca atgctcagac 2760
gcagaaggga ctgccgtcgc gcctccgact gttaccccgg ttccaagcct agaggctcct 2820
tccgaacaag caccgactga gcaaaggcct ggggtgcagg agtgctacca tggtaatgga 2880
cagagttatc gaggcacata ctccaccact gtcacaggaa gaacctgcca agcttggtca 2940
tctatgacac cacactcgca tagtcggacc ccagaatact acccaaatgc tggcttgatc 3000
atgaactact gcaggaatcc agatgctgtg gcagctcctt attgttatac gagggatccc 3060
ggtgtcaggt gggagtactg caacctgacg caatgctcag acgcagaagg gactgccgtc 3120
gcgcctccga ctgttacccc ggttccaagc ctagaggctc cttccgaaca agcaccgact 3180
gagcaaaggc ctggggtgca ggagtgctac catggtaatg gacagagtta tcgaggcaca 3240
tactccacca ctgtcacagg aagaacctgc caagcttggt catctatgac accacactcg 3300
catagtcgga ccccagaata ctacccaaat gctggcttga tcatgaacta ctgcaggaat 3360
ccagatgctg tggcagctcc ttattgttat acgagggatc ccggtgtcag gtgggagtac 3420
tgcaacctga cgcaatgctc agacgcagaa gggactgccg tcgcgcctcc gactgttacc 3480
ccggttccaa gcctagaggc tccttccgaa caagcaccga ctgagcaaag gcctggggtg 3540
caggagtgct accatggtaa tggacagagt tatcgaggca catactccac cactgtcaca 3600
ggaagaacct gccaagcttg gtcatctatg acaccacact cgcatagtcg gaccccagaa 3660
tactacccaa atgctggctt gatcatgaac tactgcagga atccagatgc tgtggcagct 3720
ccttattgtt atacgaggga tcccggtgtc aggtgggagt actgcaacct gacgcaatgc 3780
tcagacgcag aagggactgc cgtcgcgcct ccgactgtta ccccggttcc aagcctagag 3840
gctccttccg aacaagcacc gactgagcaa aggcctgggg tgcaggagtg ctaccatggt 3900

aatggacaga gttatcgagg cacatactcc accactgtca caggaagaac ctgccaagct 3960
tggtcatcta tgacaccaca ctcgcatagt cggaccccag aatactaccc aaatgctggc 4020
ttgatcatga actactgcag gaatccagat gctgtggcag ctccttattg ttatacgagg 4080
gatcccggtg tcaggtggga gtactgcaac ctgacgcaat gctcagacgc agaagggact 4140
gccgtcgcgc ctccgactgt taccccggtt ccaagcctag aggctccttc cgaacaagca 4200
ccgactgagc aaaggcctgg ggtgcaggag tgctaccatg gtaatggaca gagttatcga 4260
ggcacatact ccaccactgt cacaggaaga acctgccaag cttggtcatc tatgacacca 4320
cactcgcata gtcggacccc agaatactac ccaaatgctg gcttgatcat gaactactgc 4380
aggaatccag atgctgtggc agctccttat tgttatacga gggatcccgg tgtcaggtgg 4440
gagtactgca acctgacgca atgctcagac gcagaaggga ctgccgtcgc gcctccgact 4500
gttaccccgg ttccaagcct agaggctcct tccgaacaag caccgactga gcaaaggcct 4560
ggggtgcagg agtgctacca tggtaatgga cagagttatc gaggcacata ctccaccact 4620
gtcacaggaa gaacctgcca agcttggtca tctatgacac cacactcgca tagtcggacc 4680
ccagaatact acccaaatgc tggcttgatc atgaactact gcaggaatcc agatgctgtg 4740
gcagctcctt attgttatac gagggatccc ggtgtcaggt gggagtactg caacctgacg 4800
caatgctcag acgcagaagg gactgccgtc gcgcctccga ctgttacccc ggttccaagc 4860
ctagaggctc cttccgaaca agcaccgact gagcaaaggc ctggggtgca ggagtgctac 4920
catggtaatg gacagagtta tcgaggcaca tactccacca ctgtcacagg aagaacctgc 4980
caagcttggt catctatgac accacactcg catagtcgga ccccagaata ctacccaaat 5040
gctggcttga tcatgaacta ctgcaggaat ccagatgctg tggcagctcc ttattgttat 5100
acgagggatc ccggtgtcag gtgggagtac tgcaacctga cgcaatgctc agacgcagaa 5160
gggactgccg tcgcgcctcc gactgttacc ccggttccaa gcctagaggc tccttccgaa 5220
caagcaccga ctgagcaaag gcctggggtg caggagtgct accatggtaa tggacagagt 5280
tatcgaggca catactccac cactgtcaca ggaagaacct gccaagcttg gtcatctatg 5340
acaccacact cgcatagtcg gaccccagaa tactacccaa atgctggctt gatcatgaac 5400
tactgcagga atccagatgc tgtggcagct ccttattgtt atacgaggga tcccggtgtc 5460
aggtgggagt actgcaacct gacgcaatgc tcagacgcag aagggactgc cgtcgcgcct 5520
ccgactgtta ccccggttcc aagcctagag gctccttccg aacaagcacc gactgagcaa 5580
aggcctgggg tgcaggagtg ctaccatggt aatggacaga gttatcgagg cacatactcc 5640
accactgtca caggaagaac ctgccaagct tggtcatcta tgacaccaca ctcgcatagt 5700

cggaccccag aatactaccc aaatgctggc ttgatcatga actactgcag gaatccagat 5760
gctgtggcag ctccttattg ttatacgagg gatcccggtg tcaggtggga gtactgcaac 5820
ctgacgcaat gctcagacgc agaagggact gccgtcgcgc ctccgactgt taccccggtt 5880
ccaagcctag aggctccttc cgaacaagca ccgactgagc aaaggcctgg ggtgcaggag 5940
tgctaccatg gtaatggaca gagttatcga ggcacatact ccaccactgt cacaggaaga 6000
acctgccaag cttggtcatc tatgacacca cactcgcata gtcggacccc agaatactac 6060
ccaaatgctg gcttgatcat gaactactgc aggaatccag atgctgtggc agctccttat 6120
tgttatacga gggatcccgg tgtcaggtgg gagtactgca acctgacgca atgctcagac 6180
gcagaaggga ctgccgtcgc gcctccgact gttaccccgg ttccaagcct agaggctcct 6240
tccgaacaag caccgactga gcaaaggcct ggggtgcagg agtgctacca tggtaatgga 6300
cagagttatc gaggcacata ctccaccact gtcacaggaa gaacctgcca agcttggtca 6360
tctatgacac cacactcgca tagtcggacc ccagaatact acccaaatgc tggcttgatc 6420
atgaactact gcaggaatcc agatgctgtg gcagctcctt attgttatac gagggatccc 6480
ggtgtcaggt gggagtactg caacctgacg caatgctcag acgcagaagg gactgccgtc 6540
gcgcctccga ctgttacccc ggttccaagc ctagaggctc cttccgaaca agcaccgact 6600
gagcaaaggc ctggggtgca ggagtgctac catggtaatg gacagagtta tcgaggcaca 6660
tactccacca ctgtcacagg aagaacctgc caagcttggt catctatgac accacactcg 6720
catagtcgga ccccagaata ctacccaaat gctggcttga tcatgaacta ctgcaggaat 6780
ccagatgctg tggcagctcc ttattgttat acgagggatc ccggtgtcag gtgggagtac 6840
tgcaacctga cgcaatgctc agacgcagaa gggactgccg tcgcgcctcc gactgttacc 6900
ccggttccaa gcctagaggc tccttccgaa caagcaccga ctgagcaaag gcctggggtg 6960
caggagtgct accatggtaa tggacagagt tatcgaggca catactccac cactgtcaca 7020
ggaagaacct gccaagcttg gtcatctatg acaccacact cgcatagtcg gaccccagaa 7080
tactacccaa atgctggctt gatcatgaac tactgcagga atccagatgc tgtggcagct 7140
ccttattgtt atacgaggga tcccggtgtc aggtgggagt actgcaacct gacgcaatgc 7200
tcagacgcag aagggactgc cgtcgcgcct ccgactgtta ccccggttcc aagcctagag 7260
gctccttccg aacaagcacc gactgagcaa aggcctgggg tgcaggagtg ctaccatggt 7320
aatggacaga gttatcgagg cacatactcc accactgtca caggaagaac ctgccaagct 7380
tggtcatcta tgacaccaca ctcgcatagt cggaccccag aatactaccc aaatgctggc 7440

ttgatcatga actactgcag gaatccagat gctgtggcag ctccttattg ttatacgagg 7500
gatcccggtg tcaggtggga gtactgcaac ctgacgcaat gctcagacgc agaagggact 7560
gccgtcgcgc ctccgactgt taccccggtt ccaagcctag aggctccttc cgaacaagca 7620
ccgactgagc aaaggcctgg ggtgcaggag tgctaccatg gtaatggaca gagttatcga 7680
ggcacatact ccaccactgt cacaggaaga acctgccaag cttggtcatc tatgacacca 7740
cactcgcata gtcggacccc agaatactac ccaaatgctg gcttgatcat gaactactgc 7800
aggaatccag atgctgtggc agctccttat tgttatacga gggatcccgg tgtcaggtgg 7860
gagtactgca acctgacgca atgctcagac gcagaaggga ctgccgtcgc gcctccgact 7920
gttaccccgg ttccaagcct agaggctcct tccgaacaag caccgactga gcagaggcct 7980
ggggtgcagg agtgctacca cggtaatgga cagagttatc gaggcacata ctccaccact 8040
gtcactggaa gaacctgcca agcttggtca tctatgacac cacactcgca tagtcggacc 8100
ccagaatact acccaaatgc tggcttgatc atgaactact gcaggaatcc agatgctgtg 8160
gcagctcctt attgttatac gagggatccc ggtgtcaggt gggagtactg caacctgacg 8220
caatgctcag acgcagaagg gactgccgtc gcgcctccga ctgttacccc ggttccaagc 8280
ctagaggctc cttccgaaca agcaccgact gagcaaaggc ctggggtgca ggagtgctac 8340
catggtaatg gacagagtta tcgaggcaca tactccacca ctgtcacagg aagaacctgc 8400
caagcttggt catctatgac accacactcg catagtcgga ccccagaata ctacccaaat 8460
gctggcttga tcatgaacta ctgcaggaat ccagatgctg tggcagctcc ttattgttat 8520
acgagggatc ccggtgtcag gtgggagtac tgcaacctga cgcaatgctc agacgcagaa 8580
gggactgccg tcgcgcctcc gactgttacc ccggttccaa gcctagaggc tccttccgaa 8640
caagcaccga ctgagcaaag gcctggggtg caggagtgct accatggtaa tggacagagt 8700
tatcgaggca catactccac cactgtcaca ggaagaacct gccaagcttg gtcatctatg 8760
acaccacact cgcatagtcg gaccccagaa tactacccaa atgctggctt gatcatgaac 8820
tactgcagga atccagatgc tgtggcagct ccttattgtt atacgaggga tcccggtgtc 8880
aggtgggagt actgcaacct gacgcaatgc tcagacgcag aagggactgc cgtcgcgcct 8940
ccgactgtta ccccggttcc aagcctagag gctccttccg aacaagcacc gactgagcag 9000
aggcctgggg tgcaggagtg ctaccacggt aatggacaga gttatcgagg cacatactcc 9060
accactgtca ctggaagaac ctgccaagct tggtcatcta tgacaccaca ctcgcatagt 9120
cggaccccag aatactaccc aaatgctggc ttgatcatga actactgcag gaatccagat 9180
gctgtggcag ctccttattg ttatacgagg gatcccggtg tcaggtggga gtactgcaac 9240

ctgacgcaat gctcagacgc agaagggact gccgtcgcgc ctccgactgt taccccggtt 9300
ccaagcctag aggctccttc cgaacaagca ccgactgagc agaggcctgg ggtgcaggag 9360
tgctaccacg gtaatggaca gagttatcga ggcacatact ccaccactgt cactggaaga 9420
acctgccaag cttggtcatc tatgacacca cactcgcata gtcggacccc agaatactac 9480
ccaaatgctg gcttgatcat gaactactgc aggaatccag atgctgtggc agctccttat 9540
tgttatacga gggatcccgg tgtcaggtgg gagtactgca acctgacgca atgctcagac 9600
gcagaaggga ctgccgtcgc gcctccgact gttaccccgg ttccaagcct agaggctcct 9660
tccgaacaag caccgactga gcagaggcct ggggtgcagg agtgctacca cggtaatgga 9720
cagagttatc gaggcacata ctccaccact gtcactggaa gaacctgcca agcttggtca 9780
tctatgacac cacactcgca tagtcggacc ccagaatact acccaaatgc tggcttgatc 9840
atgaactact gcaggaatcc agatgctgtg gcagctcctt attgttatac gagggatccc 9900
ggtgtcaggt gggagtactg caacctgacg caatgctcag acgcagaagg gactgccgtc 9960
gcgcctccga ctgttacccc ggttccaagc ctagaggctc cttccgaaca agcaccgact 10020
gagcagaggc ctggggtgca ggagtgctac cacggtaatg gacagagtta tcgaggcaca 10080
tactccacca ctgtcactgg aagaacctgc caagcttggt catctatgac accacactcg 10140
catagtcgga ccccagaata ctacccaaat gctggcttga tcatgaacta ctgcaggaat 10200
ccagatcctg tggcagcccc ttattgttat acgagggatc ccagtgtcag gtgggagtac 10260
tgcaacctga cacaatgctc agacgcagaa gggactgccg tcgcgcctcc aactattacc 10320
ccgattccaa gcctagaggc tccttctgaa caagcaccaa ctgagcaaag gcctggggtg 10380
caggagtgct accacggaaa tggacagagt tatcaaggca catacttcat tactgtcaca 10440
ggaagaacct gccaagcttg gtcatctatg acaccacact cgcatagtcg gaccccagca 10500
tactacccaa atgctggctt gatcaagaac tactgccgaa atccagatcc tgtggcagcc 10560
ccttggtgtt atacaacaga tcccagtgtc aggtgggagt actgcaacct gacacgatgc 10620
tcagatgcag aatggactgc cttcgtccct ccgaatgtta ttctggctcc aagcctagag 10680
gctttttttg aacaagcact gactgaggaa acccccgggg tacaggactg ctactaccat 10740
tatggacaga gttaccgagg cacatactcc accactgtca caggaagaac ttgccaagct 10800
tggtcatcta tgacaccaca ccagcatagt cggaccccag aaaactaccc aaatgctggc 10860
ctgaccagga actactgcag gaatccagat gctgagattc gcccttggtg ttacaccatg 10920
gatcccagtg tcaggtggga gtactgcaac ctgacacaat gcctggtgac agaatcaagt 10980

gtccttgcaa ctctcacggt ggtcccagat ccaagcacag aggcttcttc tgaagaagca 11040
ccaacggagc aaagccccgg ggtccaggat tgctaccatg gtgatggaca gagttatcga 11100
ggctcattct ctaccactgt cacaggaagg acatgtcagt cttggtcctc tatgacacca 11160
cactggcatc agaggacaac agaatattat ccaaatggtg gcctgaccag gaactactgc 11220
aggaatccag atgctgagat tagtccttgg tgttatacca tggatcccaa tgtcagatgg 11280
gagtactgca acctgacaca atgtccagtg acagaatcaa gtgtccttgc gacgtccacg 11340
gctgtttctg aacaagcacc aacggagcaa agccccacag tccaggactg ctaccatggt 11400
gatggacaga gttatcgagg ctcattctcc accactgtta caggaaggac atgtcagtct 11460
tggtcctcta tgacaccaca ctggcatcag agaaccacag aatactaccc aaatggtggc 11520
ctgaccagga actactgcag gaatccagat gctgagattc gcccttggtg ttataccatg 11580
gatcccagtg tcagatggga gtactgcaac ctgacgcaat gtccagtgat ggaatcaact 11640
ctcctcacaa ctcccacggt ggtcccagtt ccaagcacag agcttccttc tgaagaagca 11700
ccaactgaaa acagcactgg ggtccaggac tgctaccgag gtgatggaca gagttatcga 11760
ggcacactct ccaccactat cacaggaaga acatgtcagt cttggtcgtc tatgacacca 11820
cattggcatc ggaggatccc attatactat ccaaatgctg gcctgaccag gaactactgc 11880
aggaatccag atgctgagat tcgcccttgg tgttacacca tggatcccag tgtcaggtgg 11940
gagtactgca acctgacacg atgtccagtg acagaatcga gtgtcctcac aactcccaca 12000
gtggccccgg ttccaagcac agaggctcct tctgaacaag caccacctga gaaaagccct 12060
gtggtccagg attgctacca tggtgatgga cggagttatc gaggcatatc ctccaccact 12120
gtcacaggaa ggacctgtca atcttggtca tctatgatac cacactggca tcagaggacc 12180
ccagaaaact acccaaatgc tggcctgacc gagaactact gcaggaatcc agattctggg 12240
aaacaacccct ggtgttacac aaccgatccg tgtgtgaggt gggagtactg caatctgaca 12300
caatgctcag aaacagaatc aggtgtccta gagactccca ctgttgttcc agttccaagc 12360
atggaggctc attctgaagc agcaccaact gagcaaaccc ctgtggtccg gcagtgctac 12420
catggtaatg gccagagtta tcgaggcaca ttctccacca ctgtcacagg aaggacatgt 12480
caatcttggt catccatgac accacaccgg catcagagga ccccagaaaa ctacccaaat 12540
gatggcctga caatgaacta ctgcaggaat ccagatgccg atacaggccc ttggtgtttt 12600
accatggacc ccagcatcag gtgggagtac tgcaacctga cgcgatgctc agacacagaa 12660
gggactgtgg tcgctcctcc gactgtcatc caggttccaa gcctagggcc tccttctgaa 12720
caagactgta tgtttgggaa tgggaaagga taccggggca agaaggcaac cactgttact 12780

gggacgccat gccaggaatg ggctgcccag gagccccata gacacagcac gttcattcca 12840

gggacaaata aatgggcagg tctggaaaaa aattactgcc gtaaccctga tggtgacatc 12900

aatggtccct ggtgctacac aatgaatcca agaaaacttt ttgactactg tgatatccct 12960

ctctgtgcat cctcttcatt tgattgtggg aagcctcaag tggagccgaa gaaatgtcct 13020

ggaagcattg taggggggtg tgtggcccac ccacattcct ggccctggca agtcagtctc 13080

agaacaaggt ttggaaagca cttctgtgga ggcaccttaa tatccccaga gtgggtgctg 13140

actgctgctc actgcttgaa gaagtcctca aggccttcat cctacaaggt catcctgggt 13200

gcacaccaag aagtgaacct cgaatctcat gttcaggaaa tagaagtgtc taggctgttc 13260

ttggagccca cacaagcaga tattgccttg ctaaagctaa gcaggcctgc cgtcatcact 13320

gacaaagtaa tgccagcttg tctgccatcc ccagactaca tggtcaccgc caggactgaa 13380

tgttacatca ctggctgggg agaaacccaa ggtacctttg ggactggcct tctcaaggaa 13440

gcccagctcc ttgttattga gaatgaagtg tgcaatcact ataagtatat ttgtgctgag 13500

catttggcca gaggcactga cagttgccag ggtgacagtg gagggcctct ggtttgcttc 13560

gagaaggaca aatacatttt acaaggagtc acttcttggg gtcttggctg tgcacgcccc 13620

aataagcctg gtgtctatgc tcgtgtttca aggtttgtta cttggattga gggaatgatg 13680

agaaataatt aattggacgg gagacagagt gaagcatcaa cctacttaga agctgaaacg 13740

tgggtaagga tttagcatgc tggaaataat agacagcaat caaacgaaga cactgttccc 13800

agctaccagc tatgccaaac cttggcattt ttggtatttt tgtgtataag cttttaaggt 13860

ctgactgaca aattctgtat taaggtgtca tagctatgac atttgttaaa aataaactct 13920

gcacttattt tgatttga 13938

<210> 17
<211> 4548
<212> PRT
<213> Homo sapiens

<400> 17

Met Glu His Lys Glu Val Val Leu Leu Leu Leu Leu Phe Leu Lys Ser
1 5 10 15

Ala Ala Pro Glu Gln Ser His Val Val Gln Asp Cys Tyr His Gly Asp
20 25 30

Gly Gln Ser Tyr Arg Gly Thr Tyr Ser Thr Thr Val Thr Gly Arg Thr
35 40 45

```
Cys Gln Ala Trp Ser Ser Met Thr Pro His Gln His Asn Arg Thr Thr
    50                  55                  60

Glu Asn Tyr Pro Asn Ala Gly Leu Ile Met Asn Tyr Cys Arg Asn Pro
65                  70                  75                  80

Asp Ala Val Ala Ala Pro Tyr Cys Tyr Thr Arg Asp Pro Gly Val Arg
                85                  90                  95

Trp Glu Tyr Cys Asn Leu Thr Gln Cys Ser Asp Ala Glu Gly Thr Ala
            100                 105                 110

Val Ala Pro Pro Thr Val Thr Pro Val Pro Ser Leu Glu Ala Pro Ser
        115                 120                 125

Glu Gln Ala Pro Thr Glu Gln Arg Pro Gly Val Gln Glu Cys Tyr His
    130                 135                 140

Gly Asn Gly Gln Ser Tyr Arg Gly Thr Tyr Ser Thr Thr Val Thr Gly
145                 150                 155                 160

Arg Thr Cys Gln Ala Trp Ser Ser Met Thr Pro His Ser His Ser Arg
                165                 170                 175

Thr Pro Glu Tyr Tyr Pro Asn Ala Gly Leu Ile Met Asn Tyr Cys Arg
            180                 185                 190

Asn Pro Asp Ala Val Ala Ala Pro Tyr Cys Tyr Thr Arg Asp Pro Gly
        195                 200                 205

Val Arg Trp Glu Tyr Cys Asn Leu Thr Gln Cys Ser Asp Ala Glu Gly
    210                 215                 220

Thr Ala Val Ala Pro Pro Thr Val Thr Pro Val Pro Ser Leu Glu Ala
225                 230                 235                 240

Pro Ser Glu Gln Ala Pro Thr Glu Gln Arg Pro Gly Val Gln Glu Cys
                245                 250                 255

Tyr His Gly Asn Gly Gln Ser Tyr Arg Gly Thr Tyr Ser Thr Thr Val
            260                 265                 270

Thr Gly Arg Thr Cys Gln Ala Trp Ser Ser Met Thr Pro His Ser His
```

275 280 285

Ser Arg Thr Pro Glu Tyr Tyr Pro Asn Ala Gly Leu Ile Met Asn Tyr
290 295 300

Cys Arg Asn Pro Asp Ala Val Ala Ala Pro Tyr Cys Tyr Thr Arg Asp
305 310 315 320

Pro Gly Val Arg Trp Glu Tyr Cys Asn Leu Thr Gln Cys Ser Asp Ala
325 330 335

Glu Gly Thr Ala Val Ala Pro Pro Thr Val Thr Pro Val Pro Ser Leu
340 345 350

Glu Ala Pro Ser Glu Gln Ala Pro Thr Glu Gln Arg Pro Gly Val Gln
355 360 365

Glu Cys Tyr His Gly Asn Gly Gln Ser Tyr Arg Gly Thr Tyr Ser Thr
370 375 380

Thr Val Thr Gly Arg Thr Cys Gln Ala Trp Ser Ser Met Thr Pro His
385 390 395 400

Ser His Ser Arg Thr Pro Glu Tyr Tyr Pro Asn Ala Gly Leu Ile Met
405 410 415

Asn Tyr Cys Arg Asn Pro Asp Ala Val Ala Ala Pro Tyr Cys Tyr Thr
420 425 430

Arg Asp Pro Gly Val Arg Trp Glu Tyr Cys Asn Leu Thr Gln Cys Ser
435 440 445

Asp Ala Glu Gly Thr Ala Val Ala Pro Pro Thr Val Thr Pro Val Pro
450 455 460

Ser Leu Glu Ala Pro Ser Glu Gln Ala Pro Thr Glu Gln Arg Pro Gly
465 470 475 480

Val Gln Glu Cys Tyr His Gly Asn Gly Gln Ser Tyr Arg Gly Thr Tyr
485 490 495

Ser Thr Thr Val Thr Gly Arg Thr Cys Gln Ala Trp Ser Ser Met Thr
500 505 510

```
Pro His Ser His Ser Arg Thr Pro Glu Tyr Tyr Pro Asn Ala Gly Leu
        515                 520                 525

Ile Met Asn Tyr Cys Arg Asn Pro Asp Ala Val Ala Ala Pro Tyr Cys
    530                 535                 540

Tyr Thr Arg Asp Pro Gly Val Arg Trp Glu Tyr Cys Asn Leu Thr Gln
545                 550                 555                 560

Cys Ser Asp Ala Glu Gly Thr Ala Val Ala Pro Pro Thr Val Thr Pro
                565                 570                 575

Val Pro Ser Leu Glu Ala Pro Ser Glu Gln Ala Pro Thr Glu Gln Arg
            580                 585                 590

Pro Gly Val Gln Glu Cys Tyr His Gly Asn Gly Gln Ser Tyr Arg Gly
        595                 600                 605

Thr Tyr Ser Thr Thr Val Thr Gly Arg Thr Cys Gln Ala Trp Ser Ser
    610                 615                 620

Met Thr Pro His Ser His Ser Arg Thr Pro Glu Tyr Tyr Pro Asn Ala
625                 630                 635                 640

Gly Leu Ile Met Asn Tyr Cys Arg Asn Pro Asp Ala Val Ala Ala Pro
                645                 650                 655

Tyr Cys Tyr Thr Arg Asp Pro Gly Val Arg Trp Glu Tyr Cys Asn Leu
            660                 665                 670

Thr Gln Cys Ser Asp Ala Glu Gly Thr Ala Val Ala Pro Pro Thr Val
        675                 680                 685

Thr Pro Val Pro Ser Leu Glu Ala Pro Ser Glu Gln Ala Pro Thr Glu
    690                 695                 700

Gln Arg Pro Gly Val Gln Glu Cys Tyr His Gly Asn Gly Gln Ser Tyr
705                 710                 715                 720

Arg Gly Thr Tyr Ser Thr Thr Val Thr Gly Arg Thr Cys Gln Ala Trp
                725                 730                 735

Ser Ser Met Thr Pro His Ser His Ser Arg Thr Pro Glu Tyr Tyr Pro
            740                 745                 750
```

```
Asn Ala Gly Leu Ile Met Asn Tyr Cys Arg Asn Pro Asp Ala Val Ala
        755                 760                 765

Ala Pro Tyr Cys Tyr Thr Arg Asp Pro Gly Val Arg Trp Glu Tyr Cys
    770                 775                 780

Asn Leu Thr Gln Cys Ser Asp Ala Glu Gly Thr Ala Val Ala Pro Pro
785                 790                 795                 800

Thr Val Thr Pro Val Pro Ser Leu Glu Ala Pro Ser Glu Gln Ala Pro
                805                 810                 815

Thr Glu Gln Arg Pro Gly Val Gln Glu Cys Tyr His Gly Asn Gly Gln
            820                 825                 830

Ser Tyr Arg Gly Thr Tyr Ser Thr Thr Val Thr Gly Arg Thr Cys Gln
        835                 840                 845

Ala Trp Ser Ser Met Thr Pro His Ser His Ser Arg Thr Pro Glu Tyr
    850                 855                 860

Tyr Pro Asn Ala Gly Leu Ile Met Asn Tyr Cys Arg Asn Pro Asp Ala
865                 870                 875                 880

Val Ala Ala Pro Tyr Cys Tyr Thr Arg Asp Pro Gly Val Arg Trp Glu
                885                 890                 895

Tyr Cys Asn Leu Thr Gln Cys Ser Asp Ala Glu Gly Thr Ala Val Ala
            900                 905                 910

Pro Pro Thr Val Thr Pro Val Pro Ser Leu Glu Ala Pro Ser Glu Gln
        915                 920                 925

Ala Pro Thr Glu Gln Arg Pro Gly Val Gln Glu Cys Tyr His Gly Asn
    930                 935                 940

Gly Gln Ser Tyr Arg Gly Thr Tyr Ser Thr Thr Val Thr Gly Arg Thr
945                 950                 955                 960

Cys Gln Ala Trp Ser Ser Met Thr Pro His Ser His Ser Arg Thr Pro
                965                 970                 975

Glu Tyr Tyr Pro Asn Ala Gly Leu Ile Met Asn Tyr Cys Arg Asn Pro
            980                 985                 990
```

Asp Ala Val Ala Ala Pro Tyr Cys Tyr Thr Arg Asp Pro Gly Val Arg
995 1000 1005

Trp Glu Tyr Cys Asn Leu Thr Gln Cys Ser Asp Ala Glu Gly Thr
1010 1015 1020

Ala Val Ala Pro Pro Thr Val Thr Pro Val Pro Ser Leu Glu Ala
1025 1030 1035

Pro Ser Glu Gln Ala Pro Thr Glu Gln Arg Pro Gly Val Gln Glu
1040 1045 1050

Cys Tyr His Gly Asn Gly Gln Ser Tyr Arg Gly Thr Tyr Ser Thr
1055 1060 1065

Thr Val Thr Gly Arg Thr Cys Gln Ala Trp Ser Ser Met Thr Pro
1070 1075 1080

His Ser His Ser Arg Thr Pro Glu Tyr Tyr Pro Asn Ala Gly Leu
1085 1090 1095

Ile Met Asn Tyr Cys Arg Asn Pro Asp Ala Val Ala Ala Pro Tyr
1100 1105 1110

Cys Tyr Thr Arg Asp Pro Gly Val Arg Trp Glu Tyr Cys Asn Leu
1115 1120 1125

Thr Gln Cys Ser Asp Ala Glu Gly Thr Ala Val Ala Pro Pro Thr
1130 1135 1140

Val Thr Pro Val Pro Ser Leu Glu Ala Pro Ser Glu Gln Ala Pro
1145 1150 1155

Thr Glu Gln Arg Pro Gly Val Gln Glu Cys Tyr His Gly Asn Gly
1160 1165 1170

Gln Ser Tyr Arg Gly Thr Tyr Ser Thr Thr Val Thr Gly Arg Thr
1175 1180 1185

Cys Gln Ala Trp Ser Ser Met Thr Pro His Ser His Ser Arg Thr
1190 1195 1200

Pro Glu Tyr Tyr Pro Asn Ala Gly Leu Ile Met Asn Tyr Cys Arg

```
    1205                    1210                    1215

Asn Pro Asp Ala Val Ala Ala Pro Tyr Cys Tyr Thr Arg Asp Pro
    1220                    1225                    1230

Gly Val Arg Trp Glu Tyr Cys Asn Leu Thr Gln Cys Ser Asp Ala
    1235                    1240                    1245

Glu Gly Thr Ala Val Ala Pro Pro Thr Val Thr Pro Val Pro Ser
    1250                    1255                    1260

Leu Glu Ala Pro Ser Glu Gln Ala Pro Thr Glu Gln Arg Pro Gly
    1265                    1270                    1275

Val Gln Glu Cys Tyr His Gly Asn Gly Gln Ser Tyr Arg Gly Thr
    1280                    1285                    1290

Tyr Ser Thr Thr Val Thr Gly Arg Thr Cys Gln Ala Trp Ser Ser
    1295                    1300                    1305

Met Thr Pro His Ser His Ser Arg Thr Pro Glu Tyr Tyr Pro Asn
    1310                    1315                    1320

Ala Gly Leu Ile Met Asn Tyr Cys Arg Asn Pro Asp Ala Val Ala
    1325                    1330                    1335

Ala Pro Tyr Cys Tyr Thr Arg Asp Pro Gly Val Arg Trp Glu Tyr
    1340                    1345                    1350

Cys Asn Leu Thr Gln Cys Ser Asp Ala Glu Gly Thr Ala Val Ala
    1355                    1360                    1365

Pro Pro Thr Val Thr Pro Val Pro Ser Leu Glu Ala Pro Ser Glu
    1370                    1375                    1380

Gln Ala Pro Thr Glu Gln Arg Pro Gly Val Gln Glu Cys Tyr His
    1385                    1390                    1395

Gly Asn Gly Gln Ser Tyr Arg Gly Thr Tyr Ser Thr Thr Val Thr
    1400                    1405                    1410

Gly Arg Thr Cys Gln Ala Trp Ser Ser Met Thr Pro His Ser His
    1415                    1420                    1425
```

```
Ser Arg  Thr Pro Glu Tyr Tyr  Pro Asn Ala Gly Leu  Ile Met Asn
    1430                 1435                 1440

Tyr Cys  Arg Asn Pro Asp Ala  Val Ala Ala Pro Tyr  Cys Tyr Thr
    1445                 1450                 1455

Arg Asp  Pro Gly Val Arg Trp  Glu Tyr Cys Asn Leu  Thr Gln Cys
    1460                 1465                 1470

Ser Asp  Ala Glu Gly Thr Ala  Val Ala Pro Pro Thr  Val Thr Pro
    1475                 1480                 1485

Val Pro  Ser Leu Glu Ala Pro  Ser Glu Gln Ala Pro  Thr Glu Gln
    1490                 1495                 1500

Arg Pro  Gly Val Gln Glu Cys  Tyr His Gly Asn Gly  Gln Ser Tyr
    1505                 1510                 1515

Arg Gly  Thr Tyr Ser Thr Thr  Val Thr Gly Arg Thr  Cys Gln Ala
    1520                 1525                 1530

Trp Ser  Ser Met Thr Pro His  Ser His Ser Arg Thr  Pro Glu Tyr
    1535                 1540                 1545

Tyr Pro  Asn Ala Gly Leu Ile  Met Asn Tyr Cys Arg  Asn Pro Asp
    1550                 1555                 1560

Ala Val  Ala Ala Pro Tyr Cys  Tyr Thr Arg Asp Pro  Gly Val Arg
    1565                 1570                 1575

Trp Glu  Tyr Cys Asn Leu Thr  Gln Cys Ser Asp Ala  Glu Gly Thr
    1580                 1585                 1590

Ala Val  Ala Pro Pro Thr Val  Thr Pro Val Pro Ser  Leu Glu Ala
    1595                 1600                 1605

Pro Ser  Glu Gln Ala Pro Thr  Glu Gln Arg Pro Gly  Val Gln Glu
    1610                 1615                 1620

Cys Tyr  His Gly Asn Gly Gln  Ser Tyr Arg Gly Thr  Tyr Ser Thr
    1625                 1630                 1635

Thr Val  Thr Gly Arg Thr Cys  Gln Ala Trp Ser Ser  Met Thr Pro
    1640                 1645                 1650
```

His Ser His Ser Arg Thr Pro Glu Tyr Tyr Pro Asn Ala Gly Leu
1655 1660 1665

Ile Met Asn Tyr Cys Arg Asn Pro Asp Ala Val Ala Ala Pro Tyr
1670 1675 1680

Cys Tyr Thr Arg Asp Pro Gly Val Arg Trp Glu Tyr Cys Asn Leu
1685 1690 1695

Thr Gln Cys Ser Asp Ala Glu Gly Thr Ala Val Ala Pro Pro Thr
1700 1705 1710

Val Thr Pro Val Pro Ser Leu Glu Ala Pro Ser Glu Gln Ala Pro
1715 1720 1725

Thr Glu Gln Arg Pro Gly Val Gln Glu Cys Tyr His Gly Asn Gly
1730 1735 1740

Gln Ser Tyr Arg Gly Thr Tyr Ser Thr Thr Val Thr Gly Arg Thr
1745 1750 1755

Cys Gln Ala Trp Ser Ser Met Thr Pro His Ser His Ser Arg Thr
1760 1765 1770

Pro Glu Tyr Tyr Pro Asn Ala Gly Leu Ile Met Asn Tyr Cys Arg
1775 1780 1785

Asn Pro Asp Ala Val Ala Ala Pro Tyr Cys Tyr Thr Arg Asp Pro
1790 1795 1800

Gly Val Arg Trp Glu Tyr Cys Asn Leu Thr Gln Cys Ser Asp Ala
1805 1810 1815

Glu Gly Thr Ala Val Ala Pro Pro Thr Val Thr Pro Val Pro Ser
1820 1825 1830

Leu Glu Ala Pro Ser Glu Gln Ala Pro Thr Glu Gln Arg Pro Gly
1835 1840 1845

Val Gln Glu Cys Tyr His Gly Asn Gly Gln Ser Tyr Arg Gly Thr
1850 1855 1860

Tyr Ser Thr Thr Val Thr Gly Arg Thr Cys Gln Ala Trp Ser Ser
1865 1870 1875

Met Thr Pro His Ser His Ser Arg Thr Pro Glu Tyr Tyr Pro Asn
1880 1885 1890

Ala Gly Leu Ile Met Asn Tyr Cys Arg Asn Pro Asp Ala Val Ala
1895 1900 1905

Ala Pro Tyr Cys Tyr Thr Arg Asp Pro Gly Val Arg Trp Glu Tyr
1910 1915 1920

Cys Asn Leu Thr Gln Cys Ser Asp Ala Glu Gly Thr Ala Val Ala
1925 1930 1935

Pro Pro Thr Val Thr Pro Val Pro Ser Leu Glu Ala Pro Ser Glu
1940 1945 1950

Gln Ala Pro Thr Glu Gln Arg Pro Gly Val Gln Glu Cys Tyr His
1955 1960 1965

Gly Asn Gly Gln Ser Tyr Arg Gly Thr Tyr Ser Thr Thr Val Thr
1970 1975 1980

Gly Arg Thr Cys Gln Ala Trp Ser Ser Met Thr Pro His Ser His
1985 1990 1995

Ser Arg Thr Pro Glu Tyr Tyr Pro Asn Ala Gly Leu Ile Met Asn
2000 2005 2010

Tyr Cys Arg Asn Pro Asp Ala Val Ala Ala Pro Tyr Cys Tyr Thr
2015 2020 2025

Arg Asp Pro Gly Val Arg Trp Glu Tyr Cys Asn Leu Thr Gln Cys
2030 2035 2040

Ser Asp Ala Glu Gly Thr Ala Val Ala Pro Pro Thr Val Thr Pro
2045 2050 2055

Val Pro Ser Leu Glu Ala Pro Ser Glu Gln Ala Pro Thr Glu Gln
2060 2065 2070

Arg Pro Gly Val Gln Glu Cys Tyr His Gly Asn Gly Gln Ser Tyr
2075 2080 2085

Arg Gly Thr Tyr Ser Thr Thr Val Thr Gly Arg Thr Cys Gln Ala

2090 2095 2100

Trp Ser Ser Met Thr Pro His Ser His Ser Arg Thr Pro Glu Tyr
2105 2110 2115

Tyr Pro Asn Ala Gly Leu Ile Met Asn Tyr Cys Arg Asn Pro Asp
2120 2125 2130

Ala Val Ala Ala Pro Tyr Cys Tyr Thr Arg Asp Pro Gly Val Arg
2135 2140 2145

Trp Glu Tyr Cys Asn Leu Thr Gln Cys Ser Asp Ala Glu Gly Thr
2150 2155 2160

Ala Val Ala Pro Pro Thr Val Thr Pro Val Pro Ser Leu Glu Ala
2165 2170 2175

Pro Ser Glu Gln Ala Pro Thr Glu Gln Arg Pro Gly Val Gln Glu
2180 2185 2190

Cys Tyr His Gly Asn Gly Gln Ser Tyr Arg Gly Thr Tyr Ser Thr
2195 2200 2205

Thr Val Thr Gly Arg Thr Cys Gln Ala Trp Ser Ser Met Thr Pro
2210 2215 2220

His Ser His Ser Arg Thr Pro Glu Tyr Tyr Pro Asn Ala Gly Leu
2225 2230 2235

Ile Met Asn Tyr Cys Arg Asn Pro Asp Ala Val Ala Ala Pro Tyr
2240 2245 2250

Cys Tyr Thr Arg Asp Pro Gly Val Arg Trp Glu Tyr Cys Asn Leu
2255 2260 2265

Thr Gln Cys Ser Asp Ala Glu Gly Thr Ala Val Ala Pro Pro Thr
2270 2275 2280

Val Thr Pro Val Pro Ser Leu Glu Ala Pro Ser Glu Gln Ala Pro
2285 2290 2295

Thr Glu Gln Arg Pro Gly Val Gln Glu Cys Tyr His Gly Asn Gly
2300 2305 2310

```
Gln Ser  Tyr Arg Gly Thr Tyr  Ser Thr Thr Val Thr  Gly Arg Thr
    2315                 2320                 2325

Cys Gln  Ala Trp Ser Ser Met  Thr Pro His Ser His  Ser Arg Thr
    2330                 2335                 2340

Pro Glu  Tyr Tyr Pro Asn Ala  Gly Leu Ile Met Asn  Tyr Cys Arg
    2345                 2350                 2355

Asn Pro  Asp Ala Val Ala Ala  Pro Tyr Cys Tyr Thr  Arg Asp Pro
    2360                 2365                 2370

Gly Val  Arg Trp Glu Tyr Cys  Asn Leu Thr Gln Cys  Ser Asp Ala
    2375                 2380                 2385

Glu Gly  Thr Ala Val Ala Pro  Pro Thr Val Thr Pro  Val Pro Ser
    2390                 2395                 2400

Leu Glu  Ala Pro Ser Glu Gln  Ala Pro Thr Glu Gln  Arg Pro Gly
    2405                 2410                 2415

Val Gln  Glu Cys Tyr His Gly  Asn Gly Gln Ser Tyr  Arg Gly Thr
    2420                 2425                 2430

Tyr Ser  Thr Thr Val Thr Gly  Arg Thr Cys Gln Ala  Trp Ser Ser
    2435                 2440                 2445

Met Thr  Pro His Ser His Ser  Arg Thr Pro Glu Tyr  Tyr Pro Asn
    2450                 2455                 2460

Ala Gly  Leu Ile Met Asn Tyr  Cys Arg Asn Pro Asp  Ala Val Ala
    2465                 2470                 2475

Ala Pro  Tyr Cys Tyr Thr Arg  Asp Pro Gly Val Arg  Trp Glu Tyr
    2480                 2485                 2490

Cys Asn  Leu Thr Gln Cys Ser  Asp Ala Glu Gly Thr  Ala Val Ala
    2495                 2500                 2505

Pro Pro  Thr Val Thr Pro Val  Pro Ser Leu Glu Ala  Pro Ser Glu
    2510                 2515                 2520

Gln Ala  Pro Thr Glu Gln Arg  Pro Gly Val Gln Glu  Cys Tyr His
    2525                 2530                 2535
```

```
Gly Asn  Gly Gln Ser Tyr Arg  Gly Thr Tyr Ser Thr  Thr Val Thr
    2540                 2545                 2550

Gly Arg  Thr Cys Gln Ala Trp  Ser Ser Met Thr Pro  His Ser His
    2555                 2560                 2565

Ser Arg  Thr Pro Glu Tyr Tyr  Pro Asn Ala Gly Leu  Ile Met Asn
    2570                 2575                 2580

Tyr Cys  Arg Asn Pro Asp Ala  Val Ala Ala Pro Tyr  Cys Tyr Thr
    2585                 2590                 2595

Arg Asp  Pro Gly Val Arg Trp  Glu Tyr Cys Asn Leu  Thr Gln Cys
    2600                 2605                 2610

Ser Asp  Ala Glu Gly Thr Ala  Val Ala Pro Pro Thr  Val Thr Pro
    2615                 2620                 2625

Val Pro  Ser Leu Glu Ala Pro  Ser Glu Gln Ala Pro  Thr Glu Gln
    2630                 2635                 2640

Arg Pro  Gly Val Gln Glu Cys  Tyr His Gly Asn Gly  Gln Ser Tyr
    2645                 2650                 2655

Arg Gly  Thr Tyr Ser Thr Thr  Val Thr Gly Arg Thr  Cys Gln Ala
    2660                 2665                 2670

Trp Ser  Ser Met Thr Pro His  Ser His Ser Arg Thr  Pro Glu Tyr
    2675                 2680                 2685

Tyr Pro  Asn Ala Gly Leu Ile  Met Asn Tyr Cys Arg  Asn Pro Asp
    2690                 2695                 2700

Ala Val  Ala Ala Pro Tyr Cys  Tyr Thr Arg Asp Pro  Gly Val Arg
    2705                 2710                 2715

Trp Glu  Tyr Cys Asn Leu Thr  Gln Cys Ser Asp Ala  Glu Gly Thr
    2720                 2725                 2730

Ala Val  Ala Pro Pro Thr Val  Thr Pro Val Pro Ser  Leu Glu Ala
    2735                 2740                 2745

Pro Ser  Glu Gln Ala Pro Thr  Glu Gln Arg Pro Gly  Val Gln Glu
    2750                 2755                 2760
```

Cys Tyr His Gly Asn Gly Gln Ser Tyr Arg Gly Thr Tyr Ser Thr
2765 2770 2775

Thr Val Thr Gly Arg Thr Cys Gln Ala Trp Ser Ser Met Thr Pro
2780 2785 2790

His Ser His Ser Arg Thr Pro Glu Tyr Tyr Pro Asn Ala Gly Leu
2795 2800 2805

Ile Met Asn Tyr Cys Arg Asn Pro Asp Ala Val Ala Ala Pro Tyr
2810 2815 2820

Cys Tyr Thr Arg Asp Pro Gly Val Arg Trp Glu Tyr Cys Asn Leu
2825 2830 2835

Thr Gln Cys Ser Asp Ala Glu Gly Thr Ala Val Ala Pro Pro Thr
2840 2845 2850

Val Thr Pro Val Pro Ser Leu Glu Ala Pro Ser Glu Gln Ala Pro
2855 2860 2865

Thr Glu Gln Arg Pro Gly Val Gln Glu Cys Tyr His Gly Asn Gly
2870 2875 2880

Gln Ser Tyr Arg Gly Thr Tyr Ser Thr Thr Val Thr Gly Arg Thr
2885 2890 2895

Cys Gln Ala Trp Ser Ser Met Thr Pro His Ser His Ser Arg Thr
2900 2905 2910

Pro Glu Tyr Tyr Pro Asn Ala Gly Leu Ile Met Asn Tyr Cys Arg
2915 2920 2925

Asn Pro Asp Ala Val Ala Ala Pro Tyr Cys Tyr Thr Arg Asp Pro
2930 2935 2940

Gly Val Arg Trp Glu Tyr Cys Asn Leu Thr Gln Cys Ser Asp Ala
2945 2950 2955

Glu Gly Thr Ala Val Ala Pro Pro Thr Val Thr Pro Val Pro Ser
2960 2965 2970

Leu Glu Ala Pro Ser Glu Gln Ala Pro Thr Glu Gln Arg Pro Gly

2975 2980 2985

Val Gln Glu Cys Tyr His Gly Asn Gly Gln Ser Tyr Arg Gly Thr
2990 2995 3000

Tyr Ser Thr Thr Val Thr Gly Arg Thr Cys Gln Ala Trp Ser Ser
3005 3010 3015

Met Thr Pro His Ser His Ser Arg Thr Pro Glu Tyr Tyr Pro Asn
3020 3025 3030

Ala Gly Leu Ile Met Asn Tyr Cys Arg Asn Pro Asp Ala Val Ala
3035 3040 3045

Ala Pro Tyr Cys Tyr Thr Arg Asp Pro Gly Val Arg Trp Glu Tyr
3050 3055 3060

Cys Asn Leu Thr Gln Cys Ser Asp Ala Glu Gly Thr Ala Val Ala
3065 3070 3075

Pro Pro Thr Val Thr Pro Val Pro Ser Leu Glu Ala Pro Ser Glu
3080 3085 3090

Gln Ala Pro Thr Glu Gln Arg Pro Gly Val Gln Glu Cys Tyr His
3095 3100 3105

Gly Asn Gly Gln Ser Tyr Arg Gly Thr Tyr Ser Thr Thr Val Thr
3110 3115 3120

Gly Arg Thr Cys Gln Ala Trp Ser Ser Met Thr Pro His Ser His
3125 3130 3135

Ser Arg Thr Pro Glu Tyr Tyr Pro Asn Ala Gly Leu Ile Met Asn
3140 3145 3150

Tyr Cys Arg Asn Pro Asp Ala Val Ala Ala Pro Tyr Cys Tyr Thr
3155 3160 3165

Arg Asp Pro Gly Val Arg Trp Glu Tyr Cys Asn Leu Thr Gln Cys
3170 3175 3180

Ser Asp Ala Glu Gly Thr Ala Val Ala Pro Pro Thr Val Thr Pro
3185 3190 3195

Val Pro Ser Leu Glu Ala Pro Ser Glu Gln Ala Pro Thr Glu Gln
3200 3205 3210

Arg Pro Gly Val Gln Glu Cys Tyr His Gly Asn Gly Gln Ser Tyr
3215 3220 3225

Arg Gly Thr Tyr Ser Thr Thr Val Thr Gly Arg Thr Cys Gln Ala
3230 3235 3240

Trp Ser Ser Met Thr Pro His Ser His Ser Arg Thr Pro Glu Tyr
3245 3250 3255

Tyr Pro Asn Ala Gly Leu Ile Met Asn Tyr Cys Arg Asn Pro Asp
3260 3265 3270

Ala Val Ala Ala Pro Tyr Cys Tyr Thr Arg Asp Pro Gly Val Arg
3275 3280 3285

Trp Glu Tyr Cys Asn Leu Thr Gln Cys Ser Asp Ala Glu Gly Thr
3290 3295 3300

Ala Val Ala Pro Pro Thr Val Thr Pro Val Pro Ser Leu Glu Ala
3305 3310 3315

Pro Ser Glu Gln Ala Pro Thr Glu Gln Arg Pro Gly Val Gln Glu
3320 3325 3330

Cys Tyr His Gly Asn Gly Gln Ser Tyr Arg Gly Thr Tyr Ser Thr
3335 3340 3345

Thr Val Thr Gly Arg Thr Cys Gln Ala Trp Ser Ser Met Thr Pro
3350 3355 3360

His Ser His Ser Arg Thr Pro Glu Tyr Tyr Pro Asn Ala Gly Leu
3365 3370 3375

Ile Met Asn Tyr Cys Arg Asn Pro Asp Pro Val Ala Ala Pro Tyr
3380 3385 3390

Cys Tyr Thr Arg Asp Pro Ser Val Arg Trp Glu Tyr Cys Asn Leu
3395 3400 3405

Thr Gln Cys Ser Asp Ala Glu Gly Thr Ala Val Ala Pro Pro Thr
3410 3415 3420

Ile Thr Pro Ile Pro Ser Leu Glu Ala Pro Ser Glu Gln Ala Pro
3425 3430 3435

Thr Glu Gln Arg Pro Gly Val Gln Glu Cys Tyr His Gly Asn Gly
3440 3445 3450

Gln Ser Tyr Gln Gly Thr Tyr Phe Ile Thr Val Thr Gly Arg Thr
3455 3460 3465

Cys Gln Ala Trp Ser Ser Met Thr Pro His Ser His Ser Arg Thr
3470 3475 3480

Pro Ala Tyr Tyr Pro Asn Ala Gly Leu Ile Lys Asn Tyr Cys Arg
3485 3490 3495

Asn Pro Asp Pro Val Ala Ala Pro Trp Cys Tyr Thr Thr Asp Pro
3500 3505 3510

Ser Val Arg Trp Glu Tyr Cys Asn Leu Thr Arg Cys Ser Asp Ala
3515 3520 3525

Glu Trp Thr Ala Phe Val Pro Pro Asn Val Ile Leu Ala Pro Ser
3530 3535 3540

Leu Glu Ala Phe Phe Glu Gln Ala Leu Thr Glu Glu Thr Pro Gly
3545 3550 3555

Val Gln Asp Cys Tyr Tyr His Tyr Gly Gln Ser Tyr Arg Gly Thr
3560 3565 3570

Tyr Ser Thr Thr Val Thr Gly Arg Thr Cys Gln Ala Trp Ser Ser
3575 3580 3585

Met Thr Pro His Gln His Ser Arg Thr Pro Glu Asn Tyr Pro Asn
3590 3595 3600

Ala Gly Leu Thr Arg Asn Tyr Cys Arg Asn Pro Asp Ala Glu Ile
3605 3610 3615

Arg Pro Trp Cys Tyr Thr Met Asp Pro Ser Val Arg Trp Glu Tyr
3620 3625 3630

Cys Asn Leu Thr Gln Cys Leu Val Thr Glu Ser Ser Val Leu Ala
3635 3640 3645

Thr Leu Thr Val Val Pro Asp Pro Ser Thr Glu Ala Ser Ser Glu
3650 3655 3660

Glu Ala Pro Thr Glu Gln Ser Pro Gly Val Gln Asp Cys Tyr His
3665 3670 3675

Gly Asp Gly Gln Ser Tyr Arg Gly Ser Phe Ser Thr Thr Val Thr
3680 3685 3690

Gly Arg Thr Cys Gln Ser Trp Ser Ser Met Thr Pro His Trp His
3695 3700 3705

Gln Arg Thr Thr Glu Tyr Tyr Pro Asn Gly Gly Leu Thr Arg Asn
3710 3715 3720

Tyr Cys Arg Asn Pro Asp Ala Glu Ile Ser Pro Trp Cys Tyr Thr
3725 3730 3735

Met Asp Pro Asn Val Arg Trp Glu Tyr Cys Asn Leu Thr Gln Cys
3740 3745 3750

Pro Val Thr Glu Ser Ser Val Leu Ala Thr Ser Thr Ala Val Ser
3755 3760 3765

Glu Gln Ala Pro Thr Glu Gln Ser Pro Thr Val Gln Asp Cys Tyr
3770 3775 3780

His Gly Asp Gly Gln Ser Tyr Arg Gly Ser Phe Ser Thr Thr Val
3785 3790 3795

Thr Gly Arg Thr Cys Gln Ser Trp Ser Ser Met Thr Pro His Trp
3800 3805 3810

His Gln Arg Thr Thr Glu Tyr Tyr Pro Asn Gly Gly Leu Thr Arg
3815 3820 3825

Asn Tyr Cys Arg Asn Pro Asp Ala Glu Ile Arg Pro Trp Cys Tyr
3830 3835 3840

Thr Met Asp Pro Ser Val Arg Trp Glu Tyr Cys Asn Leu Thr Gln
3845 3850 3855

Cys Pro Val Met Glu Ser Thr Leu Leu Thr Thr Pro Thr Val Val

3860 3865 3870

Pro Val Pro Ser Thr Glu Leu Pro Ser Glu Glu Ala Pro Thr Glu
3875 3880 3885

Asn Ser Thr Gly Val Gln Asp Cys Tyr Arg Gly Asp Gly Gln Ser
3890 3895 3900

Tyr Arg Gly Thr Leu Ser Thr Thr Ile Thr Gly Arg Thr Cys Gln
3905 3910 3915

Ser Trp Ser Ser Met Thr Pro His Trp His Arg Arg Ile Pro Leu
3920 3925 3930

Tyr Tyr Pro Asn Ala Gly Leu Thr Arg Asn Tyr Cys Arg Asn Pro
3935 3940 3945

Asp Ala Glu Ile Arg Pro Trp Cys Tyr Thr Met Asp Pro Ser Val
3950 3955 3960

Arg Trp Glu Tyr Cys Asn Leu Thr Arg Cys Pro Val Thr Glu Ser
3965 3970 3975

Ser Val Leu Thr Thr Pro Thr Val Ala Pro Val Pro Ser Thr Glu
3980 3985 3990

Ala Pro Ser Glu Gln Ala Pro Pro Glu Lys Ser Pro Val Val Gln
3995 4000 4005

Asp Cys Tyr His Gly Asp Gly Arg Ser Tyr Arg Gly Ile Ser Ser
4010 4015 4020

Thr Thr Val Thr Gly Arg Thr Cys Gln Ser Trp Ser Ser Met Ile
4025 4030 4035

Pro His Trp His Gln Arg Thr Pro Glu Asn Tyr Pro Asn Ala Gly
4040 4045 4050

Leu Thr Glu Asn Tyr Cys Arg Asn Pro Asp Ser Gly Lys Gln Pro
4055 4060 4065

Trp Cys Tyr Thr Thr Asp Pro Cys Val Arg Trp Glu Tyr Cys Asn
4070 4075 4080

```
Leu Thr  Gln Cys Ser Glu Thr  Glu Ser Gly Val Leu  Glu Thr Pro
    4085                 4090                 4095

Thr Val  Val Pro Val Pro Ser  Met Glu Ala His Ser  Glu Ala Ala
    4100                 4105                 4110

Pro Thr  Glu Gln Thr Pro Val  Val Arg Gln Cys Tyr  His Gly Asn
    4115                 4120                 4125

Gly Gln  Ser Tyr Arg Gly Thr  Phe Ser Thr Thr Val  Thr Gly Arg
    4130                 4135                 4140

Thr Cys  Gln Ser Trp Ser Ser  Met Thr Pro His Arg  His Gln Arg
    4145                 4150                 4155

Thr Pro  Glu Asn Tyr Pro Asn  Asp Gly Leu Thr Met  Asn Tyr Cys
    4160                 4165                 4170

Arg Asn  Pro Asp Ala Asp Thr  Gly Pro Trp Cys Phe  Thr Met Asp
    4175                 4180                 4185

Pro Ser  Ile Arg Trp Glu Tyr  Cys Asn Leu Thr Arg  Cys Ser Asp
    4190                 4195                 4200

Thr Glu  Gly Thr Val Val Ala  Pro Pro Thr Val Ile  Gln Val Pro
    4205                 4210                 4215

Ser Leu  Gly Pro Pro Ser Glu  Gln Asp Cys Met Phe  Gly Asn Gly
    4220                 4225                 4230

Lys Gly  Tyr Arg Gly Lys Lys  Ala Thr Thr Val Thr  Gly Thr Pro
    4235                 4240                 4245

Cys Gln  Glu Trp Ala Ala Gln  Glu Pro His Arg His  Ser Thr Phe
    4250                 4255                 4260

Ile Pro  Gly Thr Asn Lys Trp  Ala Gly Leu Glu Lys  Asn Tyr Cys
    4265                 4270                 4275

Arg Asn  Pro Asp Gly Asp Ile  Asn Gly Pro Trp Cys  Tyr Thr Met
    4280                 4285                 4290

Asn Pro  Arg Lys Leu Phe Asp  Tyr Cys Asp Ile Pro  Leu Cys Ala
    4295                 4300                 4305
```

```
Ser Ser  Ser Phe Asp Cys Gly  Lys Pro Gln Val Glu  Pro Lys Lys
    4310                 4315                 4320

Cys Pro  Gly Ser Ile Val Gly  Gly Cys Val Ala His  Pro His Ser
    4325                 4330                 4335

Trp Pro  Trp Gln Val Ser Leu  Arg Thr Arg Phe Gly  Lys His Phe
    4340                 4345                 4350

Cys Gly  Gly Thr Leu Ile Ser  Pro Glu Trp Val Leu  Thr Ala Ala
    4355                 4360                 4365

His Cys  Leu Lys Lys Ser Ser  Arg Pro Ser Ser Tyr  Lys Val Ile
    4370                 4375                 4380

Leu Gly  Ala His Gln Glu Val  Asn Leu Glu Ser His  Val Gln Glu
    4385                 4390                 4395

Ile Glu  Val Ser Arg Leu Phe  Leu Glu Pro Thr Gln  Ala Asp Ile
    4400                 4405                 4410

Ala Leu  Leu Lys Leu Ser Arg  Pro Ala Val Ile Thr  Asp Lys Val
    4415                 4420                 4425

Met Pro  Ala Cys Leu Pro Ser  Pro Asp Tyr Met Val  Thr Ala Arg
    4430                 4435                 4440

Thr Glu  Cys Tyr Ile Thr Gly  Trp Gly Glu Thr Gln  Gly Thr Phe
    4445                 4450                 4455

Gly Thr  Gly Leu Leu Lys Glu  Ala Gln Leu Leu Val  Ile Glu Asn
    4460                 4465                 4470

Glu Val  Cys Asn His Tyr Lys  Tyr Ile Cys Ala Glu  His Leu Ala
    4475                 4480                 4485

Arg Gly  Thr Asp Ser Cys Gln  Gly Asp Ser Gly Gly  Pro Leu Val
    4490                 4495                 4500

Cys Phe  Glu Lys Asp Lys Tyr  Ile Leu Gln Gly Val  Thr Ser Trp
    4505                 4510                 4515

Gly Leu  Gly Cys Ala Arg Pro  Asn Lys Pro Gly Val  Tyr Ala Arg
    4520                 4525                 4530
```

```
Val Ser  Arg Phe Val Thr Trp  Ile Glu Gly Met Met  Arg Asn Asn
    4535                4540                4545

<210>  18
<211>  83
<212>  PRT
<213>  consensus sequence

<220>
<221>  MISC_FEATURE
<222>  (1)..(83)
<223>

<220>
<221>  MISC_FEATURE
<222>  (1)..(83)
<223>  "X" unknown amino acid

<220>
<221>  MISC_FEATURE
<222>  (1)..(83)
<223>  "Xaa" not conserved amino acid

<400>  18

Thr Arg Asp Cys Tyr Trp Gly Xaa Gly Glu Ser Tyr Arg Gly Thr Val
1               5                   10                  15

Ser Thr Thr Lys Ser Gly Lys Pro Cys Gln Arg Trp Xaa Xaa Xaa Xaa
            20                  25                  30

Pro His Gln His Lys Phe Asn Pro Xaa Xaa Xaa Xaa Xaa Xaa Xaa Leu
        35                  40                  45

Glu Glu Asn Tyr Cys Arg Asn Pro Asp Gly Asp Xaa Glu Gly Pro Trp
    50                  55                  60

Cys Tyr Thr Xaa Xaa Xaa Xaa Xaa Xaa Trp Glu Tyr Cys Asp Ile Pro
65                  70                  75                  80

Arg Cys Glu
```